# EUROPEAN PATENT SPECIFICATION

(11) **EP 3 111 927 B1**
(45) Date of publication and mention of the grant of the patent: **11.12.2019**
(21) Application number: 16164849.8
(22) Date of filing: 28.05.2010
(51) Int. Cl.: A61K 9/12, A61K 9/00, A61K 31/40, A61K 31/167, A61K 31/56, A61K 31/58, A61K 31/46, A61K 31/137, A61P 11/00, A61K 9/16, A61K 31/573, A61M 15/00

(54) **COMPOSITIONS FOR RESPIRATORY DELIVERY OF ACTIVE AGENTS AND ASSOCIATED METHODS AND SYSTEMS**
ZUSAMMENSETZUNGEN ZUR FREISETZUNG VON WIRKSTOFFEN ÜBER DIE ATEMWEGE SOWIE ZUGEHÖRIGE VERFAHREN UND SYSTEME
COMPOSITIONS POUR ADMINISTRATION RESPIRATOIRE D'AGENTS ACTIFS ET MÉTHODES ET SYSTÈMES ASSOCIÉS

(30) Priority: 29.05.2009 US 182565 P; 04.11.2009 US 258172 P; 01.03.2010 US 309365 P; 17.05.2010 US 345536 P
(43) Date of publication of application: 04.01.2017
(62) Divisional of application: 10727551.3
(73) Proprietor: Pearl Therapeutics, Inc., Redwood City, CA 94063 (US)
(72) Inventor: VEHRING, Reinhard, Redwood City, CA 94063 (US); HARTMAN, Michael, Steven, Palo Alto, CA 94306 (US); SMITH, Adrian, Edward, Emerald Hills, CA 94062 (US); JOSHI, Vidya, B., Redwood City, CA 94065 (US); DWIVEDI, Sarvajna, Kumar, Redwood City, CA 94065 (US)
(74) Representative: CSY London

(56) References cited:
- WO-A1-96/19198
- US-A1- 2003 114 428
- US-A1- 2008 226 564

## Description

### Technical Field

The present disclosure relates generally to pharmaceutical formulations for delivery of one or more active agents via the respiratory tract. In certain aspects, the present disclosure relates to compositions, and systems for pulmonary delivery of active agents via a metered dose inhaler.

### Background

Methods of targeted drug delivery that deliver an active agent at the site of action are often desirable. For example, targeted delivery of active agents can reduce undesirable side effects, lower dosing requirements and decrease therapeutic costs. In the context of respiratory delivery, inhalers are well known devices for administering an active agent to a subject's respiratory tract, and several different inhaler systems are currently commercially available. Three common inhaler systems include dry powder inhalers, nebulizers and metered dose inhalers (Malls).

Malls may be used to deliver medicaments in a solubilized form or as a suspension. Typically, Malls use a relatively high vapor pressure propellant to expel aerosolized droplets containing an active agent into the respiratory tract when the MDI is activated. Dry powder inhalers generally rely on the patient's inspiratory efforts to introduce a medicament in a dry powder form to the respiratory tract. On the other hand, nebulizers form a medicament aerosol to be inhaled by imparting energy to a liquid solution or suspension.

Malls are active delivery devices that utilize the pressure generated by a propellant. Conventionally, chlorofluorocarbons (CFCs) have been used as propellants in MDI systems because of their low toxicity, desirable vapor pressure and suitability for formulation of stable suspensions. However, traditional CFC propellants are understood to have a negative environmental impact, which has led to the development of alternative propellants that are believed to be more environmentally-friendly, such as per fluorinated compounds (PFCs) and hydro fluoroalkanes (HFAs).

The active agent to be delivered by an MDI is typically provided as a fine particulate dispersed within a propellant or combination of two or more propellants (i.e., a propellant "system"). To form the fine particulates, the active agent is typically micronized. Fine particles of active agent suspended in a propellant or propellant system tend to aggregate or flocculate rapidly. This is particularly true of active agents present in micronized form. In turn, aggregation or flocculation of these fine particles may complicate the delivery of the active agent. For example, aggregation or flocculation can lead to mechanical failures, such as those that might be caused by obstruction of the valve orifice of the aerosol container. Unwanted aggregation or flocculation of drug particles may also lead to rapid sedimentation or creaming of drug particles, and such behaviour may result in inconsistent dose delivery, which can be particularly troublesome with highly potent, low dose medicaments. Another problem associated with such suspension MDI formulations relates to crystal growth of the drug during storage, resulting in a decrease over time of aerosol properties and delivered dose uniformity of such Malls. More recently, solution approaches, such as those disclosed in U.S. Patent No. 6,964,759, have been proposed for MDI formulations containing anticholinergics.

One approach to improve aerosol performance in dry powder inhalers has been to incorporate fine particle carrier particles, such as lactose. Use of such fine excipients has not been investigated to any great extent for Malls. A recent report by Young et al., "The influence of micronized particulates on the aerosolization properties of pressurized metered dose inhalers"; Aerosol Science 40, pgs. 324-337 (2009), suggests that the use of such fine particle carriers in Malls result in a decrease in aerosol performance.

In traditional CFC systems, when the active agent present in an MDI formulation is solubilized within the propellant or propellant system, surfactants are often used to coat the surfaces of the active agent to minimize or prevent the problem of aggregation and maintain a substantially uniform dispersion. The use of surfactants in this manner is sometimes referred to as "stabilizing" the suspension. However, many surfactants that are soluble and thus effective in CFC systems are not effective in HFA and PFC propellant systems because such surfactants exhibit different solubility characteristics in non-CFC propellants.
US2008/226564 discloses respiratory dispersions of bioactive agents with perforations for the pulmonary delivery.

### Brief Description of the Drawings

FIG. 1 is a graph, which depicts the particle size distribution exhibited by an exemplary co-suspension composition according to the present description, which included glycopyrrolate, a long-acting muscarinic antagonist, as the active agent. Co-suspension MDIs were subjected to temperature cycling conditions (alternating 6h hold time at -5 or 40 °C) for 12 weeks.
FIG. 2 is a graph, which depicts the particle size distribution exhibited by an exemplary co-suspension composition according to the present description, which included glycopyrrolate, a long-acting muscarinic antagonist, as the active agent. Co-suspension MDIs were subjected to temperature cycling conditions (alternating 6h hold time at -5 or 40 °C) for 24 weeks.
FIG. 3 provides a micrograph illustrating the morphologies of a variety of suspending particles prepared according to Example 5.
FIG. 4 is a photograph of two vials that allows visualization of a co-suspension formed using active agent particles formed using glycopyrrolate and suspending particles formed using a saccharide.
FIG. 5 is a graph, which depicts the particle size distribution of an exemplary glycopyrrolate co-suspension prepared according to the present description, containing 4.5 µg per actuation delivered dose of glycopyrrolate and 6mg/mL suspending particles and subjected to temperature cycling conditions (alternating 6h hold time at -5 or 40 °C).
FIG. 6 is a graph, which depicts the particle size distribution of an exemplary glycopyrrolate co-suspension prepared according to the present description, containing 36 µg per actuation delivered dose of glycopyrrolate and 6mg/mL suspending particles and subjected to temperature cycling conditions (alternating 6h hold time at -5 or 40 °C).
FIG. 7 is a graph, which depicts the delivered dose through canister life of an exemplary glycopyrrolate co-suspension prepared according to the present description, containing 4.5 µg per actuation delivered dose of glycopyrrolate and 6mg/mL suspending particles
FIG. 8 is a graph, which depicts the delivered dose through canister life of an exemplary glycopyrrolate co-suspension prepared according to the present description, containing 36 µg per actuation delivered dose of glycopyrrolate and 6mg/mL suspending particles
FIG. 9 is a graph, which depicts the particle size distribution of an exemplary glycopyrrolate co-suspension prepared according to the present description, containing 36 µg per actuation delivered dose of glycopyrrolate and 6mg/mL suspending particles and subjected to 12 months storage at 25°C/60% RH unprotected.
FIG. 10 is a graph, which depicts the delivered dose through canister life of an exemplary glycopyrrolate co-suspension prepared according to the present description, containing 32 µg per actuation delivered dose of glycopyrrolate and 6mg/mL suspending particles and subjected to temperature cycling conditions (alternating 6h hold time at -5 or 40 °C).
FIG. 11 is a graph, which depicts the particle size distribution of an exemplary glycopyrrolate co-suspension prepared according to the present description, containing 32 µg per actuation delivered dose of glycopyrrolate and 6mg/mL suspending particles and subjected to temperature cycling conditions (alternating 6h hold time at -5 or 40 °C)
FIG. 12 is a graph, which depicts the particle size distribution of an exemplary glycopyrrolate co-suspension prepared according to the present description, containing 24 µg per actuation delivered dose of glycopyrrolate and 6mg/mL suspending particles and subjected to 6 weeks storage at 50°C/ambient relative humidity and 12 weeks at 40°C
FIG. 13 is a photograph that allows visualization of co-suspension compositions prepared according to the present description which include formoterol fumarate active agent particles.
FIG. 14 is a graph, which depicts the delivered dose uniformity achieved by formoterol co-suspension compositions prepared according to the present description.
FIG. 15 is a graph, which depicts the aerodynamic particle size distribution determined by cascade impaction of exemplary formoterol co-suspension compositions prepared according to the present description and stored for three months at 25 °C/ 75% RH, with protective overwrap, or at 40 °C / 75%RH with protective overwrap.
FIG. 16 is a graph, which depicts the chemical stability of exemplary co-suspension compositions including crystalline formoterol as the active agent. The results depicted in this figure allow comparison of the chemical stability of formoterol achieved in a co-suspension composition formulated using crystalline formoterol material with the chemical stability of suspension formulations prepared using spray dried formoterol fumarate.
FIG. 17 through FIG. 20 are electron micrographs of suspending particles prepared from various different materials, with Figure 17 providing a micrograph of trehalose suspending particles, Figure 18 providing a micrograph of HP-β-cyclodextrin suspending particles, Figure 19 providing a micrograph of Ficoll MP 70 suspending particles, and Figure 20 provding a micrograph of inulin suspending particles.
FIG. 21 provides a graph that depicts the aerodynamic particle size distribution determined by cascade impaction of exemplary co-suspension compositions prepared according to the present description and including glycopyrrolate active agent particles.
FIG. 22 provides a graph that depicts the aerodynamic particle size distribution determined by cascade impaction of exemplary co-suspension compositions prepared according to the present description and including formoterol active agent particles.
FIG. 23 provides a graph that depicts the delivered dose uniformity achieved by ultra low-dose formoterol co-suspension compositions prepared according to the present description.
FIG. 24 is a graph, which depicts the delivered dose uniformity of a co-suspension formulation containing glycopyrrolate and formoterol fumarate prepared according to the present description.
FIG. 25 is a graph, which depicts the delivered dose ratio of the co-suspension formulation described in relation to FIG. 24.
FIG. 26 is a graph, which depicts the delivered dose uniformity of a second co-suspension formulation containing formoterol fumarate and glycopyrrolate prepared according to the present description.
FIG. 27 is a graph, which depicts the delivered dose ratio of the second co-suspension formulation described in relation to FIG. 26.
FIG. 28 is a graph, which depicts the delivered dose uniformity of glycopyrrolate and formoterol fumarate in a co-suspension formulation prepared according to the present description upon storage under different conditions, as indicated.
FIG. 29 is a graph, which depicts the particle size distribution of glycopyrrolate (top) and formoterol (bottom) in exemplary co-suspension formulations prepared according to the present description upon storage under different conditions, as indicated.
FIG. 30 provides graphs illustrating the particle size distribution of glycopyrrolate (top) and formoterol (bottom) achieved by an exemplary co-suspension upon storage at indicated conditions.
FIG. 31 provides graphs illustrating the particle size distribution of glycopyrrolate (top) and formoterol (bottom) achieved by an exemplary dual co-suspension compared to particle size distributions achieved by formulations including either glycopyrrolate or formoterol fumarate alone.
FIG. 32 is a graph that depicts the formoterol fumarate particle size distribution achieved by a co-suspension prepared according to the present description, which included microcrystalline formoterol fumarate and glycopyrrolate active agent particles compared to a co-suspension only containing crystalline formoterol fumarate.
FIG. 33 is a graph that depicts the glycopyrrolate particle size distribution achieved by a dual co-suspension prepared according to the present description, which included microcrystalline glycopyrrolate active agent particles and microcrystalline formoterol fumarate active agent particles with two different particle size distributions (denoted "fine" and "coarse") or spray dried formoterol fumarate.
FIG. 34 is a graph that depicts the formoterol fumarate particle size distribution achieved by a second dual co-suspension prepared according to the present description, which included microcrystalline formoterol fumarate and microcrystalline glycopyrrolate active agent particles compared to one that contained microcrystalline glycopyrrolate actie agent particles and spray dried formoterol fumarate particles.
FIG. 35 is a graph, which depicts the delivered dose uniformity of glycopyrrolate and formoterol fumarate in an exemplary dual co-suspension formulation prepared according to the present description.
FIG. 36 depicts the delivered dose uniformity for each active agent included in an exemplary triple co-suspension composition, which included microcyrstalline glycopyrrolate, formoterol fumarate and mometasone furoate active agent particles.
FIG. 37 is a graph depicting the formoterol fumarate aerodynamic particle size distributions achieved in a triple co-suspension prepared according to the present description, which included microcrystalline glycopyrrolate, formoterol fumarate and mometasone furcate active agent particles, compared to that achieved in a dual co-suspension which included glycopyrrolate and formoterol fumarate.
FIG. 38 is a graph depicting the glycopyrrolate aerodynamic particle size distributions achieved in a triple co-suspension prepared according to the present description, which included microcrystalline glycopyrrolate, formoterol fumarate and mometasone furcate active agent particles, compared to that achieved in a dual co• suspension which included glycopyrrolate and formoterol fumarate.
FIG. 39 is a graph depicting the glycopyrrolate and tiotropium bromide aerodynamic particle size distributions achieved by a triple co-suspension prepared according to the present description, which in addition to either glycopyrrolate or tiotropium bromide included formoterol fumarate and mometasone furcate microcrystalline active agent particles.

### Detailed Description

The present invention is defined in the appended claims. Subject matters which are not encompassed by the scope of the claims do not form part of the presently claimed invention.

According to a first embodiment, the invention provides a co-suspension deliverable from a metered dose inhaler, the stable co-suspension comprising:
a suspension medium comprising a pharmaceutically acceptable propellant;
a plurality of active agent particles, wherein the active agent particles comprise glycopyrrolate and at least 90% of the active agent particles by volume exhibits an optical diameter of 7 µm or less; and
a plurality of respirable suspending particles comprising perforated phospholipid microstructures,
wherein the total mass of the respirable suspending particles exceeds the total mass of the active agent particles, the plurality of active agent particles associate with the plurality of respirable suspending particles to form a co-suspension despite buoyancy difference between the active agent particles and the respirable suspending particles within the suspension medium.

According to a second embodiment, the invention provides a metered dose inhaler containing a co-suspension of the invention.

According to a third embodiment, the invention provides a co-suspension of the invention for use in medicine.

According to a fourth embodiment, the invention provides a co-suspension of the invention for use in treating a patient suffering from an inflammatory or obstructive pulmonary disease or condition.

The methods described herein include methods for treating a pulmonary disease or disorder amenable to treatment by respiratory delivery of a co-suspension composition as described herein. For example, the compositions, methods and systems described herein can be used to treat inflammatory or obstructive pulmonary diseases or conditions.
The compositions, methods and systems described herein can be used to treat patients suffering from a disease or disorder selected from asthma, chronic obstructive pulmonary disease (COPD), exacerbation of airways hyper reactivity consequent to other drug therapy, allergic rhinitis, sinusitis, pulmonary vasoconstriction, inflammation, allergies, impeded respiration, respiratory distress syndrome, pulmonary hypertension, pulmonary vasoconstriction, and any other respiratory disease, condition, trait, genotype or phenotype that can respond to the administration of, for example, a LAMA, LABA, corticosteroid, or other active agent as described herein, whether alone or in combination with other therapies. In certain embodiments, the compositions, described herein can be used to treat pulmonary inflammation and obstruction associated with cystic fibrosis. As used herein, the terms "COPD" and "chronic obstructive pulmonary disease" encompass chronic obstructive lung disease (COLD), chronic obstructive airway disease (COAD), chronic airflow limitation (CAL) and chronic obstructive respiratory disease (CORD) and include chronic bronchitis, bronchiectasis, and emphysema. As used herein, the term "asthma" refers to asthma of whatever type or genesis, including intrinsic (non-allergic) asthma and extrinsic (allergic) asthma, mild asthma, moderate asthma, severe asthma, bronchitis asthma, exercise-induced asthma, occupational asthma and asthma induced following bacterial infection. Asthma is also to be understood as embracing wheezy-infant syndrome.

### Definitions

Unless specifically defined otherwise, the technical terms, as used herein, have their normal meaning as understood in the art. The following terms are specifically defined for the sake of clarity.

The term "active agent" is used herein to include any agent, drug, compound, composition or other substance that may be used on, or administered to a human or animal for any purpose, including therapeutic, pharmaceutical, pharmacological, diagnostic, cosmetic and prophylactic agents and immunomodulators. The term "active agent" may be used interchangeably with the terms, "drug," "pharmaceutical," "medicament," "drug substance," or "therapeutic." As used herein the "active agent" may also encompass natural or homeopathic products that are not generally considered therapeutic.

The terms "associate," "associate with" or "association" refers to an interaction or relationship between a chemical entity, composition, or structure in a condition of proximity to a surface, such as the surface of another chemical entity, composition, or structure. The association includes, for example, adsorption, adhesion, covalent bonding, hydrogen bonding, ionic bonding and electrostatic attraction, Lifshitz-van der Waals interactions and polar interactions. The term "adhere" or "adhesion" is a form of association and is used as a generic term for all forces tending to cause a particle or mass to be attracted to a surface. "Adhere" also refers to bringing and keeping particles in contact with each other, such that there is substantially no visible separation between particles due to their different buoyancies in a propellant under normal conditions. In one embodiment, a particle that attaches to or binds to a surface is encompassed by the term "adhere." Normal conditions may include storage at room temperature or under an accelerative force due to gravity. As described herein, active agent particles may associate with suspending particles to form a co-suspension, where there is substantially no visible separation between the suspending particles and the active agent particles or flocculates thereof due to differences in buoyancy within a propellant.

"Suspending particles" refer to a material or combination of materials that is acceptable for respiratory delivery, and acts as a vehicle for active agent particles. Suspending particles interact with the active agent particles to facilitate repeatable dosing, delivery or transport of active agent to the target site of delivery, i.e., the respiratory tract. The suspending particles described herein are dispersed within a suspension medium including a propellant or propellant system, and can be configured according to any shape, size or surface characteristic suited to achieving a desired suspension stability or active agent delivery performance. Exemplary suspending particles include particles that exhibit a particle size that facilitates respiratory delivery of active agent and have physical configurations suited to formulation and delivery of the stabilized suspensions as described herein.

The term "co-suspension" refers to a suspension of two or more types of particles having different compositions within a suspension medium, wherein one type of particle associates at least partially with one or more of the other particle types. The association leads to an observable change in one or more characteristics of at least one of the individual particle types suspended in the suspension medium. Characteristics modified by the association may include, for example, one or more of the rate of aggregation or flocculation, the rate and nature of separation, i.e. sedimentation or creaming, density of a cream or sediment layer, adhesion to container walls, adhesion to valve components, and rate and the level of dispersion upon agitation.

Exemplary methods for assessing whether a co-suspension is present can include the following: If one particle type has a pycnometric density greater than the propellant and another particle type has a pycnometric density lower than the propellant, a visual observation of the creaming or sedimentation behavior can be employed to determine the presence of a co-suspension. The term "pycnometric density" refers to the density of a material that makes up a particle, excluding voids within the particle. In one embodiment, the materials can be formulated or transferred into a transparent vial, typically a glass vial, for visual observation. After initial agitation the vial is left undisturbed for a sufficient time for formation of a sediment or cream layer, typically 24 hours. If the sediment or cream layer is observed to be completely or mostly a uniform single layer, a co-suspension is present. The term "co-suspension" includes partial co-suspensions, where a majority of the at least two particle types associate with each other, however, some separation (i.e., less than a majority) of the at least two particle types may be observed.

The exemplary co-suspension test may be performed at different propellant temperatures to accentuate the sedimentation or creaming behavior of particle types with a density close to the propellant density at room temperature. If the different particle types have the same nature of separation, i.e. all sediment or all cream, the presence of a co-suspension can be determined by measuring other characteristics of the suspension, such as rate of aggregation or flocculation, rate of separation, density of cream or sediment layer, adhesion to container walls, adhesion to valve components, and rate and level of dispersion upon agitation, and comparing them to the respective characteristics of the similarly suspended individual particle types. Various analytical methods generally known to those skilled in the art can be employed to measure these characteristics.

In the context of a composition containing or providing respirable aggregates, particles, drops, etc., such as compositions described herein, the term "fine particle dose" or "FPD" refers to the dose, either in total mass or fraction of the nominal dose or metered dose, that is within a respirable range. The dose that is within the respirable range is measured *in vitro* to be the dose that deposits beyond the throat stage of a cascade impactor, i.e., the sum of dose delivered at stages 3 through filter in a Next Generation Impactor operated at a flow rate of 30 l/min.

In the context of a composition containing or providing respirable aggregates, particles, drops, etc., such as compositions described herein, the term "fine particle fraction" or "FPF" refers to the proportion of the delivered material relative to the delivered dose (i.e., the amount that exits the actuator of a delivery device, such as an MDI) that is within a respirable range. The amount of delivered material within the respirable range is measured *in vitro* as the amount of material that deposits beyond the throat stage of a cascade impactor, e.g., the sum of the material delivered at stages 3 through filter in a Next Generation Impactor operated at a flow rate of 30 l/min.

As used herein, the term "inhibit" refers to a measurable lessening of the tendency of a phenomenon, symptom or condition to occur or the degree to which that phenomenon, symptom or condition occurs. The term "inhibit" or any form thereof, is used in its broadest sense and includes minimize, prevent, reduce, repress, suppress, curb, constrain, restrict, slow progress of and the like.

"Mass median aerodynamic diameter" or "MMAD" as used herein refers to the aerodynamic diameter of an aerosol below which 50% of the mass of the aerosol consists of particles with an aerodynamic diameter smaller than the MMAD, with the MMAD being calculated according to monograph 601 of the United States Pharmacopeia ("USP").

When referred to herein, the term "optical diameter" indicates the size of a particle as measured by the Fraunhofer diffraction mode using a laser diffraction particle size analyzer equipped with a dry powder dispenser (e.g., Sympatec GmbH, Clausthal-Zellerfeld, Germany).

The term solution mediated transformation refers to the phenomenon in which a more soluble form of a solid material (i.e. particles with small radius of curvature (a driving force for Ostwald ripening), or amorphous material) dissolves and recrystallizes into the more stable crystal form that can coexist in equilibrium with its saturated propellant solution.

A "patient" refers to an animal in which one or more active agents as described herein will have a therapeutic effect. In one embodiment, the patient is a human being.

"Perforated microstructures" refer to suspending particles that include a structural matrix that exhibits, defines or comprises voids, pores, defects, hollows, spaces, interstitial spaces, apertures, perforations or holes that allow the surrounding suspension medium to permeate, fill or pervade the microstructure, such as those materials and preparations described in U.S. Patent No. 6,309,623 to Weers, et al. The primary form of the perforated microstructure is, generally, not essential, and any overall configuration that provides the desired formulation characteristics is contemplated herein. Accordingly, in one embodiment, the perforated microstructures may comprise approximately spherical shapes, such as hollow, porous, spray-dried microspheres. However, collapsed, corrugated, deformed or fractured particulates of any primary form or aspect ratio may also be compatible.

As is true of suspending particles described herein, perforated microstructures may be formed of any biocompatible material that does not substantially degrade or dissolve in the selected suspension medium. While a wide variety of materials may be used to form the particles, in some embodiments, the structural matrix is associated with, or includes, a surfactant such as, a phospholipid or fluorinated surfactant. Although not required, the incorporation of a compatible surfactant in the perforated microstructure or, more generally, the suspending particles, can improve the stability of the respiratory dispersions, increase pulmonary deposition and facilitate the preparation of the suspension.

The term "suspension medium" as used herein refers to a substance providing a continuous phase within which active agent particles and suspending particles can be dispersed to provide a co-suspension formulation. The suspension medium used in co-suspension formulations described herein includes propellant. As used herein, the term "propellant" refers to one or more pharmacologically inert substances which exert a sufficiently high vapor pressure at normal room temperature to propel a medicament from the canister of an MDI to a patient on actuation of the MDI's metering valve. Therefore, the term "propellant" refers to both a single propellant and to a combination of two or more different propellants forming a "propellant system."

The term "respirable" generally refers to particles, aggregates, drops, etc. sized such that they can be inhaled and reach the airways of the lung.

When used to refer to co-suspension compositions described herein, the terms "physical stability" and "physically stable" refer to a composition that is resistant to one or more of aggregation, flocculation, and particle size changes due to solution mediated transformations and is capable of substantially maintaining the MMAD of suspending particles and the fine particle dose. In one embodiment, physical stability may be evaluated through subjecting compositions to accelerated degradation conditions, such as by temperature cycling as described herein.

When referring to active agents, the term "potent" indicates active agents that are therapeutically effective at or below doses ranging from about 0.01 mg/kg to about 1 mg/kg. Typical doses of potent active agents generally range from about 100 µg to about 100 mg.

When referring to active agents, the term "highly potent" indicates active agents that are therapeutically effective at or below doses of about 10 µg/kg. Typical doses of highly potent active agents generally range up to about 100 µg.

The terms "suspension stability" and "stable suspension" refer to suspension formulations capable of maintaining the properties of a co-suspension of active agent particles and suspending particles over a period of time. In one embodiment, suspension stability may be measured through delivered dose uniformity achieved by co-suspension compositions described herein.

The term "substantially insoluble" means that a composition is either totally insoluble in a particular solvent or it is poorly soluble in that particular solvent. The term "substantially insoluble" means that a particular solute has a solubility of less than one part per 100 parts solvent. The term "substantially insoluble" includes the definitions of "slightly soluble" (from 100 to 1000 parts solvent per 1 part solute), "very slightly soluble" (from 1000 to 10,000 parts solvent per 1 part solute) and "practically insoluble" (more than 10,000 parts solvent per 1 part solute) as given in Table 16-1 of Remington: The Science and Practice of Pharmacy, 21 st ed. Lippincott, Williams & Wilkins, 2006, p. 212.

The term "surfactant," as used herein, refers to any agent which preferentially adsorbs to an interface between two immiscible phases, such as the interface between water and an organic polymer solution, a water/air interface or organic solvent/air interface. Surfactants generally possess a hydrophilic moiety and a lipophilic moiety, such that, upon adsorbing to microparticles, they tend to present moieties to the continuous phase that do not attract similarly-coated particles, thus reducing particle agglomeration. In some embodiments, surfactants may also promote adsorption of a drug and increase bioavailability of the drug.

A "therapeutically effective amount" is the amount of compound which achieves a therapeutic effect by inhibiting a disease or disorder in a patient or by prophylactically inhibiting or preventing the onset of a disease or disorder. A therapeutically effective amount may be an amount which relieves to some extent one or more symptoms of a disease or disorder in a patient; returns to normal either partially or completely one or more physiological or biochemical parameters associated with or causative of the disease or disorder; and/or reduces the likelihood of the onset of the disease of disorder.

The terms "chemically stable" and "chemical stability" refer to co-suspension formulations wherein the individual degradation products of active agent remain below the limits specified by regulatory requirements during the shelf life of the product for human use (e.g., 1% of total chromatographic peak area per ICH guidance Q3B(R2)) and there is acceptable mass balance (e.g., as defined in ICH guidance Q1E) between active agent assay and total degradation products.

### Compositions

The compositions described herein are co-suspensions that include a suspension medium including a propellant, active agent particles, and suspending particles. Of course, if desired, the compositions described herein may include one or more additional constituents. Moreover, variations and combinations of components of the compositions described herein may be used. For example, the active agent particles included in the co-suspension formulations may include two or more active agents, or two or more different species of active agent particles may be used, with each different species of active agent particle including one or more different active agents. Alternatively, two or more species of suspending particles may be used in compositions for the delivery of one or more active agents or active agent particles. Even further, for example, the compositions may include an active agent disposed within the material forming a suspending particle and another active agent(s) co-suspended as active agent particles with the suspending particles.

It has been found that, in formulations according to the present description, active agent particles exhibit an association with the suspending particles such that separation of the active agent particles from the suspending particles is substantially prevented, resulting in co-location of active agent particles and suspending particles within the suspension medium. Generally, due to density differences between distinct species of particles and the medium within which they are suspended (e.g., a propellant or propellant system), buoyancy forces cause creaming of particles with lower density than the propellant and sedimentation of particles with higher density than the propellant. Therefore, in suspensions that consist of a mixture of different types of particles with different density or different tendencies to flocculate, sedimentation or creaming behavior is expected to be specific to each of the different particle types and expected to lead to separation of the different particle types within the suspension medium.

However, the combinations of propellant, active agent particles and suspending particles described herein provide co-suspensions wherein active agent particles and suspending particles co-locate within the propellant (i.e., the active agent particles associate with the suspending particles such that suspending particles and active agent particles do not exhibit substantial separation relative to each other, such as by differential sedimentation or creaming, even after a time sufficient for the formation of a cream or sediment layer). In particular embodiments, for example, the compositions described herein form co-suspensions wherein the suspending particles remain associated with active agent particles when subjected to buoyancy forces amplified by temperature fluctuations and/or centrifugation at accelerations up to an over, for example, 1 g, 10 g, 35 g, 50 g, and 100 g. However, the co-suspensions described herein are not defined by a specific threshold force of association. For example, a co-suspension as contemplated herein may be successfully achieved where the active agent particles associate with the suspending particles such that there is no substantial separation of active agent particles and suspending particles within the continuous phase formed by the suspension medium under typical patient use conditions.

Co-suspensions of active agent particles and suspending particles according to the present description provide desirable chemical stability, suspension stability, and active agent delivery characteristics. For example, in certain embodiments, when present within an MDI canister, co-suspensions as described herein can inhibit or reduce one or more of the following: flocculation of active agent material; differential sedimentation or creaming of active agent particles an suspending particles; solution mediated transformation of active agent material; and loss of active agent to the surfaces of the container closure system, in particular the metering valve components. Such qualities work to achieve and preserve aerosol performance as the co-suspension formulation is delivered from an MDI such that desirable fine particle fraction, fine particle dose and delivered dose uniformity characteristics are achieved and substantially maintained throughout emptying of an MDI canister within which the co-suspension formulation is contained. Additionally, co-suspensions according to the present description can provide a stable formulation that provides consistent dosing characteristics, even for potent and highly potent active agents, while utilizing a relatively simple HFA suspension medium that does not require modification by the addition of, for example, cosolvents, antisolvents, solubilizing agents or adjuvants.

Providing a co-suspension according to the present description may also simplify formulation, delivery and dosing of the desired active agents. Without being bound by a particular theory, it is thought that by achieving a co-suspension of active agent particles and suspending particles, the delivery, physical stability, and dosing of an active agent contained within such a dispersion may be substantially controlled through control of the size, composition, morphology and relative amount of the suspending particles, and is less dependent upon the size and morphology of the particles of active agent. Moreover, in specific embodiments, the pharmaceutical compositions described herein can be formulated with a non-CFC propellant or propellant system substantially free of antisolvents, solubilizing agents, cosolvents, or adjuvants.

Co-suspension compositions formulated according to the present teachings can inhibit physical and chemical degradation of the active agents included therein. For example, in specific embodiments, the compositions described herein may inhibit one or more of chemical degradation, flocculation, aggregation and solution mediated transformation of the active agents included in the compositions. The chemical and suspension stability provided by the co-suspension compositions described herein allows the compositions to be dispensed in a manner that achieves desirable delivered dose uniformity throughout emptying of an MDI canister ("DDU"), even where the active agents to be delivered are highly potent and delivered at very low doses.

Co-suspension compositions as described herein can achieve a DDU of ± 30%, or better for each of the active agents included therein. In one such embodiment, compositions described herein achieve a DDU of ± 25%, or better, for each of the active agents included therein. In another such embodiment, compositions described herein achieve a DDU of ± 20%, or better, for each of the active agents included therein. Moreover, co-suspension compositions according to the present description serve to substantially preserve FPF and FPD performance throughout emptying of an MDI canister, even after being subjected to accelerated degradation conditions. For instance, compositions according to the present description maintain as much as 80%, 90%, 95%, or more, of the original FPF or FPD performance, even after being subjected to accelerated degradation conditions.

Co-suspension compositions described herein provide the added benefit of achieving such performance while being formulated using non-CFC propellants. In specific embodiments, the compositions described herein achieve one or more of a targeted DDU, FPF or FPD, while being formulated with suspension medium including only one or more non-CFC propellants and without the need to modify the characteristics of the non-CFC propellant, such as by the addition of, for example, one or more cosolvent, antisolvent, solubilizing agent, adjuvant or other propellant modifying material.

### Suspension Medium

The suspension medium included in a composition described herein includes one or more propellants. In general, suitable propellants for use as suspension mediums are those propellant gases that can be liquefied under pressure at room temperature, and upon inhalation or topical use, are safe and toxicologically innocuous. Additionally, it is desirable that the selected propellant be relatively non-reactive with the suspending particles or active agent particles. Exemplary compatible propellants include hydrofluoroalkanes (HFAs), perfluorinated compounds (PFCs), and chlorofluorocarbons (CFCs).

Specific examples of propellants that may be used to form the suspension medium of the co-suspensions disclosed herein include 1,1,1,2-tetrafluoroethane (CF₃CH₂F) (HFA-134a), 1,1,1,2,3,3,3-heptafluoro-n-propane (CF₃CHFCF₃) (HFA-227), perfluoroethane, monochloro-fluoromethane, 1,1 difluoroethane, and combinations thereof. Even further, suitable propellants include, for example: short chain hydrocarbons; C₁-₄ hydrogen-containing chlorofluorocarbons such as CH₂CIF, CCl₂FCHCIF, CF₃CHClF, CHF₂CCIF₂, CHClFCHF₂, CF₃CH₂Cl, and CClF₂CH₃; C₁-₄ hydrogen-containing fluorocarbons (e.g., HFAs) such as CHF₂CHF₂, CF₃CH₂F, CHF₂CH₃, and CF₃CHFCF₃; and perfluorocarbons such as CF₃CF₃ and CF₃CF₂CF₃.

Specific fluorocarbons, or classes of fluorinated compounds, that may be used as suspension media include, but are not limited to, fluoroheptane, fluorocycloheptane, fluoromethylcycloheptane, fluorohexane, fluorocyclohexane, fluoropentane, fluorocyclopentane, fluoromethylcyclopentane, fluorodimethyl-cyclopentanes, fluoromethylcyclobutane, fluorodimethylcyclobutane, fluorotrimethyl-cyclobutane, fluorobutane, fluorocyclobutane, fluoropropane, fluoroethers, fluoropolyethers and fluorotriethylamines. These compounds may be used alone or in combination with more volatile propellants.

In addition to the aforementioned fluorocarbons and hydrofluoroalkanes, various exemplary chlorofluorocarbons and substituted fluorinated compounds may also be used as suspension media. In this respect, FC-11 (CCl₃F), FC-11B1 (CBrCl₂F), FC-11 B2 (CBr₂ClF), FC12B2 (CF₂Br₂), FC21 (CHCl₂F), FC21B1 (CHBrCIF), FC-21B2 (CHBr₂F), FC-31B1 (CH₂BrF), FC113A (CCl₃CF₃), FC-122 (CClF₂CHCl₂), FC-123 (CF₃CHCl₂), FC-132 (CHCIFCHCIF), FC-133 (CHClFCHF₂), FC-141 (CH₂ClCHClF), FC-141B (CCl₂FCH₃), FC-142 (CHF₂CH₂Cl), FC-151 (CH₂FCH₂Cl), FC-152 (CH₂FCH₂F), FC-1112 (CClF=CClF), FC-1121 (CHCl=CFCl) and FC-1131 (CHCI=CHF) may also be used, while recognizing the possible attendant environmental concerns. As such, each of these compounds may be used, alone or in combination with other compounds (i.e., less volatile fluorocarbons) to form the stabilized suspensions disclosed herein.

In some embodiments, the suspension medium may be formed of a single propellant. In other embodiments, a combination of propellants may be used to form the suspension medium. In some embodiments, relatively volatile compounds may be mixed with lower vapor pressure components to provide suspension media having specified physical characteristics selected to improve stability or enhance the bioavailability of the dispersed active agent. In some embodiments, the lower vapor pressure compounds will comprise fluorinated compounds (e.g., fluorocarbons) having a boiling point greater than about 25°C. In some embodiments, lower vapor pressure fluorinated compounds for use in the suspension medium may include perfluorooctylbromide C₈F₁₇Br (PFOB or perflubron), dichlorofluorooctane CₛF₁₆Cl₂, perfluorooctylethane CₛF₁₇C₂H₅ (PFOE), perfluorodecylbromide C₁₀F₂₁,Br (PFDB) or perfluorobutylethane C₄F₉C₂H₅. In certain embodiments, these lower vapor pressure compounds are present in a relatively low level. Such compounds may be added directly to the suspension medium or may be associated with the suspending particles.

In some embodiments, the suspension medium may be formed of a propellant or propellant system that is substantially free of additional materials, including, for example, antisolvents, solubilizing agents, cosolvents or adjuvants. However, in other embodiments, depending on the selection of propellant, the properties of the suspending particles, or the nature of the active agents to be delivered, additional materials, such as, for example, one or more of an appropriate antisolvent, solubilizing agents, cosolvent or adjuvant may be added, for example, to adjust vapor pressure, stability, or solubility of suspended particles. For example, propane, ethanol, isopropyl alcohol, butane, isobutane, pentane, isopentane or a dialkyl ether, such as dimethyl ether, may be incorporated with the propellant in the suspension medium. Similarly, the suspension medium may contain a volatile fluorocarbon. In other embodiments, one or both of polyvinylpyrrolidone ("PVP") or polyethylene glycol ("PEG") may be added to the suspension medium. Adding PVP or PEG to the suspension medium may achieve one or more desired functional characteristics, and in one example, PVP or PEG may be added to the suspension medium as a crystal growth inhibitor. In general, where used, up to about 1% w/w of the propellant may comprise a volatile cosolvent or adjuvant such as a hydrocarbon or fluorocarbon. In other embodiments, the suspension medium may comprise less than about 0.01%, 0.1%, or 0.5% w/w cosolvent or adjuvant. Where PVP or PEG are included in the suspension medium, such constituents may be included at up to about 1% w/w, or they may comprise less than about 0.01%, 0.1%, or 0.5% w/w of the suspension medium.

### Active agent particles

The active agent particles included in the co-suspensions described herein are formed of a material capable of being dispersed and suspended within the suspension medium and are sized to facilitate delivery of respirable particles from the co-suspension. In one embodiment, therefore, the active agent particles are provided as a micronized material wherein at least 90% of the active agent particles by volume exhibit an optical diameter of about 7 µm or less. In other embodiments, the active agent particles are provided as a micronized material wherein at least 90% of the active agent particles by volume exhibit an optical diameter selected from a range of about 7 µm to about 1 µm, about 5 µm to about 2 µm, and about 3 µm to about 2 µm. In further embodiments, the active agent particles are provided as a micronized material wherein at least 90% of the active agent particles by volume exhibit an optical diameter selected from 6 µm or less, 5 µm or less, 4 µm or less, or 3 µm or less. In another embodiment, the active agent particles are provided as a micronized material wherein at least 50% of the active agent particle material by volume exhibits an optical diameter of about 4 µm or less. In further embodiments, the active agent particles are provided as a micronized material wherein at least 50% of the active agent particle material by volume exhibits an optical diameter selected from about 3 µm or less, about 2 µm or less, about 1.5 µm or less, and about 1 µm or less. In still further embodiments, the active agent particles are provided as a micronized material wherein at least 50% of the active agent particles by volume exhibit an optical diameter selected from a range of about 4 µm to about 1 µm, about 3 µm to about 1 µm, about 2 µm to about 1 µm, about 1.3 µm , and about 1.9 µm.

The active agent particles may be formed entirely of active agent or they may be formulated to include one or more active agents in combination with one or more excipients or adjuvants. In specific embodiments, an active agent present in the active agent present in the active agent particles may be entirely or substantially crystalline. In another embodiment, the active agent particles may include an active agent present in both crystal and amorphous states. In yet another embodiment, the active agent particles may include an active agent present in substantially an amorphous state. In yet a further embodiment, where two or more active agents are present in active agent particles, at least one such active agent may be present in crystalline or substantially crystalline form and at least another active agent may be present in an amorphous state. In still another embodiment, where two or more active agents are present in active agent particles, each such active agent may be present in crystalline or substantially crystalline form. Where the active agent particles described herein include one or more active agents in combination with one or more excipients or adjuvants, the excipients and adjuvants can be selected based on the chemical and physical properties of the active agent used. Moreover, suitable excipients for formulation of active agent particles include those described herein in association with the suspending particles. In specific embodiments, for example, the active agent particles may be formulated with one or more of the lipid, phospholipid, carbohydrate, amino acid, organic salt, peptide, protein, alditols, synthetic or natural polymer, or surfactant materials as described, for example, in association with the suspending particles.

In other embodiments including two or more active agents, at least one of the active agents is included in active agent particles co-suspended with suspending particles, while at least one other active agent may be included in suspending particles utilized in the co-suspension. For example, one or more active agents may be added to a solution of one or more of the lipid, phospholipid, carbohydrate, amino acid, organic salt, peptide, protein, alditols, synthetic or natural polymer, or surfactant materials and spray-dried to form one or more different species of suspending particle that contain the active agent within the material forming the suspending particle.

Any suitable process may be employed to achieve micronized active agent material for inclusion in the compositions described herein. A variety of processes may be used to create active agent particles suitable for use in the co-suspension formulations described herein, including, but not limited to, micronization by milling or grinding processes, crystallization or recrystallization processes, and processes using precipitation from supercritical or near-supercritical solvents, spray drying, spray freeze drying, or lyophilization. Patent references teaching suitable methods for obtaining micronized active agent particles include, for example, in U.S. Patent No. 6,063,138, U.S. Patent No. 5,858,410, U.S. Patent No. 5,851,453, U.S. Patent No. 5,833,891, U.S. Patent No. 5, 707,634, and International Patent Publication No. WO 2007/009164. Where the active agent particles include active agent material formulated with one or more excipient or adjuvant, micronized active agent particles can be formed using one or more of the preceding processes and such processes can be utilized to achieve active agent particles having a desired size distribution and particle configuration.

The active agent particles may be provided in any suitable concentration within the suspension medium. The active agent included in the active agent particles is substantially insoluble in the suspension medium. In some embodiments, the active agent, despite being substantially insoluble, exhibits measurable solubility in the suspension medium. However, even where the active agent exhibits measurable solubility in the suspension medium, the compositions described herein work to preserve the physical stability of such active agents. In particular, in specific embodiments, an active agent included in the compositions described herein may exhibit sufficient solubility in the suspension medium such that as much as 5% of the total active agent mass dissolves in the suspension medium. Alternatively, the solubility of an active agent may result in dissolution of as much as 1% of the total active agent mass in the suspension medium. In another embodiment, the solubility of an active agent may result in dissolution of as much as 0.5% of the total active agent mass in the suspension medium. In yet another embodiment, the solubility of an active agent may result in dissolution of as much as 0.05% of the total active agent mass in the suspension medium. In still another embodiment, the solubility of an active agent may result in dissolution of as much as 0.025% of the total active agent mass in the suspension medium.

A variety of therapeutic or prophylactic agents can be incorporated into the co-suspension compositions disclosed herein. Exemplary active agents include those that may be administered in the form of aerosolized medicaments, and active agents suitable for use in the compositions described herein include those that may be presented in a form or formulated in a manner which is dispersible within the selected suspension medium (e.g., is substantially insoluble or exhibits a solubility in the suspension medium that substantially maintains a co-suspension formulation), is capable of forming a co-suspension with the suspending particles, and is subject to respirable uptake in physiologically effective amounts. The active agents that may be utilized in forming the active agent particles described herein can have a variety of biological activities.

Examples of specific active agents that may be included in a composition for example, short-acting beta agonists, e.g., bitolterol, carbuterol, fenoterol, hexoprenaline, isoprenaline (isoproterenol), levosalbutamol, orciprenaline (metaproterenol), pirbuterol, procaterol, rimiterol, salbutamol (albuterol), terbutaline, tulobuterol, reproterol, ipratropium and epinephrine; long-acting β2 adrenergic receptor agonist ("LABA"), e.g., bambuterol, clenbuterol, formoterol, and salmeterol; ultra long-acting β2 adrenergic receptor agonists, e.g., carmoterol, milveterol, indacaterol, and saligenin- or indole-containing and adamantyl-derived β2 agonists; corticosteroids, e.g., beclomethasone, budesonide, ciclesonide, flunisolide, fluticasone, methyl-prednisolone, mometasone, prednisone and triamcinolone; anti-inflammatories, e.g. fluticasone propionate, beclomethasone dipropionate, flunisolide, budesonide, tripedane, cortisone, prednisone, prednisolone, dexamethasone, betamethasone, or triamcinolone acetonide; antitussives, e.g. noscapine; bronchodilators, e.g., ephedrine, adrenaline, fenoterol, formoterol isoprenaline, metaproterenol, salbutamol, albuterol, salmeterol, terbutaline; and muscarinic antagonists, including long-acting muscarinic antagonists ("LAMA") e.g., glycopyrrolate, dexipirronium, scopolamine, tropicamide, pirenzepine, dimenhydrinate, tiotropium, darotropium, aclidinium, trospium, ipatropium, atropine, benzatropin, or oxitropium.

Where appropriate, the active agents may be used in the form of salts (e.g., alkali metal or amine salts or as acid addition salts) or as esters, solvates (hydrates), derivatives, or a free base thereof. Additionally, the active agents may be in any crystalline form or isomeric form or mixture of isomeric forms, for example, as pure enantiomers, a mixture of enantiomers, as racemates or as mixtures thereof. In this regard, the form of the active agents may be selected to optimize the activity and/or stability of the active agent and/or to minimize the solubility of the active agent in the suspension medium.

Because the compositions disclosed enable the reproducible delivery of very low doses of active agents, in certain embodiments, the active agent included in the compositions described herein may be selected from one or more potent or highly potent active agents. For example, in certain embodiments, the compositions described herein may include one or more potent active agents that are to be delivered at a dose selected from between about 100 µg and about 100 mg, about 100 µg and about 10 mg, and about 100 µg and 1 mg per actuation of an MDI. In other embodiments, the compositions described herein may include one or more potent or highly potent active agents that are to be delivered at a dose selected from up to about 80 µg, up to about 40 µg, up to about 20 µg, or between about 10 µg and about 100 µg per actuation of an MDI. Additionally, in certain embodiments, the compositions described herein may include one or more highly potent active agents that are to be delivered at a dose selected from between about 0.1 and about 2 µg, about 0.1 and about 1 µg, and about 0.1 and about 0.5 µg per actuation of an MDI.

A composition as described herein contains a combination of two or more active agents. For example, a combination of two or more species of active agent particles may be co-suspended with a single species of suspending particles. Alternatively, a composition may include two or more species of active agent particles co-suspended with two or more different species of suspending particles. Even further, a composition as described herein may include two or more active agents combined within a single species of active agent particle. For example, where the active agent particles are formulated using one or more excipients or adjuvants in addition to the active agent material, such active agent particles may include individual particles that include two or more different active agents.

In certain embodiments an active agent included in the compositions described herein is glycopyrrolateincluding any pharmaceutically acceptable salts, esters, or solvates thereof.

Glycopyrrolate can be used to treat inflammatory or obstructive pulmonary diseases and disorders such as, for example, those described herein. As an anticholinergic, glycopyrrolate acts as a bronchodilator and provides an ant secretory effect, which is a benefit for use in the therapy of pulmonary diseases and disorders characterized by increased mucus secretions. Glycopyrrolate is a quaternary ammonium salt. Where appropriate, glycopyrrolate may be used in the form of salts (e.g. alkali metal or amine salts, or as acid addition salts) or as esters or as solvates (hydrates). Additionally, the glycopyrrolate may be in any crystalline form or isomeric form or mixture of isomeric forms, for example a pure enantiomer, a mixture of enantiomers, a racemate or a mixture thereof. In this regard, the form of glycopyrrolate may be selected to optimize the activity and/or stability of glycopyrrolate and/or to minimize the solubility of glycopyrrolate in the suspension medium. Suitable counter ions are pharmaceutically acceptable counter ions including, for example, fluoride, chloride, bromide, iodide, nitrate, sulphate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1-hydroxynaphthalene-2-carboxylate, 3-hydroxynaphthalene-2-carboxylate, methanesulfonate and benzenesulfonate. In particular embodiments of the compositions described herein, the bromide salt of glycopyrrolate, namely 3-[(cyclopentyl-hydroxyphenylacetyl)oxy]-1,1-dimethylpyrrolidinium bromide, is used and can be prepared according to the procedures set out in U.S. Pat. No. 2,956,062.

Where the compositions described herein include glycopyrrolate, in certain embodiments, the compositions may include sufficient glycopyrrolate to provide a target delivered dose selected from between about 10 µg and about 200 µg per actuation of an MDI, about 15 µg and about 150 µg per actuation of an MDI, and about 18 µg and 144 µg per actuation of an MDI. In other such embodiments, the formulations include sufficient glycopyrrolate to provide a dose selected from up to about 200 µg, up to about 150 µg, up to about 75 µg, up to about 40 µg, or up to about 20 µg per actuation. In yet further embodiments, the formulations include sufficient glycopyrrolate to provide a dose selected from about 18 µg per actuation, 36 µg per actuation or about 72 µg per actuation. In order to achieve targeted delivered doses as described herein, where compositions described herein include glycopyrrolate as the active agent, in specific embodiments, the amount of glycopyrrolate included in the compositions may be selected from, for example, between about 0.04 mg/mL and about 2.25 mg/mL.

In certain embodiments, the compositions described herein include a LABA active agent. In such embodiments, a LABA active agent can be selected from, for example, bambuterol, clenbuterol, formoterol, salmoterol, carmoterol, milveterol, indacaterol, and saligenin- or indole- containing and adamantyl-derived β₂ agonists, and any pharmaceutically acceptable salts, esters, isomers or solvates thereof. In certain such embodiments, formoterol is selected as the LABA active agent. Formoterol can be used to treat inflammatory or obstructive pulmonary diseases and disorders such as, for example, those described herein. Formoterol has the chemical name (±)-2-hydroxy-5-[(1RS)-1 -hydroxy-2-[[(1 RS)-2-(4-methoxyphenyl)-1 -methylethyl]-amino]ethyl] formanilide, and is commonly used in pharmaceutical compositions as the racemic fumarate dihydrate salt. Where appropriate, formoterol may be used in the form of salts (e.g. alkali metal or amine salts or as acid addition salts) or as esters or as solvates (hydrates). Additionally, the formoterol may be in any crystalline form or isomeric form or mixture of isomeric forms, for example a pure enantiomer, a mixture of enantiomers, a racemate or a mixture thereof. In this regard, the form of formoterol may be selected to optimize the activity and/or stability of formoterol and/or to minimize the solubility of formoterol in the suspension medium. Pharmaceutically acceptable salts of formoterol include, for example, salts of inorganic acids such as hydrochloric, hydrobromic, sulfuric and phosphoric acids, and organic acids such as fumaric, maleic, acetic, lactic, citric, tartaric, ascorbic, succinic, glutaric, gluconic, tricarballylic, oleic, benzoic, p-methoxybenzoic, salicylic, o- and p-hydroxybenzoic, p-chlorobenzoic, methanesulfonic, p-toluenesulfonic and 3-hydroxy-2-naphthalene carboxylic acids.
Hydrates of formoterol are described, for example, in U.S. Pat. No. 3,994,974 and U.S. Pat. No. 5,684,199. Specific crystalline forms of formoterol and other β₂ adrenergic receptor agonists are described, for example, in WO95/05805, and specific isomers of formoterol are described in U.S. Patent No. 6,040,344.

In specific embodiments, the formoterol material utilized to form the formoterol particles is formoterol fumarate, and in one such embodiment, the formoterol fumarate is present in the dihydrate form. Where the compositions described herein include formoterol, in certain embodiments, the compositions described herein may include formoterol at a concentration that achieves a targeted delivered dose selected from between about 1 µg and about 30 µg, about 1 µg and about 10 µg, about 2 µg and 5 µg, about 2 µg and about 10 µg, about 5 µg and about 10 µg, and 3 µg and about 30 µg per actuation of an MDI. In other embodiments, the compositions described herein may include formoterol in an amount sufficient to provide a targeted delivered dose selected from up to about 30 µg, up to about 10 µg, up to about 5 µg, up to about 2.5 µg, up to about 2 µg, or up to about 1.5 µg per actuation. In order to achieve targeted delivered doses as described herein, where compositions described herein include formoterol as the active agent, in specific embodiments, the amount of formoterol included in the compositions may be selected from, for example, between about 0.01 mg/mL and about 1 mg/mL, between about 0.01 mg/mL and about 0.5 mg/mL, and between about 0.03 mg/mL and about 0.4 mg/mL.

Where the pharmaceutical co-suspension compositions described herein include a LABA active agent, in certain embodiments, the active agent may be salmeterol, including any pharmaceutically acceptable salts, esters, isomers or solvates thereof. Salmeterol can be used to treat inflammatory or obstructive pulmonary diseases and disorders such as, for example, those described herein. Salmeterol, pharmaceutically acceptable salts of salmeterol, and methods for producing the same are described, for example, in U.S. Patent No. 4,992,474, U.S. Patent No. 5,126,375, and U.S. patent 5,225,445.

Where salmeterol is included as a LABA active agent, in certain embodiments, the compositions described herein may include salmeterol at a concentration that achieves a delivered dose selected from between about 2 µg and about 120 µg, about 4 µg and about 40 µg, about 8 µg and 20 µg, about 8 µg and about 40 µg, about 20 µg and about 40 µg, and 12 µg and about 120 µg per actuation of an MDI. In other embodiments, the compositions described herein may include salmeterol in an amount sufficient to provide a delivered dose selected from up to about 120 µg, up to about 40 µg, up to about 20 µg, up to about 10 µg, up to about 8 µg, or up to about 6 µg per actuation of an MDI. In order to achieve targeted delivered doses as described herein, where compositions described herein include salmeterol as the active agent, in specific embodiments, the amount of salmeterol included in the compositions may be selected from, for example, between about 0.04 mg/mL and about 4 mg/mL, between about 0.04 mg/mL and about 2.0 mg/mL, and between about 0.12 mg/mL and about 0.8 mg/mL. For example, the compositions described herein may include sufficient salmeterol to provide a target delivered dose selected from between about 4 µg and about 120 µg, about 20 µg and about 100 µg, and between about 40 µg and about 120 µg per actuation of an MDI. In still other embodiments, the compositions described herein may include sufficient salmeterol to provide a targeted delivered dose selected from up to about 100 µg, up to about 40 µg, or up to about 15 µg per actuation of an MDI.

In still other embodiments, the compositions described herein include a corticosteroid. Such active agents may be selected from, for example, beclomethasone, budesonide, ciclesonide, flunisolide, fluticasone, methyl-prednisolone, mometasone, prednisone and trimacinolone. Where the compositions include a corticosteroid active agent, in particular embodiments, mometasone may be selected. Mometasone, pharmaceutically acceptable salts of mometasone, such as mometasone furoate, and preparation of such materials are known and described, for example, in U.S. Pat. No. 4,472,393, U.S. Pat. No. 5,886,200, and U.S. Pat. No. 6,177,560. Mometasone is suitable for use in treating diseases or disorders associated with pulmonary inflammation or obstruction, such as those described herein (see, e.g., U.S. Pat. No. 5,889,015, U.S. Pat. No. 6,057,307, U.S. Pat. No. 6,057,581, U.S. Pat. No. 6,677,322, U.S. Pat. No. 6,677,323 and U.S. Pat. No. 6,365,581).

Where the compositions described herein include mometasone, in particular embodiments, the compositions include mometasone, including any pharmaceutically acceptable salts, esters, isomers or solvates thereof, in an amount sufficient to provide a target delivered dose selected from between about 20 µg and about 400 µg, about 20 µg and about 200 µg, about 50 µg and about 200 µg, about 100 µg and about 200 µg, about 20 µg and about 100 µg, and about 50 µg and about 100 |jg per actuation of an MDI. In still other embodiments, the compositions described herein may include mometasone, including any pharmaceutically acceptable salts, esters, isomers or solvates thereof, in an amount sufficient to provide a targeted delivered dose selected from up to about 400 µg, up to about 200 µg, or up to about 100 µg per actuation of an MDI.

In other embodiments, the compositions described herein include a corticosteroid selected from fluticasone and budesonide. Both fluticasone and budesonide are suitable for use in treatment of conditions associated with pulmonary inflammation or obstruction, such as those described herein. Fluticasone, pharmaceutically acceptable salts of fluticasone, such as fluticasone propionate, and preparation of such materials are known, and described, for example, in U.S. Pat. No. 4,335,121, U.S. Pat. No. 4,187,301, and U.S. Pat. Pub. No. US2008125407. Budesonide is also well known and described, for example, in U.S. Pat. No. 3,929,768. In certain embodiments, compositions described herein may include fluticasone, including any pharmaceutically acceptable salts, esters, isomers or solvates thereof, in an amount sufficient to provide a target delivered dose selected from between about 20 µg and about 200 µg, about 50 µg and about 175 µg, and between about 80 µg and about 160 µg per actuation of an MDI. In other embodiments, the compositions described herein may include fluticasone, including any pharmaceutically acceptable salts, esters, isomers or solvates thereof, in an amount sufficient to provide a targeted delivered dose selected from up to about 175 µg up to about 100 µg or up to about 80 µg per actuation of an MDI. In particular embodiments, compositions described herein may include budesonide, including any pharmaceutically acceptable salts, esters, isomers or solvates thereof, in an amount sufficient to provide target delivered dose selected from between about 30 µg and about 240 µg, about 30 µg and about 120 µg, and between about 30 µg and about 50 µg per actuation of an MDI. In still other embodiments, the compositions described herein may include budesonide, including any pharmaceutically acceptable salts, esters, isomers or solvates thereof, in an amount sufficient to provide a targeted delivered dose selected from up to about 240 µg, up to about 120 µg, or up to about 50 µg per actuation of an MDI.

The co-suspension compositions described herein may include two or more active agents. In particular embodiments, where two or more active agents are included, the compositions described herein may include a combination of active agents selected from a combination of a glycopyrrolate and LABA active agents, a combination of glycopyrrolate and corticosteroid active agents. In other embodiments, the co-suspension compositions described herein may include three or more active agents. In certain such embodiments, the composition includes a combination of active agents selected from a combination of a glycopyrrolate, LABA and corticosteroid active agents. For example, a co-suspension composition as described herein may include a combination of active agents selected from a combination of glycopyrrolate and formoterol, a combination of glycopyrrolate, formoterol, and budesonide, and a combination of glycopyrrolate, formoterol, and mometasone.

### Suspending particles

The suspending particles included in the co-suspension compositions described herein work to facilitate stabilization and delivery of the active agent included in the compositions. Though various forms of suspending particles may be used, the suspending particles are typically formed from pharmacologically inert material that is acceptable for inhalation and is substantially insoluble in the propellant selected. Generally, the majority of suspending particles are sized within a respirable range. In particular embodiments, therefore, the MMAD of the suspending particles will not exceed about 10 µm but is not lower than about 500 nm. In an alternative embodiment, the MMAD of the suspending particles is between about 5 µm and about 750 nm. In yet another embodiment, the MMAD of the suspending particles is between about 1 µm and about 3 µm. When used in an embodiment for nasal delivery from an MDI, the MMAD of the suspending particles is between 10 µm and 50 µm.

In order to achieve respirable suspending particles within the MMAD ranges described, the suspending particles will typically exhibit a volume median optical diameter between about 0.2 µm and about 50 µm. In one embodiment, the suspending particles exhibit a volume median optical diameter that does not exceed about 25 µm. In another embodiment, the suspending particles exhibit a volume median optical diameter selected from between about 0.5 µm and about 15 µm, between about 1.5 µm and about 10 µm, and between about 2 µm and about 5 µm.

The concentration of suspending particles included in a composition according to the present description can be adjusted, depending on, for example, the amount of active agent particles and suspension medium used. In one embodiment, the suspending particles are included in the suspension medium at a concentration selected from about 1 mg/mL to about 15 mg/mL, about 3 mg/mL to about 10 mg/mL, 5 mg/mL to about 8 mg/mL, and about 6 mg/mL. In another embodiment, the suspending particles are included in the suspension medium at a concentration of up to about 30 mg/mL. In yet another embodiment, the suspending particles are included in the suspension medium at a concentration of up to about 25 mg/mL.

The relative amount of suspending particles to active agent particles is selected to achieve a co-suspension as contemplated herein. A co-suspension composition may be achieved where the amount of suspending particles, as measured by mass, exceeds that of the active agent particles. For example, in specific embodiments, the ratio of the total mass of the suspending particles to the total mass of active agent particles may be between about 3:1 and about 15:1, or alternatively from about 2:1 and 8:1. Alternatively, the ratio of the total mass of the suspending particles to the total mass of active agent particles may be above about 1, such as up to about 1.5, up to about 5, up to about 10, up to about 15, up to about 17, up to about 20, up to about 30, up to about 40, up to about 50, up to about 60, up to about 75, up to about 100, up to about 150, and up to about 200, depending on the nature of the suspending particles and active agent particles used. In further embodiments, the ratio of the total mass of the suspending particles to the total mass of the active agent particles may be selected from between about 10 and about 200, between about 60 and about 200, between about 15 and about 60, between about 15 and about 170, between about 15 and about 60, about 16, about 60, and about 170.

In other embodiments, the amount of suspending particles, as measured by mass, is less than that of the active agent particles. For example, in particular embodiments, the mass of the suspending particles may be as low as 20% of the total mass of the active agent particles. However, in some embodiments, the total mass of the suspending particles may also approximate or equal the total mass of the active agent particles.

Suspending particles suitable for use in the compositions described herein may be formed of one or more pharmaceutically acceptable materials or excipients that are suitable for inhaled delivery and do not substantially degrade or dissolve in the suspension medium. In one embodiment, perforated microstructures, as defined herein, may be used as the suspending particles. Exemplary excipients that may be used in the formulation of suspending particles described herein include but are not limited to (a) carbohydrates, e.g., monosaccharides such as fructose, galactose, glucose, D-mannose, sorbose, and the like; disaccharides, such as sucrose, lactose, trehalose, cellobiose, and the like; cyclodextrins, such as 2-hydroxypropyl-β-cyclodextrin; and polysaccharides, such as raffinose, maltodextrins, dextrans, starches, chitin, chitosan, inulin, and the like; (b) amino acids, such as alanine, glycine, arginine, aspartic acid, glutamic acid, cysteine, lysine, leucine, isoleucine, valine, and the like; (c) metal and organic salts prepared from organic acids and bases, such as sodium citrate, sodium ascorbate, magnesium gluconate, sodium gluconate, tromethamin hydrochloride, and the like; (d) peptides and proteins such as aspartame, trileucine, human serum albumin, collagen, gelatin, and the like; (e) alditols, such as mannitol, xylitol, and the like; (f) synthetic or natural polymers or combinations thereof, such as polylactides, polylactide-glycolides, cyclodextrins, polyacrylates, methylcellulose, carboxymethylcellulose, polyvinyl alcohols, polyanhydrides, polylactams, polyvinyl pyrrolidones, hyaluronic acid, polyethylene glycols; and (g) surfactants including fluorinated and nonfluorinated compounds such as saturated and unsaturated lipids, nonionic detergents, nonionic block copolymers, ionic surfactants and combinations thereof.

Additionally, phospholipids from both natural and synthetic sources may be used in preparing suspending particles suitable for use in the compositions described herein. In particular embodiments, the phospholipid chosen will have a gel to liquid crystal phase transition of greater than about 40°C. Exemplary phospholipids are relatively long chain (i.e., C₁₆-C₂₂) saturated lipids and may comprise saturated phospholipids, such as saturated phosphatidylcholines having acyl chain lengths of 16 C or 18 C (palmitoyl and stearoyl). Exemplary phospholipids include phosphoglycerides such as dipalmitoylphosphatidylcholine, disteroylphosphatidylcholine, diarachidoylphosphatidylcholine, dibehenoylphosphatidylcholine, diphosphatidyl glycerol, short-chain phosphatidylcholines, long-chain saturated phosphatidylethanolamines, long-chain saturated phosphatidylserines, long-chain saturated phosphatidylglycerols, and long-chain saturated phosphatidylinositols. Additional excipients are disclosed in International Patent Publication No. WO 96/32149 and U.S. Patent Nos. 6,358,530, 6,372,258 and 6,518,239.

In particular embodiments, the suspending particles may be formed using one or more lipids, phospholipids or saccharides, as described herein. In some embodiments, suspending particles include one or more surfactants. The use of suspending particles formed of or incorporating one or more surfactants may promote absorption of the selected active agent, thereby increasing bioavailability. The suspending particles described herein, such as, for example, suspending particles formed using one or more lipids, can be formed to exhibit a desired surface rugosity (roughness), which can further reduce inter-particle interactions and improve aerosolization by reducing the surface area available for particle-particle interaction. In further embodiments, if suitable, a lipid that is naturally occurring in the lung could be used in forming the suspending particles, as such suspending particles that have the potential to reduce opsonization (and thereby reducing phagocytosis by alveolar macrophages), thus providing a longer-lived controlled release particle in the lung.

In another aspect, the suspending particles utilized in the compositions described herein may be selected to increase storage stability of the selected active agent, similar to that disclosed in International Patent Publication No WO 2005/000267. For example, in one embodiment, the suspending particles my include pharmaceutically acceptable glass stabilization excipients having a Tg of at least 55 °C, at least 75 °C, or at least 100 °C. Glass formers suitable for use in compositions described herein include, but are not limited to, one or more of trileucine, sodium citrate, sodium phosphate, ascorbic acid, inulin, cyclodextrin, polyvinyl pyrrolidone, mannitol, sucrose, trehalose, lactose, and, proline. Examples of additional glass-forming excipients are disclosed in U. S. Patent Nos. RE 37,872, 5,928,469, 6,258,341, and 6,309,671. In particular embodiments, suspending particles may include a calcium salt, such as calcium chloride, as described, for example, in U.S. Patent No. 7,442,388.

The suspending particles may be designed, sized and shaped as desired to provide desirable stability and active agent delivery characteristics. In one exemplary embodiment, the suspending particles comprise perforated microstructures as described herein. Where perforated microstructures are used as suspending particles in the compositions described herein, they may be formed using one or more excipients as described herein. For example, in particular embodiments, perforated microstructures may include at least one of the following: lipids, phospholipids, nonionic detergents, nonionic block copolymers, ionic surfactants, biocompatible fluorinated surfactants and combinations thereof, particularly those approved for pulmonary use. Specific surfactants that may be used in the preparation of perforated microstructures include poloxamer 188, poloxamer 407 and poloxamer 338. Other specific surfactants include oleic acid or its alkali salts. In one embodiment, the perforated microstructures include greater than about 10% w/w surfactant.

In some embodiments, suspending particles may be prepared by forming an oil-in-water emulsion, using a fluorocarbon oil (e.g., perfluorooctyl bromide, perfluorodecalin) which may be emulsified using a surfactant such as a long chain saturated phospholipid. The resulting perfluorocarbon in water emulsion may be then processed using a high pressure homogenizer to reduce the oil droplet size. The perfluorocarbon emulsion may be fed into a spray dryer, optionally with an active agent solution, if it is desirable to include active agent within the matrix of the perforated microstructures. As is well known, spray drying is a one-step process that converts a liquid feed to a dried particulate form. Spray drying has been used to provide powdered pharmaceutical material for various administrative routes, including inhalation. Operating conditions of the spray dryer (such as inlet and outlet temperature, feed rate, atomization pressure, flow rate of the drying air and nozzle configuration) can be adjusted to produce the desired particle size producing a yield of the resulting dry microstructures. Such methods of producing exemplary perforated microstructures are disclosed in U.S. Patent No. 6,309,623 to Weers et al.

Perforated microstructures as described herein may also be formed through lyophilization and subsequent milling or micronization. Lyophilization is a freeze-drying process in which water is sublimed from the composition after it is frozen. This process allows drying without elevated temperatures. In yet further embodiments, the suspending particles may be produced using a spray freeze drying process, such as is disclosed in U.S. Patent 5,727,333.

Furthermore, suspending particles as described herein may include bulking agents, such as polymeric particles. Polymeric polymers may be formed from biocompatible and/or biodegradable polymers, copolymers or blends. In one embodiment, polymers capable of forming aerodynamically light particles may be used, such as functionalized polyester graft copolymers and biodegradable polyanhydrides. For example, bulk eroding polymers based on polyesters including poly(hydroxy acids) can be used. Polyglycolic acid (PGA), polyactic acid (PLA) or copolymers thereof may be used to form suspending particles. The polyester may include a charged or functionalizable group, such as an amino acid. For example, suspending particles may be formed of poly(D,L-lactic acid) and/or poly(D,L-lactic-co-glycolic acid) (PLGA), which incorporate a surfactant such as DPPC.

Other potential polymer candidates for use in suspending particles may include polyamides, polycarbonates, polyalkylenes such as polyethylene, polypropylene, poly(ethylene glycol), poly(ethylene oxide), poly(ethylene terephthalate), poly vinyl compounds such as polyvinyl alcohols, polyvinyl ethers, and polyvinyl esters, polymers of acrylic and methacrylic acids, celluloses and other polysaccharides, and peptides or proteins, or copolymers or blends thereof. Polymers may be selected with or modified to have the appropriate stability and degradation rates in vivo for different controlled drug delivery applications.

In an embodiment of a composition as described herein that includes glycopyrrolate and one or more of fluticasone, mometasone and budesonide as an active agent, the ratio of the total mass of the suspending particles to the total mass of the active agent particles may be selected from between about 1 and about 20, between about 2.5 and about 15, and about 2.5 and about 10. In an embodiment of a composition as described herein that includes one or more of fluticasone, mometasone, and budesonide as an active agent, the ratio of the total mass of the suspending particles to the total mass of the active agent particles may be selected from between about 1 and about 15, between about 1.5 and about 10, and between about 2.5 and about 8. In another embodiment as described, where the composition includes salmeterol as an active agent, the ratio of the total mass of the suspending particles to the total mass of the active agent particles may be selected from between about 10 and about 30, between about 15 and about 25, and about 20. In yet a further embodiment, where a composition as described herein includes formoterol as an active agent, the ratio of the total mass of the suspending particles to the total mass of the active agent particles may be selected from between about 10 and about 200, between about 50 and about 125, about 5 and about 50, between about 1 and about 15, between about 1.5 and about 10, and between about 2.5 and about 8.

The compositions described herein may include two or more species of suspending particles. For example, the compositions described herein may include a single species of active agent particle and two or more species of suspending particles. Alternatively, in other embodiments, the compositions described herein may include two or more species of active agent particles combined with two or more species of suspending particles. Even further, compositions according to the present description can include suspending particles that include one or more active agents incorporated into the suspending particles Where active agent is incorporated into suspending particles, the suspending particles will be of a respirable size and can be formulated and produced using, for example, the methods and materials described herein in association with the active agent particles, the suspending particles and the experimental Examples provided.

Compositions formulated according to the present teachings can inhibit degradation of active agent included therein. For example, in specific embodiments, the compositions described herein inhibit one or more of flocculation, aggregation and the solution mediated transformation of active agent material included in the compositions. The pharmaceutical compositions described herein are suited for respiratory delivery via and MDI in a manner that achieves desirable delivered dose uniformity ("DDU") of each active agent included in a combination of two or more active agents, even with combinations including potent and highly potent actives. As is illustrated in detail in the Examples included herein, even when delivering very low doses of two or more active agents, compositions described herein can achieve a DDU of ± 30%, or better, for each active agent throughout emptying of an MDI canister. In one such embodiment, compositions described herein achieve a DDU of ± 25%, or better, for each active agent throughout emptying of an MDI canister. In yet another such embodiment, compositions described herein achieve a DDU for the active agent of ± 20%, or better, for each active agent throughout emptying of an MDI canister.

Pharmaceutical compositions described herein also serve to substantially preserve FPF and FPD performance throughout emptying of an MDI canister, even after being subjected to accelerated degradation conditions. For instance, compositions according to the present description maintain as much as 80%, 90%, 95%, or more, of the original FPF and FPD performance throughout emptying of an MDI canister, even after being subjected to accelerated degradation conditions. Compositions described herein provide the added benefit of achieving such performance while being formulated using non-CFC propellants and eliminating or substantially avoiding combination effects often experienced with compositions incorporating multiple active agents. In specific embodiments, the compositions described herein achieve one or all of a targeted DDU, FPF and FPD performance while being formulated with suspension medium including only one or more non-CFC propellants and without the need to modify the characteristics of the non-CFC propellant, such as by the addition of, for example, one or more cosolvent, antisolvent, solubilizing agent, adjuvant or other propellant modifying material.

### Methods

Compositions formulated according to the present teachings can inhibit degradation of the active agent included therein. For example, in specific embodiments, the compositions described herein inhibit one or more of flocculation, aggregation and Ostwald ripening of the active agent(s) included in the compositions. The stability provided by the compositions described herein allows the compositions to be dispensed in a manner that achieves desirable delivered dose uniformity throughout emptying of an MDI canister ("DDU"), even where the active agent to be delivered is highly potent and the delivered dose of the active agent is selected from, for example, less than one of 100 µg , 80 µg, 40 µg, 20 µg, 10 µg, 9 µg, 8 µg, 7 µg, 6 µg, 5 µg, 4 µg, 3 µg, 2 µg, 1 µg, 0.5 µg, and 0.1 µg per actuation of the MDI. As is described in detail in the Examples included herein, even at low doses of highly potent active agents, compositions described herein can achieve a DDU of ± 30%, or better, for each of the active agents included in the composition. In an alternative embodiment, compositions described herein achieve a DDU of ± 25%, or better, for each of the active agents included in the composition. In yet another embodiment, compositions described herein achieve a DDU of ± 20%, or better, for each of the active agents included in the composition.

Moreover, compositions according to the present description serve to substantially preserve FPF and FPD performance throughout emptying of an MDI canister, even after being subjected to accelerated degradation conditions. For instance, compositions according to the present description maintain as much as 80%, 90%, 95%, or more, of the original FPF and FPD performance, even when they incorporate multiple active agents. Compositions described herein provide the added benefit of achieving such performance while being formulated using non-CFC propellants. In specific embodiments, the compositions described herein achieve desired one or all of a targeted DDU, FPF and FPD performance while being formulated with suspension medium including only one or more non-CFC propellants and without the need to modify the characteristics of the non-CFC propellant, such as by the addition of, for example, one or more cosolvent, antisolvent, solubilizing agent, adjuvant or other propellant modifying material.

The stability and physical characteristics of the compositions described herein support several methods. For example, in one embodiment, a method of formulating a pharmaceutical composition for respiratory delivery of an active agent is provided herein. The method involves the steps of providing a suspension medium, one or more species of active agent particles and one or more species of suspending particles, as described herein, and combining such constituents to form a composition wherein active agent particles associate with the suspending particles and co-locate with the suspending particles within the suspension medium such that a co-suspension as described herein is formed. In one such embodiment, the association of the active agent particles and the suspending particles is such that they do not separate due to their different buoyancies in a propellant. As will be appreciated, a method of formulating a pharmaceutical composition as described herein can include providing two or more species of active agent particles in combination with one or more species of suspending particles. Alternatively, the method may include providing two or more suspending particles in combination with one or more species of active agent particles.

In further embodiments the compositions described herein support, for example, methods for forming stabilized formulations of active agents for pulmonary delivery, methods for preserving the FPF and/or FPD throughout emptying of an MDI canister, methods for pulmonary delivery of potent or highly potent active agents, and methods of achieving a DDU selected from ± 30%, or better, ± 25%, or better, and ± 20%, or better, for potent and highly potent drugs administered through pulmonary delivery.

In methods involving pulmonary delivery of active agents using compositions described herein, the compositions may be delivered by an MDI. Therefore, in particular methods, an MDI loaded with a composition described herein is obtained, and the desired active agent is administered to a patient through pulmonary delivery through actuation of the MDI. For example, after shaking the MDI device, the mouthpiece is inserted into a patient's mouth between the lips and teeth. The patient typically exhales deeply to empty the lungs and then takes a slow deep breath while actuating the cartridge of the MDI. When actuated, the specified volume of formulation travels to the expansion chamber, out the actuator nozzle and into a high-velocity spray that is drawn into the lungs of a patient. In one embodiment the dose of active agent delivered throughout emptying of an MDI canister is not more than 20% greater than the mean delivered dose and is not less than 20% less than the mean delivered dose.

In specific embodiments of methods for providing a stabilized formulation of active agent for pulmonary delivery, the present disclosure provides methods for inhibiting solution mediated transformation of an active agent in a pharmaceutical formulation for pulmonary delivery. In one embodiment, a suspension medium as described herein, such as a suspension medium formed by an HFA propellant, is obtained. Suspending particles are also obtained or prepared as described herein. One or more species of active agent particles as described herein are also obtained, and the suspension medium, suspending particles and active agent particles are combined to form a co-suspension wherein the active agent particles associate with suspending particles and co-locate with the suspending particles within the continuous phase formed by the suspension medium. When compared to the active agent contained in the same suspension medium in the absence of suspending particles, co-suspensions according to the present description have been found to exhibit a higher tolerance to solution mediated transformation and irreversible crystal aggregation, and thus can lead to improved stability and dosing uniformity, allowing the formulation of active agents that are somewhat physically unstable in the suspension medium alone.

In specific embodiments of methods for preserving the FPF and/or FPD provided by a pharmaceutical formulation for pulmonary delivery, a respirable co-suspension as described herein is provided which is capable of maintaining the FPD and/or the FPF to within ± 20%, ± 10%, or even ± 5% the initial FPD and/or FPF, respectively, throughout emptying of an MDI canister. Such performance can be achieved even after the co-suspension is subjected to accelerated degradation conditions. In one embodiment, a suspension medium as described herein, such as a suspension medium formed by an HFA propellant, is obtained. Suspending particles are also obtained or prepared as described herein. One or more species of active agent particles as described herein are also obtained, and the suspension medium, suspending particles and active agent particles are combined to form a co- suspension wherein the active agent particles associate with suspending particles and co-locate with the suspending particles within the suspension medium. Even after exposure of such composition to one or more temperature cycling events, the co-suspension maintains an FPD or FPF within ± 20%, ± 10%, or even ± 5% of the respective values measured prior to exposure of the composition to the one or more temperature cycling events.

Methods for treating patients suffering from an inflammatory or obstructive pulmonary disease or condition are provided herein. Specific such methods include pulmonary delivery of a therapeutically effective amount of a pharmaceutical composition described herein, and in certain such methods pulmonary administration of the pharmaceutical composition is accomplished by delivering the composition using an MDI. In certain embodiments, the compositions, and systems described herein can be used to treat patients suffering from a disease or disorder selected from asthma, COPD, exacerbation of airways hyper reactivity consequent to other drug therapy, allergic rhinitis, sinusitis, pulmonary vasoconstriction, inflammation, allergies, impeded respiration, respiratory distress syndrome, pulmonary hypertension, pulmonary vasoconstriction, and any other respiratory disease, condition, trait, genotype or phenotype that can respond to the administration of, for example, a LAMA, LABA, corticosteroid, or other active agent as described herein, whether alone or in combination with other therapies. In certain embodiments, the compositions, described herein can be used to treat pulmonary inflammation and obstruction associated with cystic fibrosis. In specific methods for treating patients suffering from an inflammatory or obstructive pulmonary disease or condition, the pulmonary disease or condition is selected from those specifically described herein, and the method includes pulmonary delivery of a co-suspension composition according to the present description to the patient via an MDI, wherein the pulmonary delivery of such composition includes administering one or more active agents at a dose or dose range as described in association with the co-suspension compositions disclosed herein.

### Metered Dose Inhaler Systems

As described in relation to the methods provided herein, the compositions disclosed herein may be used in an MDI system. MDIs are configured to deliver a specific amount of a medicament in aerosol form. In one embodiment, an MDI system includes a pressurized, liquid phase formulation-filled canister disposed in an actuator formed with a mouthpiece. The MDI system may include the formulations described herein, which include a suspension medium, at least one species of active agent particles and at least one species of suspending particles. The canister used in the MDI be any of any suitable configuration, and in one exemplary embodiment, the canister may have a volume ranging from about 5 mL to about 25 mL, such as, for example a canister having a 19 mL volume. After shaking the device, the mouthpiece is inserted into a patient's mouth between the lips and teeth. The patient typically exhales deeply to empty the lungs and then takes a slow deep breath while actuating the cartridge.

Inside an exemplary cartridge is a metering valve including a metering chamber capable of holding a defined volume of the formulation (e.g., 63 µl or any other suitable volume available in commercially available metering valves), which is released into an expansion chamber at the distal end of the valve stem when actuated. The actuator retains the canister and may also include a port with an actuator nozzle for receiving the valve stem of the metering valve. When actuated, the specified volume of formulation travels to the expansion chamber, out the actuator nozzle and into a high-velocity spray that is drawn into the lungs of a patient. Any active agents and reagents used in the following examples are either commercially available or, with the benefit of the teachings provided herein, can be prepared according to standard literature procedures by those skilled in the art.

### Example 1

Active agent particles formed of glycopyrrolate (Pyrrolidinium, 3-((cyclopentylhydroxyphenylacetyl)oxy)-1, 1-dimethyl-, bromide) were formed by micronizing glycopyrrolate using a jet mill. The particle size distribution of the micronized glycopyrrolate (GP) was determined by laser diffraction. 50% by volume of the micronized particles exhibited an optical diameter smaller than 2.1 µm, 90% by volume were smaller than 5 µm.

Suspending particles were manufactured as follows: 500 ml of a fluorocarbon-in-water emulsion of PFOB (perfluoroctyl bromide) stabilized by a phospholipid was prepared. 18.7 g of the phospholipid, DSPC (1,2-Distearoyl-sn-Glycero-3-Phosphocholine), and 1.3g of calcium chloride were homogenized in 400 ml of hot water (75 °C) using a high shear mixer. 100 ml of PFOB were added slowly during homogenization. The resulting coarse emulsion was then further homogenized using a high pressure homogenizer (Model C3, Avestin, Ottawa, CA) at pressures of up to 170 MPa for 5 passes.

The emulsion was spray dried in nitrogen using the following spray drying conditions: Inlet temperature 95 °C, outlet temperature 72 °C, emulsion feed rate 2.4 mL/min, total gas flow 525 L/min. The particle size distribution of the suspending particles was determined by laser diffraction. 50% by volume of the suspending particles were smaller than 2.9 µm, the Geometric Standard Deviation of the distribution was 1.8.

Metered dose inhalers were prepared by weighing the target masses of micronized GP particles and suspending particles into fluorinated ethylene polymer (FEP) coated aluminum canisters (Presspart, Blackburn, UK) with 19 mL volume. The target masses and the target delivered dose assuming 20% actuator deposition are given in Table 1 for five different configurations (configurations 1A through 1C representing different suspensions of GP particles and suspending particles; configuration 1D representing GP particles alone; configuration 1E representing suspending particles alone). The canisters were crimp sealed with 63 µl valves (# BK 357, Bespak, King's Lynn, UK) and filled with 12.4 g of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK). Additional inhalers for visual observation of suspension quality were prepared using glass vials.

**Table 1: Results for Glycopyrrolate Co-suspensions of Example 1**

| Configuration ID | GP (mg/can) | Suspending particles (mg/can) | Target delivered dose (µg) | Delivered Dose (µg) | FPF (%) | MMAD (µm) |
|---|---|---|---|---|---|---|
| 1A | 3.4 | 61 | 16.5 | 17.8 | 41.3 | 3.7 |
| 1B | 4.1 | 61 | 20 | 19.4 | 42.0 | 3.9 |
| 1C | 4.1 | 15 | 20 | 19.2 | 42.7 | 3.2 |
| 1D | 4.1 | 0 | 20 | 11.1-15.3 | 27.0 | 3.3 |
| 1E | 0 | 61 | - | - | 53.6 * | 3.2 |

| | | | | | | |
|---|---|---|---|---|---|---|
| * Based on DSPC assay. | | | | | | |

Aerosol performance was assessed shortly after manufacturing in accordance with USP <601> (United States Pharmacopeia Monograph 601). A Next Generation Impactor (NGI) operated at a flow rate of 30 L/min was used for determination of particle size distribution. Sample canisters were seated into an actuator with two waste actuations and two additional waste priming actuations. Five actuations were collected in the NGI with a USP throat attached. The valve, actuator, throat, NGI cups, stages, and filter were rinsed with volumetrically dispensed solvent. The sample solutions were assayed using a drug specific chromatographic method. The fine particle fraction was defined using the sum of stages 3 through filter. Delivered dose uniformity through use testing was performed using a Dose Uniformity Sampling Apparatus as described in USP <601>. Inhalers were seated and primed as described before. Two actuations were collected and assayed at beginning, middle and end of use.

Visual observation of the co-suspended configurations (1A, 1B, 1C) showed no sedimentation of drug crystals. The suspension flocculated slowly and formed a homogeneous, single cream layer similar to the comparator configuration 1E, which included suspending particles suspended alone. In contrast, the micronized GP particles alone (configuration 1D) flocculated and sedimented quickly. Configuration 1B showed no indication of separation of GP particles from the suspending particles even after centrifugation at 35***g*** for 20 minutes. The same result was observed (i.e., lack of GP particle separation) when centrifuged up to 200***g***. Configuration 1C (low suspending concentration) showed a small amount of GP crystals settling out after centrifugation at 35***g*** for 20 minutes.

While the co-suspended configurations achieved a delivered dose within 10 % of target, the GP particles suspended alone showed much higher variability in delivered dose in a range significantly below target. The fine particle fraction relative to configuration 1D was improved by more than 50%. The MMADs of the co-suspended configurations were acceptable and depended on the suspension concentration of the suspending particles. The delivered dose uniformity through use was tested for configurations 1B and 1C. All individual delivered doses were within ±20% of mean. The results showed that the drug crystals forming the GP particles associate to the suspending particles, a co-suspension was formed, and the aerosol performance of the co-suspension was mostly determined by the suspending particles.

The association between GP crystals and suspending particles was strong enough to overcome buoyancy forces, as it was observed that GP crystals do not separate from the perforated microstructures and settling of the crystals is inhibited

### Example 2

Glycopyrrolate (GP) particles were formed by micronization using a jet mill. Suspending particles were manufactured as described in Example 1. The particle size distribution of the micronized GP was determined by laser diffraction. 50% by volume of the micronized particles exhibited an optical diameter smaller than 1.7 µm, 90% by volume exhibited an optical diameter smaller than 4.1 µm. Five different lots of metered dose inhalers were different lots were made. For configurations 2A, 2B and 2C the total concentration of DSPC, CaCl₂, and GP in the feedstock was 40 mg/mL, for configuration 2D and 2E this concentration was doubled.

Metered dose inhalers were prepared by weighing the target masses of GP particles and suspending particles into canisters as described in Example 1. No further excipients were used. The target masses were 4 mg / canister for GP particles and 60 mg / canister for the suspending particles, resulting in a suspending particle to GP particle ratio of 15 for configurations 2A and 2D. The target masses were 5.1 mg / canister for GP particles and 51 mg / canister for the suspending particles, resulting in a suspending particle to GP particle ratio of 10 for configuration 2B. The target masses were 8 mg / canister for GP particles and 60 mg / canister for the suspending particles, resulting in a suspending particle to GP particle ratio of 7.5 for configurations 2C and 2E. Propellant and container closure system were as described in Example 1.

The GP crystals were placed in HFA 134a in a canister under pressure and were equilibrated for 3 weeks at room temperature to determine their solubility in the propellant. The samples were filtered under pressure at ambient temperature through filters with a pore width of 0.22 µm. The filtrate was evaporated and the GP dissolved in methanol and chromatographically analyzed. A solubility of 0.17 ± 0.07 µg/g was found. Using this value it was determined that 2.1 µg or 0.05% of GP present in the canister dissolved in the propellant. Previous articles teach that microcrystalline material with a measurable solubility in the propellant will not be physically stable due to solution mediated transformation [N. C. Miller, The Effects of Water in Inhalation Suspension Aerosol Formulations, in: P. A. Byron, Ed. , Respiratory Drug Delivery, CRC Press, 1990, p 250], or that actives with solubility's above 0.1 µg/g should be formulated with an adjuvant to prevent a solution mediated transformation [P. Rogueda, Novel Hydrofluoroalkane Suspension Formulations for Respiratory Drug Delivery, Expert Opin. Drug Deliv. 2, 625-638, 2005]

The filled metered dose inhalers were stored valve down without overwrap at two different conditions: 1) refrigerated at 5°C; and 2) room temperature at 25°C / 60% RH. Aerosol performance and delivered dose uniformity tests as described in Example 1 were carried out at different time points. The results, which are summarized in Table 2, show a stable fine particle fraction at refrigerated and room temperature conditions.

**Table 2: Fine particle fraction of configurations in Example 2**

| # | Storage | FPF in % | | | |
|---|---|---|---|---|---|
| | | **Initial** | **2 months** | **3 months** | **6 months** |
| 2A | 5°C | 49 | 51 | 52 | - |
| | 25 °C/60 % RH | | 48 | 51 | - |
| 2B | 25 °C/60 % RH | 50 | 46 | 49 | 48 |
| 2D | 5 °C | 51 | 54 | 54 | - |
| | 25 °C/60 % RH | | 46 | 49 | 49 |

Configurations 2C and 2E were subjected to a temperature cycling test. The canisters were subjected to -5 °C and 40 °C alternating between temperatures every 6 hours for a total duration of twelve weeks. Fine particle fraction was 53% for both configurations at the beginning of the study. After twelve weeks of cycling the FPF was unchanged, i.e. at 55% for configuration 2C and at 53% for configuration 2E.

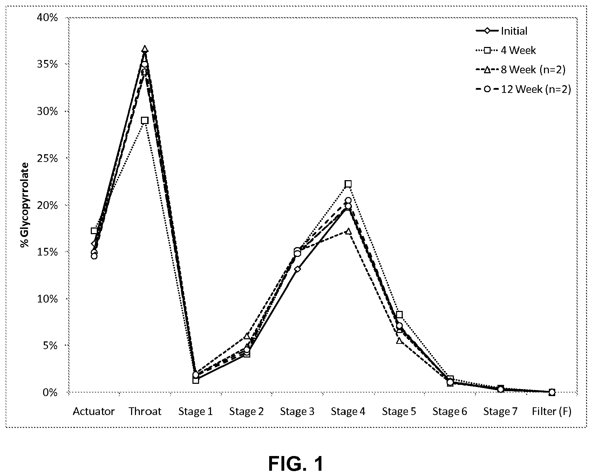

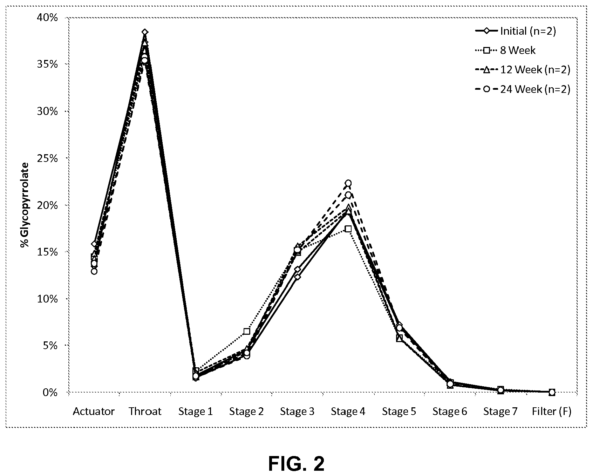

The delivered dose uniformity through use was tested at the 1, 2 and 6 month time points. All individual delivered doses were within ±20% of mean. Figures 1 and 2 show the aerosol particle size distributions as measured by the NGI for configurations 2A and 2B, respectively. Also shown are the amounts of drug recovered from actuator, and from the induction port (throat) and its mouth piece adaptor. Recovered masses are expressed as percent of nominal dose. For configuration 2A, aerodynamic particle size distribution individual replicates are shown at 4, 8 and 12weeks and at 8, 12 and 24 week for configuration 2B. Though there is a measureable fraction of the suspended GP dissolved in the propellant, there is no evidence of a coarsening of the size distributions. Moreover, as evidenced by these Examples, the aerosol performance of a co-suspension at suitable suspending particle to GP ratios is determined largely by the suspending particles.

### Example 3

Several similar batches of suspending particles were made as described in Example 1. The suspending particles were combined with glycopyrrolate (GP) particles that were micronized to different extents, using two different types of jet mills with various milling parameters. The optical diameter and particle size distribution of the micronized GP particles was determined by laser diffraction. Table 3 lists the d₅₀ and d₉₀ values for the different lots of micronized material used. d₅₀ and d₉₀ denote the particle size at which the cumulative volume distribution reported by the particle sizing instrument reaches 50% and 90% respectively.

Twelve different lots of metered dose inhalers were prepared as described in Example 1. In all cases the suspension concentration of GP particles in HFA 134a was in the range of 0.32 - 0.45 mg/mL and the suspension concentration of the suspending particles was in the range of 5.8 - 6.1 mg/mL. The configurations were deemed similar enough to pool the data for a meta-analysis presented in this Example.

The filled metered dose inhalers were stored valve down without overwrap at two different conditions: refrigerated at 5 °C and controlled room temperature at 25 °C / 60% RH. Aerosol performance tests as described in Example 1 were carried out at different time points. The results did not show any statistically significant trend as a function of time up to twelve weeks of storage. No difference between room temperature storage and refrigerated storage was discernible. Hence, results from different stress conditions and time points were pooled to determine how the particle size distribution of the micronized material affects aerosol performance.

Table 3 summarizes the MMAD results of the meta-analysis. The first column describes the six different configurations. The second column identifies how many individual lots were used in the compilation of the data for the respective configuration. The third column lists the number of individual MMAD determinations used to calculate the average MMAD for the respective configuration. Columns four and five show the d₉₀ and d₅₀ of the micronized material used to manufacture the co-suspensions. The results are sorted by d₉₀ value from coarse to fine. The last two columns display the average MMAD and standard deviation.

**Table 3: Pooled MMAD results for 12 glycopyrrolate co-suspensions, sorted by the d₉₀ of the micronized glycopyrrolate particles.**

| Lot ID | number of lots | Number of MMAD measurements | d₉₀ (µm) | d₅₀ (µm) | Average MMAD (µm) | SD |
|---|---|---|---|---|---|---|
| 3A | 3 | 21 | 5.0 | 1.8 | 4.0 | 0.28 |
| 3B | 2 | 9 | 4.9 | 2.1 | 4.1 | 0.37 |
| 3C | 1 | 6 | 4.8 | 1.8 | 3.6 | 0.12 |
| 3D | 1 | 4 | 4.3 | 1.7 | 3.5 | 0.22 |
| 3E | 3 | 20 | 4.1 | 1.6 | 3.7 | 0.28 |
| 3F | 2 | 10 | 3.5 | 1.7 | 3.6 | 0.10 |

These results show a weak dependence of MMAD on the dgₒ of the micronized material. A similar analysis for the d₅₀ showed no statistically significant trend. It can be concluded that changes in the size distribution of the micronized material (e.g., different micronized material lots, or induced by solution mediated transformations) lead to only minor differences in the size distribution of the aerosol emitted from the metered dose inhaler.

### Example 4

Micronized glycopyrrolate (GP) particles were formed tested as described in Example 1. The optical diameter of the micronized GP particles was determined and 50% by volume of the micronized GP particles were smaller than 1.7 µm, 90% by volume were smaller than 3.8 µm.

Five batches of suspending particles were made as described in Example 1. The batches differed in concentration, C_{F}, and volume fraction of PFOB, V_{PFOB}, of the feed emulsion prior to spray drying, ranging from 20 mg/mL to 160 mg/mL and 20% to 40%, respectively. The different configurations are described in Table 4.

Metered dose inhalers were prepared by weighing the target masses of micronized GP and suspending particles into coated glass vials with 15 mL volume. The target suspension concentrations and suspending particle to GP ratios are given in Table 4 for the 26 different vials tested. The canisters were crimp sealed with 63 µl valves (Valois, Les Vaudreuil, France) and filled with 10 g or 12 g of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes.

As described in Example 1, micronized GP particles formulated alone flocculated and sedimented quickly. The glass vials in this example were left to settle for at least 24 h without agitation and then it was tested by visual observation whether the crystal, GP particles were co-suspended completely. For the vials marked with "Yes" in Table 4, no GP particles were observed at the bottom of the vials, except for very few foreign particulates in some vials. Occasional foreign particles were also visible in a similar very low amount in vials filled with suspending particles only. For the vials marked "Partial," a fraction of the GP particles was visible at the bottom of the vial.

**Table 4: Co-suspension observations for glycopyrrolate configurations with various suspending particle to glycopyrrolate particle ratios.**

| | C_{F} in mg/mL | V_{PFOB} (%) | C_{S} (mg/mL) | Suspending particle to glycopyrrolate particle ratio | Co-suspension |
|---|---|---|---|---|---|
| # | | | Suspending particle | | |
| 4A | 20 | 40 | 1.8 | 3.8 | Partial |
| | 20 | 40 | 7.2 | 15 | Yes |
| 4B | 40 | 40 | 3.0 | 1.9 | Partial |
| | 40 | 40 | 1.8 | 3.8 | Partial |
| | 40 | 40 | 3.0 | 3.8 | Yes |
| | 40 | 40 | 6.0 | 3.8 | Yes |
| | 40 | 40 | 9.0 | 5.6 | Yes |
| | 40 | 40 | 3.0 | 7.5 | Yes |
| | 40 | 40 | 6.0 | 7.5 | Yes |
| | 40 | 40 | 9.0 | 11.3 | Yes |
| | 40 | 40 | 6.0 | 15 | Yes |
| | 40 | 40 | 7.2 | 15 | Yes |
| | 40 | 40 | 9.0 | 22.5 | Yes |
| 4C | 80 | 20 | 3.0 | 1.9 | Partial |
| | 80 | 20 | 3.0 | 3.8 | Partial |
| | 80 | 20 | 6.0 | 3.8 | Yes |
| | 80 | 20 | 9.0 | 5.6 | Yes |
| | 80 | 20 | 3.0 | 7.5 | Yes |
| | 80 | 20 | 6.0 | 7.5 | Yes |
| | 80 | 20 | 9.0 | 11.3 | Yes |
| | 80 | 20 | 6.0 | 15 | Yes |
| | 80 | 20 | 9.0 | 22.5 | Yes |
| 4D | 80 | 40 | 1.8 | 3.8 | Partial |
| | 80 | 40 | 7.2 | 15 | Yes |
| 4E | 160 | 40 | 1.8 | 3.8 | Partial |
| | 160 | 40 | 7.2 | 15 | Yes |

### Example 5

Glycopyrrolate (GP) particles were micronized with a jetmill and tested as described in Example 1. 50% by volume of the micronized particles exhibited an optical diameter smaller than 1.7 µm, 90% by volume exhibited an optical diameter smaller than 4.4 µm.

Six batches of suspending particles were made by spray drying as described in Example 1. Configuration 5A was spray dried from an emulsion. Configuration 5B was manufactured in a similar fashion but using dipalmitoylphosphatidylcholine (DPPC) instead of DSPC. Configuration 5C was spray dried from an ethanolic solution. For configurations 5D, 5E, and 5F, saccharides were spray dried from aqueous solution. The spray drying parameters for all configurations are given in Table 5a.

**Table 5a: Suspending particle configurations used in Example 5.**

| Lot # | Powder composition (% w/w) | Feed composition (%v/v) | C_{F} (mg/mL) | Spray Drying Parameters | | | |
|---|---|---|---|---|---|---|---|
| | | | | Feed rate (mL/min) | Tᵢₙ (°C) | Tₒᵤₜ (°C) | Total Gas Flow (L/min) |
| 5A | 93.5 % DSPC | 80 % H₂0 | 40 | 2.4 | 95 | 72 | 526 |
| | 6.5 % CaCI2 | 20 % PFOB | | | | | |
| 5B | 92.9 % DPPC | 70 % H₂0 | 60 | 2.4 | 95 | 67 | 525 |
| | 7.1 % CaCl₂ | 30 % PFOB | | | | | |
| 5C | 100 % DSPC | 95 % Ethanol | 100 | 5 | 95 | 70 | 520 |
| | | 5 % PFOB | | | | | |
| 5D | 100 % Lactose | 100 % H₂0 | 100 | 4 | 95 | 70 | 668 |
| 5E | 100 % Trehalose | 100 % H₂0 | 10 | 2.4 | 100 | 68 | 527 |
| 5F | 100 % Trehalose | 100 % H₂0 | 89 | 4 | 100 | 71 | 670 |

The particle size distribution of the suspending particles was determined by laser diffraction. The volume median optical diameter, VMD, and geometric standard deviation, GSD, for the different configurations are given in Table 5b.

**Table 5b: Characteristics of suspending particle configurations used in Example 5.**

| Lot # | VMD (µm) | GSD | Separation | Co-suspension | Comment |
|---|---|---|---|---|---|
| 5A | 3.6 | 1.8 | Creams | Yes | No or few crystals visible on bottom of vials |
| 5B | 3.6 | 1.8 | Creams | Yes | |
| 5C | 1.2 | 1.9 | Creams | Partial | |
| 5D | 1.7 | 2.3 | Sediments | Yes | Causes GP crystals to sediment with the suspending particles |
| 5E | 0.9 | 1.7 | Sediments | Yes | |
| 5F | 1.7 | 2.4 | Sediments | Yes | |

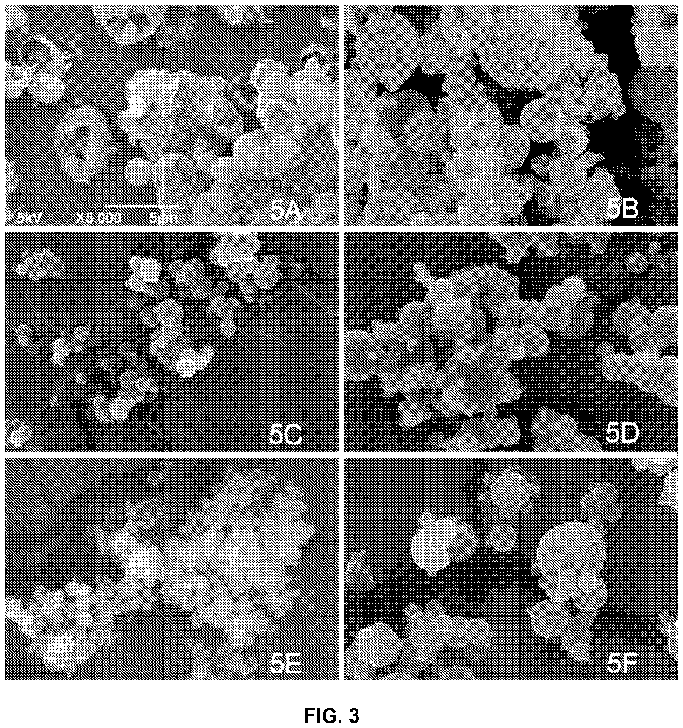

Electron micrographs of the suspending particles showed a variety of morphologies, summarized in Figure 3. The particles that were spray dried from emulsion, 5A and 5B, had high porosity and low density. The DSPC particle spray dried from an ethanolic solution, 5C, showed a much smaller particle size with no noticeable porosity, indicating a high density. All saccharides produced smooth particles with no visible porosity. Configuration 5E had the smallest particles, as expected due to its low feed concentration.

Metered dose inhalers were prepared by weighing the 4 mg of micronized GP particles and 60 mg of suspending particles into coated glass vials with 15 mL volume. The canisters were crimp sealed with 63 µl valves (Valois DF30/63 RCU, Les Vaudreuil, France) and filled with 9.5 mL of HFA 134a (Ineos Fluor, Lyndhurst, UK) by overpressure through the valve stem. After injecting the propellant, the canisters were sonicated for 15 seconds and agitated on a wrist action shaker for 30 minutes. Additional inhalers with suspending particles only were manufactured as control for each configuration.

The suspending particles in Examples 5A, 5B, and 5C, have true densities lower than the propellant. They formed a cream layer and were tested for the presence of a co-suspension as described in Example 4. No GP particles were visible at the bottom of the vials for configuration 5A and 5B. Configuration 5C formed a partial co-suspension.

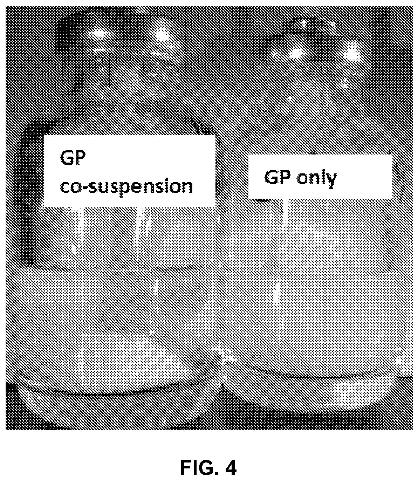

The saccharide particles sediment because they have a higher true density than the propellant. However, all control vials for the saccharide configurations showed a significantly faster sedimentation rate than micronized GP particles alone. In configurations 5D, 5E, and 5F, the sedimentation rate was similar to that of the control vials with the suspending particles alone and faster than the micronized GP particles alone, demonstrating the association of the GP crystals with the suspending particles. A co-suspension was formed in these cases. Figure 4 shows an example of this behavior for configuration 5D. The glass vial was observed one minute after agitation. The co-suspension has already settled leaving a clear propellant layer, while in the control containing GP particles alone, most of the crystals are still suspended in the propellant.

### Example 6

Glycopyrrolate (GP) was micronized using a jet mill to a volume median optical diameter (d₅₀) of 1.4µm with 90% of the cumulative distribution (d₉₀) having a volume optical diameter below 3.0µm. Suspending particles were manufactured similarly to those in Example 1. MDI canisters were manufactured using FEP coated canisters (Presspart, Blackburn, UK) to provide products with metered dose of 5.5 µg per actuation GP and 44 µg per actuation GP which correlates to approximately 4.5 µg per actuation and 36 µg per actuation GP delivered dose from a 50 µl EPDM valves (Bespak, King's Lynn, UK). The formulations contained 6mg/mL of suspending particles. MDI manufacturing was accomplished using a drug addition vessel (DAV) by first adding half of suspending particle quantity, next filling the microcrystalline GP, and lastly adding the remaining half of suspending particles to the top. Materials were added to the vessel in a humidity controlled environment of <10% RH. The DAV was then connected to a 4 L suspension vessel and flushed with HFA 134a propellant and then mixed. The temperature inside the vessel was maintained at 21-23 °C throughout the entire batch production. After recirculation of the batch for 30 min canisters were filled with the suspension mixture through the valve. Sample canisters were then selected at random for total canister assay to ensure correct formulation quantities. The freshly manufactured co-suspension MDI batch was then placed on one week quarantine before initial product performance analysis. In addition, canisters from each lot were subjected to a temperature cycling stability study. The canisters were subjected to -5 °C and 40 °C alternating between temperatures every 6 hours for a total duration of 84 cycles (3 weeks) and 168 cycles (6 weeks).

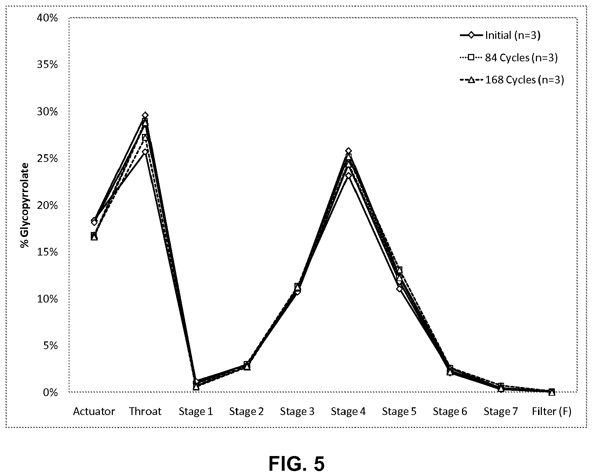

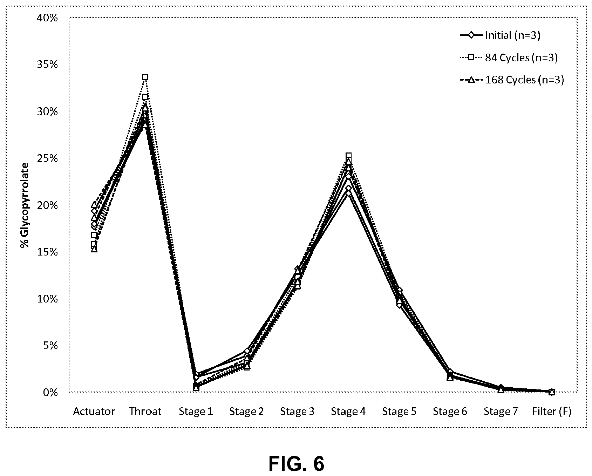

Each lot was tested for delivered dose uniformity through can life and aerodynamic particle size distribution by Next Generation Impactor (NGI) in accordance to USP <601>. The initial and temperature cycled aerodynamic particle size distributions as measured by the NGI are shown in Figures 5 and 6. Also shown are the amounts of drug recovered from valve stem and actuator (denoted as actuator), and from the induction port (throat) and its mouth piece adaptor. Recovered masses are expressed as percent of nominal dose. After 168 cycles, the % FPF (ex-actuator) is not significantly different from initial. A summary of the stability of the fine particle fraction is shown in Table 6. The fine particle fraction remained unchanged over 168 cycles, illustrating the stability of the GP co-suspensions disclosed herein across a GP dose range.

**Table 6. Temperature Cycling Stability of the Fine Particle Fraction of crystalline GP co-suspended with suspending particles at two doses in MDI containing HFA 134a**

| Time | 4.5 µg per actuation (%FPF ex-actuator) | 36 µg per actuation (%FPF ex-actuator) |
|---|---|---|
| Initial | 60.9 | 57.4 |
| 3 Weeks (84 cycles) | 61.9 | 58.0 |
| 6 Weeks (168 cycles) | 60.6 | 59.0 |

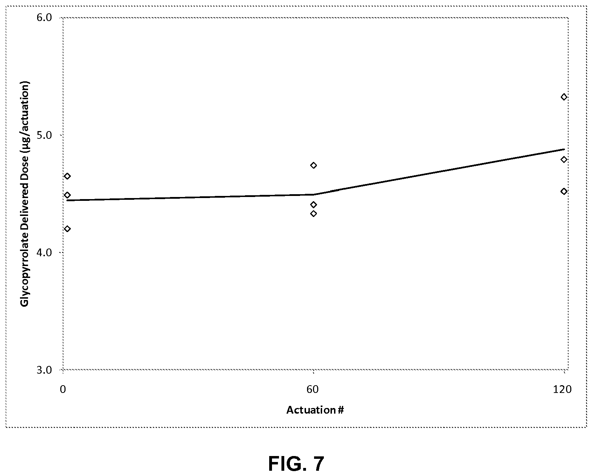

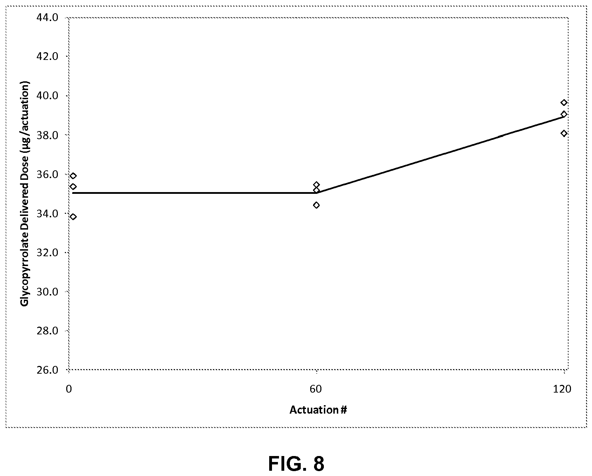

The delivered dose through life of the MDI canisters is shown in Figures 7 and 8. No change in delivered dose from beginning to middle of can is observed and a -10% increase from middle to end of canister. The change from middle to end is anticipated based upon evaporative losses of propellant as the can is emptied. Figures 7 and 8 demonstrate desirable delivered dose uniformity for MDI for doses as low as 4.5 µg per actuation.

### Example 7

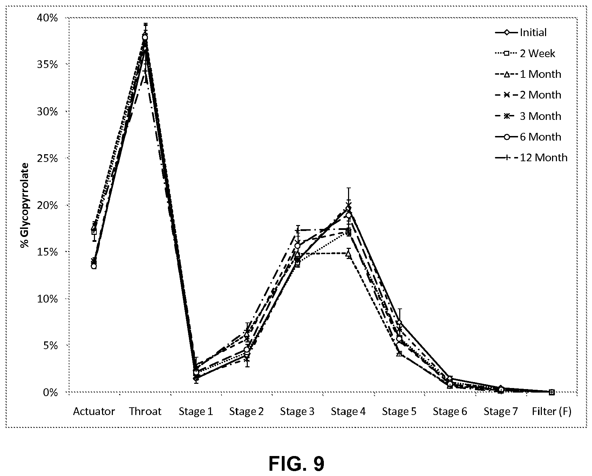

MDI Canisters were manufactured to contain 6mg/mL suspending particle concentration and to provide a metered dose of 36 µg per actuation with a 50µl valve volume according to Example 6. Micronized GP had a d₅₀ and d₉₀ of 1.6µm and 4.1µm respectively and suspending particles were manufactured similarly to the process described in Example 1. The canisters were stored without protective packaging at 25°C and 60% RH and stored for duration of 12 months. Each lot was tested for delivered dose uniformity through can life and aerodynamic particle size distribution by Next Generation Impactor (NGI) in accordance to USP <601>. Aerodynamic particle size distribution was determined by next generation impaction at 2 weeks, 1, 2, 3, 6 or 12 months. The fine particle fraction, as a percentage of GP ex-actuator, at initial sampling was 50.2%. No significant change in the fine particle fraction was noted at any of the throughout 12 months of storage at 25 °C and 60% RH without aluminum foil overwrap, with FPF of 47.7% after 12 months. Figure 9 provides a view of the entire aerodynamic size distribution for each of the stability samples demonstrating desirable consistency on aerosol delivery. A summary of the fine particle fraction is shown in Table 7.

**Table 7. Stability of the Fine Particle Fraction of crystalline GP co suspended with suspending particles in MDI containing HFA 134a stored at 25°C and 60%RH with no protective packaging**

| **Time-Point** | **% FPF (ex actuator)** |
|---|---|
| Initial | 50.2 |
| 2 Week | 46.1 |
| 1 Month | 42.0 |
| 2 Month | 46.0 |
| 3 Month | 48.9 |
| 6 Month | 47.7 |
| 12 Month | 47.7 |

### Example 8

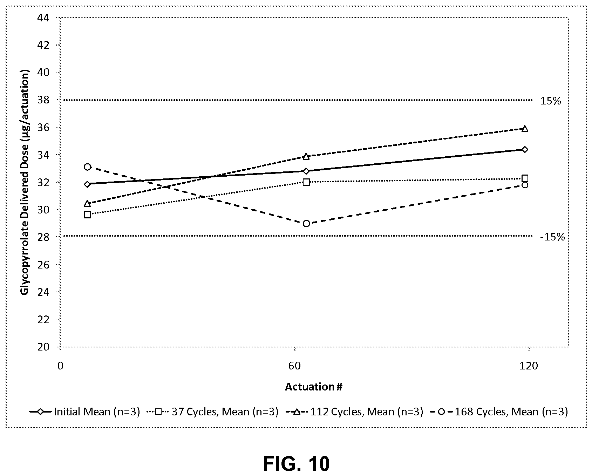

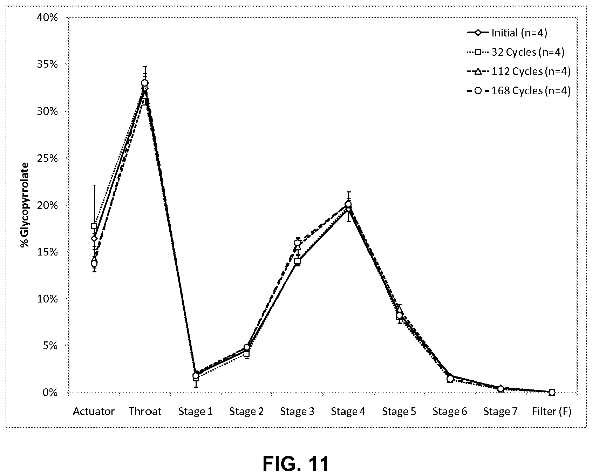

Glycopyrrolate MDI canisters containing 36 µg per actuation were prepared as described in Example 6, packaged in a heat sealed aluminum foil overwrap containing desiccant, and cycled for 6 weeks (6 hours at -5 °C and 6 hours at 40 °C). The delivered dose uniformity of glycopyrrolate through use was tested at the 0, 2, 4 and 6 weeks time points. The mean glycopyrrolate delivered dose of each lot each time period was within ±15% of the mean, with one exception, as demonstrated in Figure 10. The aerodynamic particle size distribution as measured by NGI remain unchanged after 168 temperature cycles as shown in Figure 11.

### Example 9

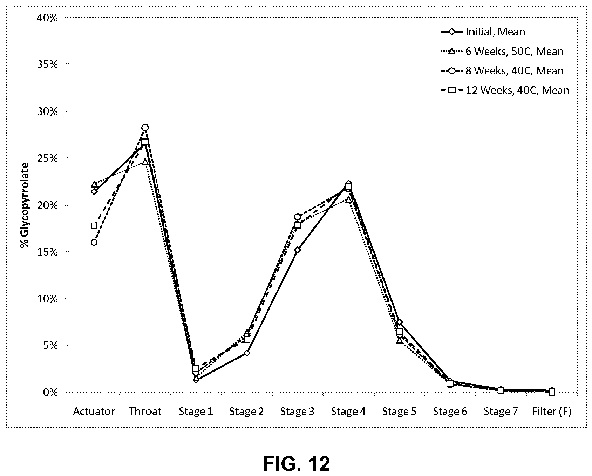

Glycopyrrolate MDI canisters containing 24 µg per actuation were prepared as described in Example 6 were stored for six weeks at 50 °C under ambient humidity. Another lot was stored for 8 weeks at 40 °C and 75% relative humidity. Yet another lot was stored for 12 weeks at 40 °C and 75% relative humidity. The initial fine particle fraction (FPF) was 59.3%. The FPF, 58.4%, of the canister stored for 6 weeks at 50 °C was unchanged compared to the initial. The initial FPF of a lot stored at 40 °C remained unchanged after 8 and 12 weeks, FPF 56.8 and 57.6% respectively. The aerodynamic particle size distributions as measured by the NGI are shown in Figure 12. The MMAD remains relatively unchanged after 6 weeks at 50 °C, 3.94 µm, and up to 12 weeks at 40 °C, 3.84 µm, compared to the initial at 3.54 µm. In addition, the FPF and the amounts of glycopyrrolate recovered from valve stem and actuator, and from the induction port (throat) and its mouth piece adaptor, remained relatively unchanged over 3 months at elevated temperatures as shown in Figure 12.

### Example 10 (reference)

Metered dose inhalers including pharmaceutical compositions of formoterol fumarate as described herein were prepared. Formoterol fumarate, (±)-2-hydroxy-5-[(1 RS)-1 -hydroxy-2-[[(1RS)-2-(4-methoxyphenyl)-1-methylethyl]-amino]ethyl] formanilide fumarate, also known as (±)-2'-hydroxy-5'-[(RS)-1-hydroxy-2-[[(RS)-p-methoxy-α-methylphenethyl]-amine]ethyl] formanilide fumarate, dihydrate was micronized to form active agent particles. The particle size distribution of the micronized formoterol fumarate (FF) was determined by laser diffraction. 50% by volume of the micronized particles exhibited an optical diameter smaller than 1.6 µm, and 90% by volume exhibited an optical diameter smaller than 3.9 µm.

Suspending particles were manufactured as follows: 503 mL of a fluorocarbon-in-water emulsion of PFOB (perfluorooctyl bromide) stabilized by a phospholipid was prepared. 20.6 g of the phospholipid, DSPC (1,2-disteroyl-sn-glycero-3-phosphocholine), and 1.9 g of calcium chloride were homogenized in 403 mL of hot water (75°C) using a high shear mixer. 100 mL of PFOB were added slowly during homogenization. The resulting coarse emulsion was then further homogenized using a high pressure homogenizer (Model C3, Avestin, Ottawa, CA) at pressures of up to 170 MPa for 5 passes.

The emulsion was spray dried in nitrogen using the following spray drying conditions: Inlet temperature 95°C, outlet temperature 71°C, emulsion feed rate 2.4 mL/min, total gas flow 498 L/min. The particle size distribution of the suspending particles was determined by laser diffraction. 50% by volume of the suspending particles were smaller than 3 µm, the geometric standard deviation of the distribution was 1.9.

Metered dose inhalers were prepared by weighing the target masses of micronized active agent particles and suspending particles into coated glass vials with 15 mL volume. The target masses and the target delivered dose assuming 20% actuator deposition are given in Table 8 for three different configurations. For each configuration, additional glass bottles were filled with the respective amount of FF active agent particles without any suspending particles. The canisters were crimp sealed with 63 µl valves (Valois, Les Vaudreuil, France) and filled with 11 g (9.1 mL at 25 °C) of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes.

**Table 8: Target doses for formoterol fumarate co-suspensions of Example 10**

| Configuration # | FF Active Agent Particles µg/can | Suspending Particles mg/can | Target delivered dose µg | Suspending Particle to active particle ratio |
|---|---|---|---|---|
| 6A | 300 | 50 | 1.7 | 167 |
| 6B | 860 | | 4.6 | 58 |
| 6C | 3010 | | 16.5 | 16.6 |

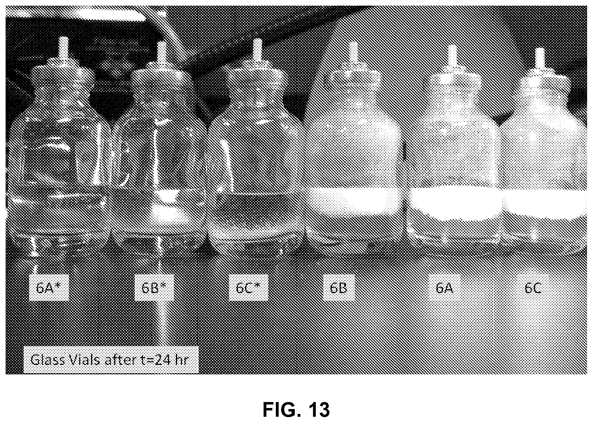

Visual observation of the co-suspended configurations (6A, 6B, 6C) showed no sedimentation of the crystalline FF forming the active agent particles. The suspension flocculated slowly and formed a homogeneous, single cream layer. For all concentrations tested the micronized active agent particles alone sedimented quickly. Pictures of the co-suspension and the traditional comparator suspensions, indicated by an asterisk, are shown in Figure 13. The vials were left to settle for 24 h without agitation. No FF crystals were visible at the bottom of any of the co-suspension vials.

The results showed that the FF crystals associated with the suspending particles. The association between FF particles and suspending particles was strong enough to overcome buoyancy forces, as FF particles did not separate from the suspending particles and settling of the active agent particles was successfully inhibited in each of the three different formulation configurations.

### Example 11 (reference)

Formoterol fumarate MDI compositions were prepared according to the present invention. Micronized formoterol fumarate was commercially obtained and its particle size distribution measured as described in Example 1 was characterized by a d₁₀, d₅₀, d₉₀ or 0.6, 1.9 and 4.4 µm respectively and a Span of 2.0. Suspending particles used were prepared in a similar manner described in Example 1. MDI manufacturing was accomplished using a drug addition vessel (DVA) by first adding half of suspending particle quantity, next filling the microcrystalline FF, and lastly adding the remaining half of suspending particles to the top. Materials were added to the DAV in a humidity controlled environment of <10% RH. The DAV was then connected to a 4 L suspension vessel. A slurry was then formed by adding a known amount of HFA-134a propellant (Ineos Fluor, Lyndhurst, UK) into the DAV, which is then removed from the suspension vessel and gently swirled. The slurry is then transferred back to the suspension mixing vessel and diluted with additional HFA-134a to form the final suspension at target concentration stirring gently with an impeller. The temperature inside the vessel was maintained at 21-23 °C throughout the entire batch production. After recirculation of the batch for 30 min, 14-mL fluorinated ethylene polymer (FEP) coated aluminum canisters (Presspart, Blackburn, UK) were filled with the suspension mixture through 50 µL EPDM valves (Bespak, King's Lynn, UK). Sample canisters were then selected at random for total canister assay to ensure correct formulation quantities.

The freshly manufactured co-suspension MDI batch was then placed on one week quarantine before initial performance analysis. Aerosol performance was assessed in accordance with USP <601> (United States Pharmacopeia monograph 601). A Next Generation Impactor (NGI) operated at a flow rate of 30 L/min was used for determination of particle size distribution. Sample canisters were seated into an actuator with two waste actuations and two additional waste priming actuations. Five actuations were collected in the NGI with a USP throat attached. The valve, actuator, throat, NGI cups, stages, and filter were rinsed with volumetrically dispensed solvent. The sample solutions were assayed using a drug specific chromatographic method. The fine particle fraction was defined using the sum of stages 3 through filter. Delivered dose uniformity through use testing was performed using a Dose Uniformity Sampling Apparatus as described by USP <601>.Two actuations were collected and assayed at beginning, middle and end of use.

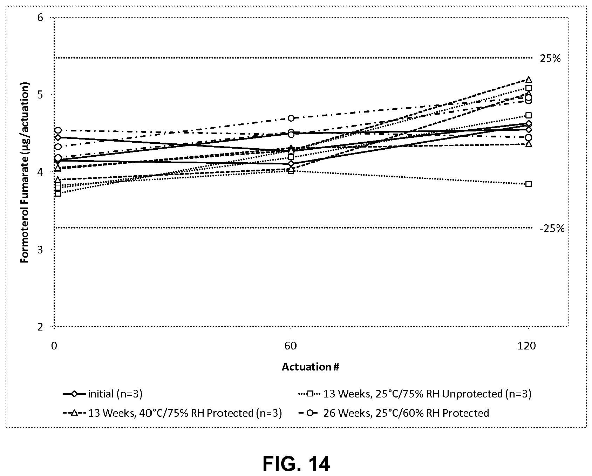

Figure 14 shows the delivered dose uniformity for a co-suspension of FF at a 4.8 µg target dose per actuation. The individual delivered dose per actuation for beginning, middle and end of actuations was within ±25% of the mean delivered dose, as demonstrated in Figure 14.

### Example 12 (reference)

Formoterol Fumarate MDI compositions were prepared according to the present invention. Micronized formoterol fumarate was commercially obtained and its particle size distribution measured as described in Example 1 was characterized by a d₁₀, d₅₀, d₉₀ or 0.6, 1.9 and 4.4 µm respectively and a Span of 2.0. Suspending particles used were prepared in a similar manner described in Example 1. MDI manufacturing was accomplished as described in Example 11.

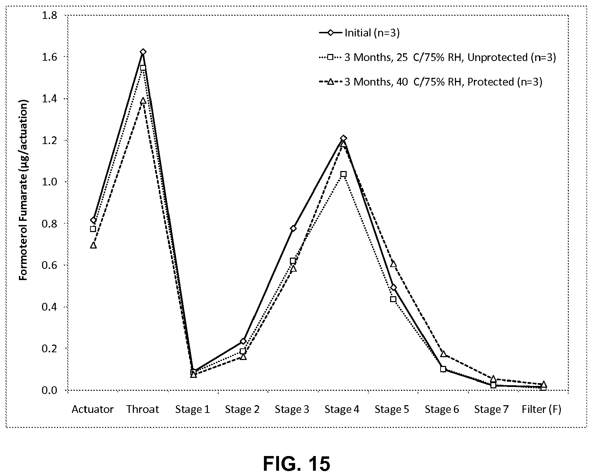

Aerosol performance was assessed in accordance with USP <601>. A Next Generation Impactor (NGI) operated at a flow rate of 30 L/min was used for determination of particle size distribution. Sample canisters were seated into an actuator with two waste actuations and two additional waste priming actuations. Five actuations were collected in the NGI with a USP throat attached. The valve, actuator, throat, NGI cups, stages, and filter were rinsed with volumetrically dispensed solvent. The sample solutions were assayed using a drug specific chromatographic method. The fine particle fraction was defined using the sum of stages 3 through filter. The aerodynamic particle size distribution of a FF co-suspension formulation was evaluated after manufacture and after three months of storage at 25 °C and 75%RH (unprotected canisters) and 40 °C and 75 %RH (protected canisters wrapped in aluminum foil pouch). The aerodynamic particle size distributions shown in Figure 15 demonstrate that the compositions described displayed desirable stability characteristics even at accelerated conditions.

### Example 13 (reference)

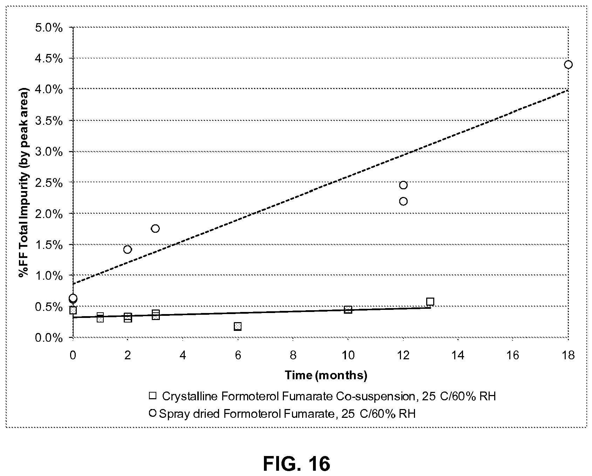

The chemical stability of formoterol fumarate (FF) included in a co-suspension formulation prepared according Example 11 was evaluated. FF MDI canisters containing HFA 134a were overwrapped with an aluminum foil pouch and stored at 25 °C and 60% relative humidity and 40 °C and 75% relative humidity for thirteen and six months, respectively. Likewise FF MDI canisters containing HFA 227ea were overwrapped with an aluminum foil pouch and stored at 25 °C and 60% relative humidity and 40 °C and 75% relative humidity for six months. The amount of impurity F, a characteristic degradation product of FF, and total impurities were determined by reverse phase HPLC assay as follows: each canister is chilled, cut open, and the can contents are transferred to a centrifuge tube; the contents were dissolved in organic solvent, followed by the addition of an aqueous solvent to precipitate excipient (DSPC) from the solution; the solution was centrifuged to produce a clear supernatant solution; and each sample solution was analyzed using a C18 column, 4.6 x 150 mm and 3.0 µm particle size. The column temperature was kept at 30 °C. The injection volume was 20 µl, and flow rate was set at 1 mL/min and detected by determining the UV absorption at 214 nm. A gradient was used mixing pH 3.1 aqueous phosphate buffer and acetonitrile, 17% acetonitrile first 27 minutes, then 50% acetonitrile for 30 seconds followed by 6.5 minutes at 75% acetonitrile and 17% acetonitrile for 8 minutes. Impurities were reported as area percent of formoterol peak area (corrected for relative response factors, where available). As shown in Figure 16 (or Table 9 and 10), a co-suspension prepared using crystalline FF active agent particles suspended in HFA 134a with suspending particles was chemically stable for 18 months at a temperature of 25°C and 60% relative humidity, in contrast a spray dried, non co-suspended, formoterol formulation, showed a faster degradation rate under the same storage conditions. Likewise, crystalline FF active agent particles formed a chemically stable co-suspension in HFA 227a, as shown in Table 11.

**Table 9. Chemical Stability of Spray Dried FF Suspending Particles in FF MDI Containing HFA 134a at 25°C/60%RH, Overwrapped in Aluminum Foil Pouches**

| **Time (months)** | **0** | **2** | **3** | **12** | **18** |
|---|---|---|---|---|---|
| Impurity F (%) | ND | 0.12% | 0.04% | 1.16% | 2.77% |
| Total Impurities (%) | 0.62% | 1.42% | 1.75% | 2.33% | 4.39% |

| | | | | | |
|---|---|---|---|---|---|
| ND= Not detected | | | | | |

**Table 10. Chemical Stability of Crystalline FF Co-suspended with Suspending Particles in FF MDI Containing HFA 134a at 25°C/60%RH, Overwrapped in Aluminum Foil Pouches**

| **Time (months)** | **0** | **1** | **2** | **3** | **6** | **10** | **13** |
|---|---|---|---|---|---|---|---|
| Impurity F (%) | 0.05% | 0.08% | 0.08% | 0.14% | 0.06% | 0.22% | 0.35% |
| Total Impurities (%) | 0.44% | 0.32% | 0.32% | 0.37% | 0.18% | 0.45% | 0.64% |

| at 40°C/75%RH, Overwrapped in Aluminum Foil Pouches | | | | | | | |
|---|---|---|---|---|---|---|---|
| **Time (months)** | **0** | **1** | | **2** | **3** | | **6** |
| Impurity F (%) | 0.05% | 0.11% | | 0.31% | 1.18% | | 1.74% |
| Total Impurities (%) | 0.44% | 0.41% | | 0.75% | 1.58% | | 2.54% |

**Table 11. Chemical Stability of Crystalline FF Co-suspended with Suspending Particles in FF MDI Containing HFA 227a at 25°C/60%RH, Overwrapped in Aluminum Foil Pouches**

| **Time (months)** | **0** | **1** | **2** | **3** | **6** |
|---|---|---|---|---|---|
| Impurity F (%) | 0.04 | 0.06 | 0.07 | 0.13 | 0.05 |
| Total Impurities (%) | 0.4 | 0.3 | 0.3 | 0.4 | 0.1 |

| at 40°C/75%RH, Overwrapped in aluminum foil pouches | | | | | |
|---|---|---|---|---|---|
| **Time (months)** | **0** | **1** | **2** | **3** | **6** |
| Impurity F (%) | 0.04 | 0.08 | 0.18 | 0.80 | 1.14 |
| Total Impurities (%) | 0.40 | 0.39 | 0.53 | 1.13 | 1.56 |

### Example 14 (reference)

Micronized formoterol fumarate dihydrate (FF) (Inke, S.A., Barcelona, Spain) used in the present example had with particle size distribution by laser diffraction of 50% by volume of the micronized particles exhibited an optical diameter smaller than 1.9 µm, 90% by volume exhibited an optical diameter smaller than 4.1 µm. Four batches of suspending particles were manufactured by spray drying as described in Example 1. All four batches were spray-dried from aqueous solution; solution concentration and spray drying parameters are given in Table 12.

**Table 12: Suspending particle configurations used in Example 14**

| # | Powder Composition | C_{f} in mg/mL | Spray Drying Parameters | | | | Particle Size Distribution | |
|---|---|---|---|---|---|---|---|---|
| | | | Feed rate in mL/min | Tᵢₙ in °C | Tₒᵤₜ in °C | Total Gas Flow in std L/min | VMD in µm | GSD |
| XA | 100 % trehalose | 80 | 10 | 150 | 82 | 385 | 1.62 | 2.20 |
| XB | 100 % HP-β-cyclodextrin | 80 | 10 | 100 | 68 | 885 | 1.61 | 2.21 |
| XC | 100% Ficoll PM 70 | 80 | 10 | 100 | 70 | 885 | 1.19 | 2.27 |
| XD | 100% Inulin | 80 | 10 | 100 | 70 | 885 | 1.23 | 2.20 |

The particle size distribution of the suspending particles was determined by laser diffraction. The volume median optical diameter (VMD) and geometric standard deviation (GSD) are given in Table 12.

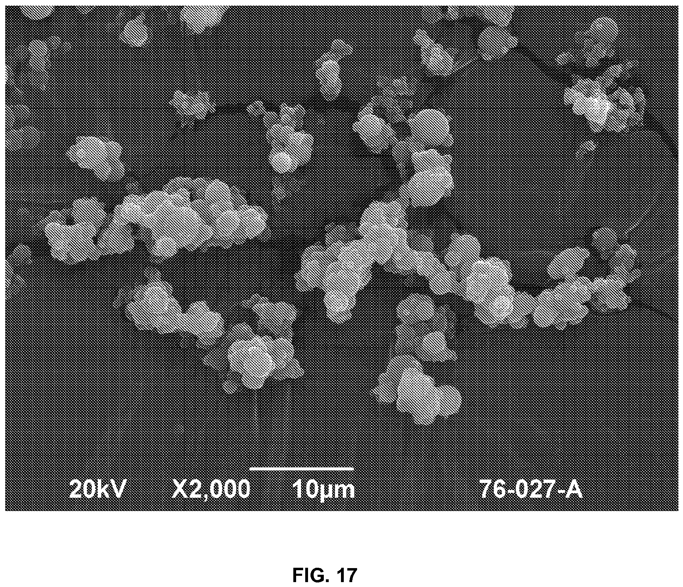

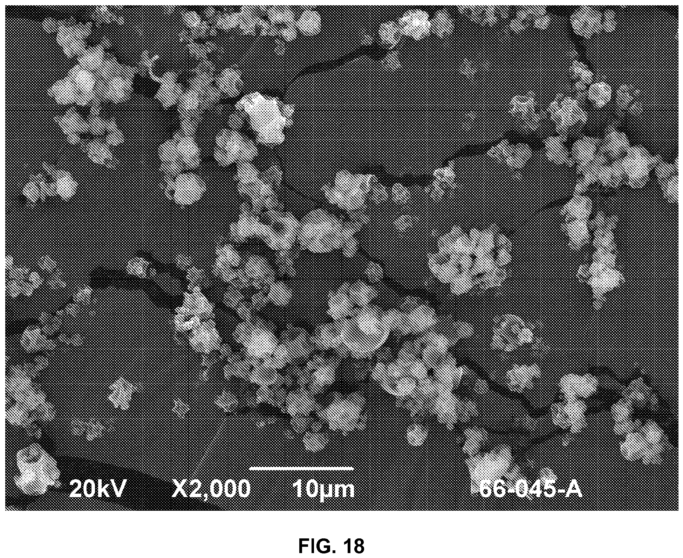

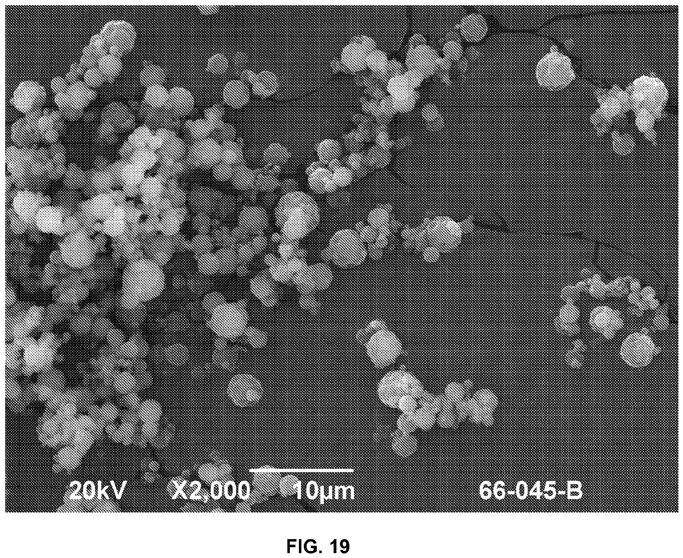

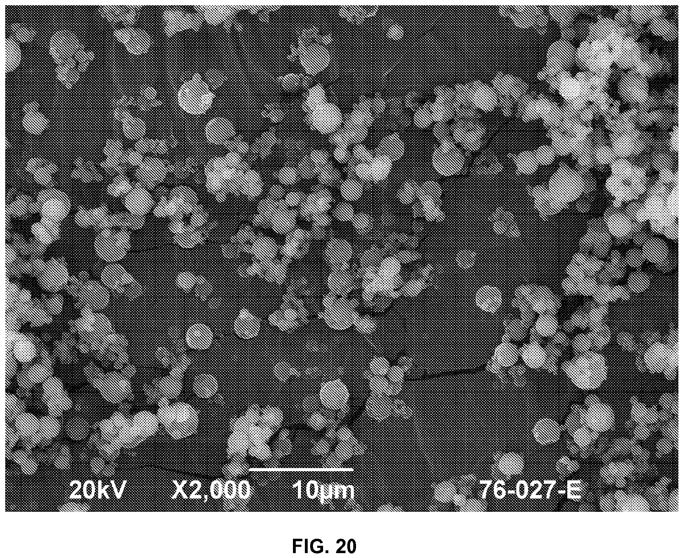

Electron micrographs of the suspending particles showed a variety of morphologies, and are shown in Figure 17 through Figure 20, with Figure 17 providing a micrograph of trehalose suspending particles, Figure 18 providing a micrograph of HP-β-cyclodextrin suspending particles, Figure 19 provding a micrograph of Ficoll MP 70 suspending particles, and Figure 20 providing a micrograph of inulin suspending particles. Trehalose particles appear to be spherical, with a smooth surface. HP-β-cyclodextrin particles show extensive wrinkling of the surface, suggesting a partially buckled exterior with a hollow core. Ficoll MP 70 and Inulin particles display some surface rugosity but are generally spheroidal.

Metered dose inhalers were prepared by weighing 0.9 mg of the micronized FF active agent particles and 60 mg of suspending particles into coated glass vials with 15 mL volume. FF was combined with each type of the four suspending particle species of Table 12. The canisters were crimp sealed with 50 µL valves (Valois DF31/50 RCU, Les Vaudreuil, France) and filled with 10 mL of HFA propellant 134a (Ineos Fluor, Lyndhurst, UK) by overpressure through the valve stem. After injecting the propellant, the canisters were sonicated for 30 seconds and agitated on a wrist action shaker for 30 minutes. Additional inhalers containing suspending particles only and active agent particles only were filled as a control for each configuration.

Crystalline FF has a greater density than propellant 134a at room temperature, as do all four species of suspending particles in the present example. Consequently both FF and suspending particles settled to the bottom of the inhalers at room temperature. To test these inhalers for active-suspending agent particle interactions indicating a co-suspension, the inhalers were immersed in an ethanol bath at ≤ -10 ° C (resulting in increased propellant density) and allowed to equilibrate for a minimum of 30 minutes. At this temperature, the FF active agent particles are less dense than the propellant and consequently cream to the top of the propellant volume, while all four species of suspending agent particles remain settled at the bottom of the propellant volume.

The tested configurations and the results of the observations are presented in Table 13. FF active agent particles alone formed a cream layer atop the propellant volume, and trehalose, HP- β-cyclodextrin, inulin, and Ficoll PM70 particles alone all settled to the bottom of the glass vial. FF active agent particles in combination with trehalose suspending particles formed a single sediment layer, with no particles creamed or afloat in the propellant, indicating that the FF particles interact with the trehalose suspending particles, and a co-suspension is formed. In the case of FF particles in combination with HP-β-cyclodextrin suspending particles, some turbidity was present in the propellant, similar to that observed in the suspending particle only control vial. Additionally, some floating flocs were observed, which may have been FF particles; however, such flocs accounted for a small amount of solid mass relative to the control vial, indicating that some if not all FF particles were interacting with the suspending agent particles. FF particles in combination with inulin suspending particles formed a single sediment layer, indicating a co-suspension was formed. Though some turbidity was present in this configuration, similar cloudiness was observed in the inulin-only control vial. FF active agent particles in combination with Ficoll PM70 suspending particles formed a sediment layer at the bottom of the vial, indicating that a co-suspension was formed. While some turbidity and floating flocs were observed in this configuration, similar turbidity, and floc frequency were observed in the Ficoll-only control vial.

**Table 13: Summary of tested configurations and results of observations**

| Container ID | Contents in 10 mL p134a | Suspending Particle to Active Particle Ratio | Observational Notes, ≤ - 10 ° C | Co-suspension |
|---|---|---|---|---|
| 0-FF | 0.9 mg FF | n/a | Creamed to top | n/a |
| T | 60 mg trehalose | n/a | Settled to bottom | n/a |
| T-FF | 60 mg trehalose, 0.9 mg FF | 67 | Sediment layer; no particles creamed | Yes |
| C | 60 mg HP-β-cyclodextrin | n/a | Settled to bottom; some turbidity | n/a |
| C-FF | 60 mg HP-β-cyclodextrin, 0.9 mg FF | 67 | Solids mostly in sediment layer at bottom; some turbidity; some floating flocs present | partial |
| I | 60 mg Inulin | n/a | Settled to bottom; some turbidity | n/a |
| I-FF | 60 mg Inulin, 0.9 mg FF | 67 | Sediment layer; no particles creamed; some turbidity | Yes |
| F | 60 mg Ficoll PM70 | n/a | Settled to bottom, with some floating flocs | n/a |
| F-FF | 60 mg Ficoll PM70, 0.9 mg FF | 67 | Sediment layer; very few floating flocs | Yes |

### Example 15

Co-suspension compositions including glycopyrrolate (GP) and formoterol fumarate (FF) active agent particles were produced and MDIs incorporating the co-suspension compositions were prepared. The co-suspension compositions produced included GP active agent particles, FF active agent particles or a combination of both GP and FF active agent particles. The GP and FF material was supplied as micronized, crystalline material with particle size distribution as shown in Table 14.

Suspending particles were manufactured via spray dried emulsion at a feed stock concentration of 80 mg/mL with a composition of 93.44% DSPC (1,2-Distearoyl-sn-Glycero-3-Phosphocholine) and 6.56% anhydrous calcium chloride (equivalent to a 2:1 DSPC:CaCl₂ mole/mole ratio). During the emulsion preparation, DSPC and CaCl₂ was dispersed with a high shear mixer at 8000-10000 rpm in a vessel containing heated water (80 ± 3 °C) with PFOB slowly added during the process. The emulsion was then processed with 6 passes in a high pressure homogenizer (10000-25000 psi). The emulsion was then spray dried via a spray dryer fitted with a 0.42" atomizer nozzle with a set atomizer gas flow of 18 SCFM. The drying gas flow rate was set to 72 SCFM with an inlet temperature of 135 °C, outlet temperature 70 °C, and an emulsion flow rate of 58 mL/min.

The co-suspensions were prepared by first dispensing the appropriate quantities of micronized GP and FF active agent particles and suspending particles into a drug addition vessel (DAV) inside a humidity controlled chamber (RH < 5%). In the present Example, the suspending particles were added in three equal portions intercalating the addition of GP and FF after the first and second addition respectively. The DAV is then sealed under a nitrogen atmosphere and connected to the suspension vessel containing 12 kg of HFA-134a (Ineos Fluor, Lyndhurst, UK). A slurry was then formed by adding 0.5-1 kg of HFA-134a into the DAV, which is then removed from the suspension vessel and gently swirled. The slurry is then transferred back to the suspension mixing vessel and diluted with additional HFA-134a to form the final suspension at target concentration stirring gently with an impeller. The suspension is then recirculated via a pump to the filling system for a minimum time prior to initiation of filling. Mixing and recirculation continue throughout the filling process. 50 µL valves (Bespak, King's Lynn, UK) are placed onto 14-mL fluorinated ethylene polymer (FEP) coated aluminum canisters (Presspart, Blackburn, UK) canisters and then purged of air either by a vacuum crimping process, or an HFA-134a purging process followed by valve crimping. The crimped canisters are then filled through-the-valve with the appropriate quantity of suspension, adjusted by the metering cylinder.

**Table 14: Glycopyrrolate and Formoterol Fumarate particle size distributions.**

| **Designation** | **d₁₀ (µm)** | **d₅₀ (µm)** | **d₉₀ (µm)** | **Span** |
|---|---|---|---|---|
| FF API | 0.6 | 1.9 | 4.1 | 1.8 |
| GP API | 0.5 | 1.3 | 3.0 | 1.9 |

MDIs containing the dual co-suspensions described in this Example were prepared to contain two different doses GP and FF. Specifically, a first run of dual co-suspension compositions were prepared to provide 18 µg per actuation GP and 4.8 µg per actuation FF ("low dose"), and a second run of dual co-suspension compositions were prepared to provide 36 µg per actuation GP and 4.8 µg per actuation FF ("high dose"). In addition to the dual co-suspensions compositions, co-suspensions including a single species of active agent particle were prepared. These compositions included either GP active agent particles or FF active agent particles and were referred to as "mono" or "monotherapy" co-suspensions. The monotherapy co-suspension compositions were prepared as described for the dual co-suspensions, except that they included only one species of active agent particles (either GP or FF). The monotherapy co-suspensions were formulated and monotherapy MDIs prepared to provide the following targeted delivered doses: 18 µg per actuation of GP, and 0.5, 1.0, 3.6 or 4.8 µg per actuation of FF. The compositions and MDIs providing 0.5 µg FF and 1 µg FF per actuation are referred to as "ultra low" dose and were manufactured in a similar manner at a 4L scale.

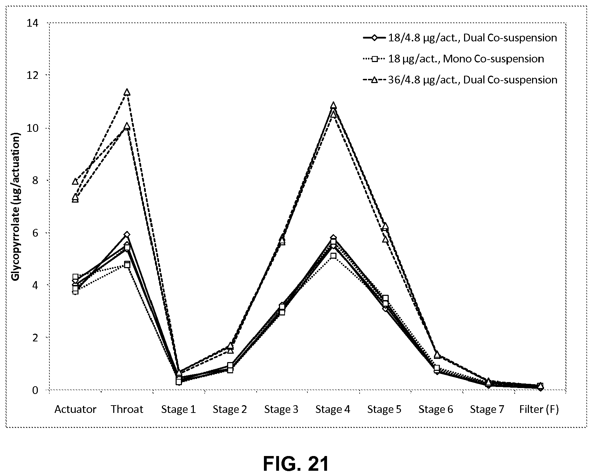

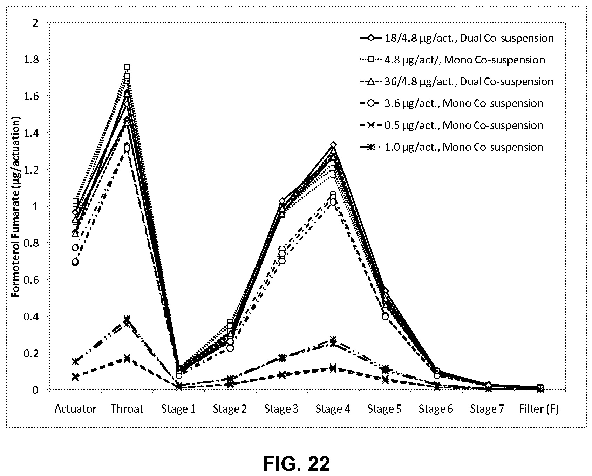

The drug specific aerodynamic size distributions achieved with MDIs containing the co-suspension compositions prepared according to this Example were determined as described in Example 1. The proportionality of the aerodynamic size distributions of GP obtained from the low and high dose dual co-suspensions as well as the equivalency between the dual and monotherapy co-suspensions is demonstrated in Figure 21. In the same manner, the proportionality of the aerodynamic size distributions of FF obtained from the dual and monotherapy co-suspensions, including the ultralow, low, and high dose compositions is demonstrated in Figure 22.

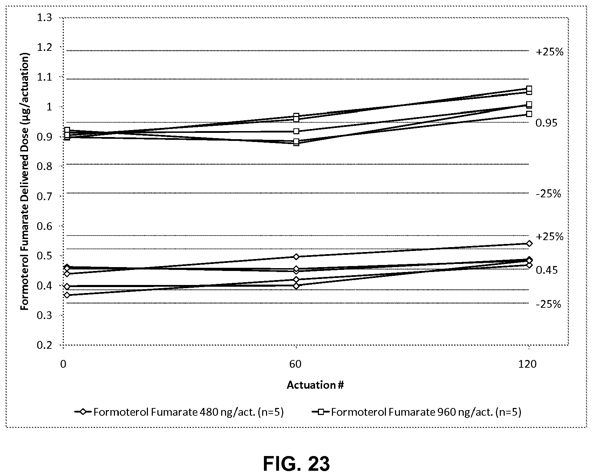

The delivered dose uniformity of the ultra low dose FF monotherapy MDIs was also measured as described in Example 1. The DDU for the FF MDI containing 0.5 µg per actuation and 1.0 µg per actuation are shown in Figure 23. Desirable dose delivery uniformity is achieved even at ultra low doses.

### Example 16 (reference)

Micronized salmeterol xinafoate (4-hydroxy-α1-[[[6-(4-phenylbutoxy)hexyl]amino] methyl]-1,3-benzenedimethanol, 1-hydroxy-2-naphthalenecarboxylate) was received by the manufacturer (Inke SA, Germany) and used as active agent particles. The particle size distribution of the salmeterol xinafoate (SX) was determined by laser diffraction. 50% by volume of the micronized particles exhibited an optical diameter smaller than 2 µm, 90% by volume exhibited an optical diameter smaller than 3.9 µm.

Suspending particles were manufactured as follows: 150 mL of a fluorocarbon-in water emulsion of PFOB (perfluoroctyl bromide) stabilized by a phospholipid was prepared. 12.3 g of the phospholipid, DSPC (1,2-Distearoyl-sn-Glycero-3-Phosphocholine), and 1.2 g of calcium chloride were homogenized in 100 mL of hot water (70 °C) using a high shear mixer. 65 mL of PFOB were added slowly during homogenization. The resulting coarse emulsion was then further homogenized using a high pressure homogenizer (Model C3, Avestin, Ottawa, CA) at pressures of up to 140 MPa for 3 passes

The emulsion was spray dried in nitrogen using the following spray drying conditions: Inlet temperature 90 °C, outlet temperature 69 °C, emulsion feed rate 2.4 mL/min, total gas flow 498 l/min. The particle size distribution of the suspending particles, VMD, was determined by laser diffraction. 50% by volume of the suspending particles were smaller than 2.7 µm, the Geometric Standard Deviation of the distribution was 2.0. Additionally, the aerodynamic particle size distribution of the suspending particles was determined with a time-of-flight particle sizer. 50% by volume of the suspending particles had an aerodynamic particle diameter smaller than 1.6 µm. The large difference between aerodynamic particle diameter and optical particle diameter indicates that the suspending particles had a low particle density < 0.5 kg/L.

Metered dose inhalers were prepared by weighing 2 mg of SX active agent particles and 60 mg of suspending particles into fluorinated ethylene polymer (FEP) coated aluminum canisters (Presspart, Blackburn, UK) with 19 mL volume. The suspending particle to active particle ratio was 30. The target delivered dose assuming 20% actuator deposition was 10 µg. The canisters were crimp sealed with 63 µl valves (# BK 357, Bespak, King's Lynn, UK) and filled with 10 mL of HFA 134a (1,1,1,2-tetrafluoroethane) by overpressure through the valve stem. After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK). Additional inhalers for visual observation of suspension quality were prepared using 15 mL glass vials including a comparator filled with micronized SX only. Aerosol performance was assessed as described in Example 1. The MMAD was 3.7 µm and the fine particle fraction was 48%. Because the SX crystals forming the active agent particles and the propellant were nearly density matched at 15 °C - 20 °C, the visual observation was conducted on glass vials that were heated up to 30 °C - 35 °C in a water bath. Under these conditions the SX active agent particles formulated alone sedimented rapidly, but no SX crystals were visible at the bottom of the co-suspension vial.

Micronized salmeterol xinafoate active agent particles were co-suspended through association with suspending particles of low density that were formulated according to the disclosure provided herein. The association between salmeterol crystals and the suspending particles was strong enough to overcome buoyancy forces as it was observed that settling of the crystals is inhibited.

### Example 17 (reference)

Micronized fluticasone propionate (S-(fluoromethyl)6^{α}, 9-difluoro-11^{β}-17-dihydroxy-16^{α}-methyl-3-oxoandrosta-1,4-diene-17^{β}-carbothioate, 17-propionate) was received as micronized by the manufacturer (Hovione FarmaCiencia SA, Loures Portugal) and used as active agent particles. The particle size distribution of the fluticasone propionate (FP) was determined by laser diffraction. 50% by volume of the micronized particles exhibited an optical diameter smaller than 2.6 µm, 90% by volume exhibited an optical diameter smaller than 6.6 µm.

The suspending particles were the same lot that was used in Example 16, and the manufacture and characteristics of the suspending particles are described there.

Metered dose inhalers were prepared as described in Example 16. Propellant type, fill weights, suspending particle to active particle ratio, and target ex actuator dose for six configurations are listed in Table 15. Additional inhalers for visual observation of suspension quality were prepared using 15 mL glass vials. Two comparator glass vials were filled with micronized FP only in either HFA 134a or HFA 227ea.

**Table 15: Configurations Example 17 and aerosol performance**

| # | HFA | FP fill weight in mg | Suspending particle to active particle ratio | Target ex actuator dose in µg | FPF in % | MMAD in µm |
|---|---|---|---|---|---|---|
| 9A | 134a | 8 | 7.5 | 40 | 34 | 4.1 |
| 9B | 227ea | | | | 31 | 4.6 |
| 9C | 134a | 16 | 3.75 | 80 | 33 | 4.5 |
| 9D | 227ea | | | | 36 | 4.5 |
| 9E | 134a | 30 | 2 | 150 | 30 | 4.9 |
| 9F | 227ea | | | | 31 | 4.9 |

Aerosol performance was assessed as described in Example 1. The results are shown in Table 15. These co-suspensions were made with micronized FP that had a relatively coarse particle size distribution. The MMAD is comparatively large and trends upward with increasing FP concentration, but is still in a range usable for respiratory drug delivery. No significant differences were observed between propellant types.

Visual observation of the co-suspended configurations in HFA 134a, 9A, 9C, and 9E, showed no sedimentation of drug crystals forming the active agent particles. The suspension flocculated slowly and formed a homogeneous, single cream layer. In contrast, micronized FP in HFA 134a sedimented. The test for the configurations in HFA 227ea was conducted at 35-40 °C as described in Example 16, because FP is nearly density matched with this propellant at room temperature. At the elevated temperature, micronized FP active agent particles sedimented in HFA 227ea, but no sedimentation of active agent particles was seen in configurations 9B, 9D, and 9F. The results show that fluticasone propionate forms co-suspensions with suspending particles in both tested propellants, when formulated according to the disclosure provided herein.

### Example 18 (reference)

The formulation of a combination product of salmeterol xinafoate (SX) active agent particles and fluticasone propionate (FP) active agent particles in a co-suspension format is described. Both FP and SX are present in the propellant as a micronized, crystalline particles. The two species of micronized active agent particles are co-suspended with spray dried suspending particles.

The fluticasone propionate and salmeterol xinafoate used were as described in Examples 16 and 17, respectively.

The suspending particles were the same lot that was used in Example 16, and the manufacture and characteristics of the suspending particles are described there.

Metered dose inhalers were prepared by weighing the target masses of micronized fluticasone propionate and salmeterol xinafoate and suspending particles into fluorinated ethylene polymer (FEP) coated aluminum canisters (Presspart, Blackburn, UK) with 19 mL volume. The canisters were crimp sealed with 63 µl valves (# BK 357, Bespak, King's Lynn, UK) and filled with 10 mL of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK). Aerosol performance was assessed shortly after manufacturing in accordance with USP 601 as previously described in Example 1. Results are reported below in Table 16.

**Table 16: Results for a co-suspension of Fluticasone Propionate (FP) and Salmeterol Xinafoate (SX) of Example 18**

| Suspending particle conc. | Target Delivered Dose FP | Target Delivered Dose SX | FP DDU | SX DDU | FP FPF | SX FPF | FP MMAD | SX MMAD |
|---|---|---|---|---|---|---|---|---|
| 5.9 mg/mL | 12 µg | 25 µg | 6.1% RSD* | 6.1% RSD* | 27% | 49% | 4.1 µm | 3.4 µm |

| | | | | | | | | |
|---|---|---|---|---|---|---|---|---|
| *no trend observed | | | | | | | | |

The MMADs of fluticasone propionate active agent particles and salmeterol xinafoate active agent particles were acceptable and similar to the aerosol performance of the respective monotherapy co-suspensions, described in Examples 16 and 17, respectively. The delivered dose uniformity through use was tested and all individual delivered doses were within ±20 % of mean, at 6.1% relative standard deviation.

Visual observation of the co-suspension was conducted in glass vials as described in Example 16. No sedimentation of active agent particles was observed. The suspension flocculated slowly and formed a homogeneous, single cream layer.

### Example 19 (reference)

The formulation of a combination product of salmeterol xinafoate (SX) active agent particles and fluticasone propionate (FP) suspending particles in a co-suspension format is described. SX is present in the propellant as micronized, crystalline active agent particles. It is co-suspended with spray dried suspending particles that incorporate micronized FP. To achieve this, FP crystals are suspended in the feedstock used to manufacture the lipid-based suspending particles.

The fluticasone propionate and salmeterol xinafoate used to form the active agent particles and suspending particles referenced in this example were as described in Examples 16 and 17, respectively.

Fluticasone propionate containing suspending particles were manufactured as follows: 200 mL of a fluorocarbon-in-water emulsion of PFOB stabilized by a phospholipid was prepared. 3.3g of the phospholipid (DSPC) and 0.8g of micronized fluticasone propionate were dispersed and 0.3g of calcium chloride dihydrate was dissolved in 100mL of warm water (70°C) using a high shear mixer. 44mL of PFOB was added slowly during dispersion. This resulting coarse emulsion was then further homogenized using a high pressure homogenizer at 140 MPa for 3 passes. The homogenization reduced the particle size of the suspended FP crystals. The emulsion was spray dried in nitrogen using the following spray drying conditions: inlet temperature 95 °C; outlet temperature 72 °C; emulsion feed rate 2.4 mL/min; and total gas flow 525 L/min.

Metered dose inhalers were prepared by weighing the target masses of micronized salmeterol xinafoate active agent particles and fluticasone propionate containing suspending particles into fluorinated ethylene polymer (FEP) coated aluminum canisters (Presspart, Blackburn, UK) with 19 mL volume. The canisters were crimp sealed with 63 µl valves (# BK 357, Bespak, King's Lynn, UK) and filled with 10 mL of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK). Aerosol performance was assessed shortly after manufacturing in accordance with USP <601> as previously described in Example 1. Results are reported below in Table 17.

**Table 17: Results for a Co-suspension of Salmeterol Xinafoate (SX) Active Agent Particles with Fluticasone Propionate Containing Suspending Particles.**

| FP-Suspending conc. | Target Delivered Dose FP | Target Delivered Dose SX | FP DDU | SX DDU | FP FPF | SX FPF | FP MMAD | SX MMAD |
|---|---|---|---|---|---|---|---|---|
| 4.2 mg/mL | 60 µg | 13 µg | 9.0% RSD* | 13% RSD* | 55% | 51% | 2.8 µm | 3.0 µm |

| | | | | | | | | |
|---|---|---|---|---|---|---|---|---|
| *with a slight upward trend | | | | | | | | |

The delivered dose uniformity through use was tested and all individual delivered doses were within ±25% of mean, at 9.0% RSD for FP and 13% RSD for SX. Visual observation of the co-suspension was conducted in glass vials and no sedimentation of active agent particles was observed. The vials were left to settle for 24 hours without agitation. The suspension flocculated slowly and formed a homogeneous, single cream layer, showing no indication of separation of SX and suspending particles.

### Example 20 (reference)

Budesonide, 16,17-(butylidenebis(oxy))-11,21-dihydroxy-, (11-β,16-α)-pregna-1,4-diene-3,20-dione, was received micronized by the manufacturer (AARTI, Mumbai, India) and used as active agent particles. The particle size distribution of the budesonide was determined by laser diffraction. 50% by volume of the micronized particles exhibited an optical diameter smaller than 1.9 µm, 90% by volume exhibited an optical diameter smaller than 4.3 µm.

Mometasone furoate, 9α, 21-Dichioro-11β, 17-dihydroxy-16α-methyipregna-1,4-dien e-3,20-dione 17-(2-furoate), was received micronized by the manufacturer (AARTI, Mumbai, India) and used as active agent particles. The particle size distribution of the budesonide was determined by laser diffraction. 50% by volume of the micronized particles exhibited an optical diameter smaller than 1.6 µm, 90% by volume exhibited an optical diameter smaller than 3.5 µm.

Suspending particles were manufactured as described in Example 1. The emulsion was spray dried in nitrogen using the following spray drying conditions: Inlet temperature 95 °C, outlet temperature 72 °C, emulsion feed rate 2.4 mL/min, total gas flow 498 L/min.

Metered dose inhalers were prepared by weighing the target masses of micronized active and suspending particles into coated glass vials with 15 mL volume. The canisters were crimp sealed with 63 µl valves (Valois, Les Vaudreuil, France) and filled with 9.2 g of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK). Aerosol performance was assessed shortly after manufacturing in accordance with USP <601> as previously described in Example 1. The suspension concentrations were 0.8 mg/mL for budesonide active agent particles, 1.1 mg/mL for mometasone furoate active agent particles, and 6 mg/mL for the suspending particles. The suspending particle to active agent particle ratio was 7.5 for budesonide and 5.5 for mometasone furoate. Target ex actuator doses were 40 µg for budesonide and 55 µg for mometasone furoate.

Visual observation of the co-suspended configurations showed no sedimentation of active agent particles. The suspensions flocculated and formed a cream layer. The vials were left to settle for 16 h without agitation. No active agent particles were visible at the bottom of the co-suspension vials. The association between active agent particles and suspending particles was strong enough to overcome buoyancy forces as settling of the active agent particles was successfully inhibited.

### Example 21

An exemplary co-suspension composition as described herein was prepared and evaluated. The composition included a combination of glycopyrrolate (GP) and formoterol fumarate (FF) active agents. GP was present in the propellant as micronized, crystalline active agent particles. It was co-suspended with spray dried suspending particles that included FF disposed within the material forming the suspending particle. To achieve this, FF was dissolved in the feedstock used to manufacture the lipid-based suspending particles.

GP active agent particles were formed by micronizing glycopyrrolate using a jet mill. The particle size distribution of the glycopyrrolate active agent particles was determined by laser diffraction. 50% by volume of the active agent particles exhibited an optical diameter smaller than 1.7 µm, and 90% by volume exhibited an optical diameter smaller than 3.5 µm.

FF-containing suspending particles were manufactured as follows: 654 mL of a fluorocarbon-in-water emulsion of PFOB (perfluorooctyl bromide) stabilized by a phospholipid was prepared; 26.5 g of the phospholipid, DSPC (1,2-disteroyl-sn-glycero-3-phosphocholine), and 2.4 g of calcium chloride were homogenized in 276 mL of hot water (80°C) using a high shear mixer; and 142 mL of PFOB were added slowly during homogenization. The resulting coarse emulsion was then further homogenized using a high pressure homogenizer (Model C3, Avestin, Ottawa, CA) at pressures of up to 170 MPa for 5 passes. 552 mg FF was dissolved in 273 mL of warm water (50°C) and most of the solution was combined with the emulsion using a high shear mixer. The emulsion was spray dried in nitrogen using the following spray drying conditions: inlet temperature 95°C; outlet temperature 68°C; emulsion feed rate 2.4 mL/min; and total gas flow 498 l/min. The final mass fraction of formoterol in the spray dried powder was 2%.

A second lot of FF-containing suspending particles was manufactured in a similar fashion. The mass fraction of FF in the spray dried powder was 1% for this lot. A third lot of suspending particles was manufactured without FF.

The particle size distribution of the suspending particles (VMD) was determined by laser diffraction. For both lots of FF containing suspending particles, 50% by volume were smaller than 3.5 µm and the Geometric Standard Deviation of the distribution was 1.7. For the suspending particles without FF, 50% by volume were smaller than 3.2 µm and the Geometric Standard Deviation of the distribution was 1.8.

MDIs were prepared by weighing the target masses of active agent particles and suspending particles into fluorinated ethylene polymer (FEP) coated aluminum canisters (Presspart, Blackburn, UK) with a 19 mL volume. The canisters were crimp sealed with 63 µl valves (# BK 357, Bespak, King's Lynn, UK) and filled with 12.4 g of HFA 134a (1,1,1,2-tetrafluoroethane) (Ineos Fluor, Lyndhurst, UK) by overpressure through the valve stem. The resulting suspension concentrations and the target delivered dose assuming 20% actuator deposition are given in Table 18a for three different configurations (configurations 1A through 1C). After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK).

**Table 18a: Configurations of the glycopyrrolate - formoterol fumarate combination co-suspensions of Example 21**

| | GP | Suspending Particle 1 | | Suspending Particle 2 | Suspending Particle to Active Particle Ratio | Ex actuator dose | |
|---|---|---|---|---|---|---|---|
| # | C_{S} [mg/mL] | FF content | C_{S} [mg/mL] | C_{S} [mg/mL] | | GP [µg] | FF [µg] |
| 1A | 0.48 | 1.9 % | 3.2 | - | 6.7 | 24.0 | 3.2 |
| 1B | | 1.0 % | 6.4 | - | 13.3 | | |
| 1C | | 1.9 % | 3.2 | 3.2 | 13.3 | | |

The filled MDIs were stored valve down at two different conditions: refrigerated at 5°C without overwrap and controlled room temperature at 25°C/60% RH with a foil overwrap. Aerosol performance and delivered dose uniformity tests were carried out at different time points. Aerosol performance was assessed shortly after manufacturing in accordance with USP <601>. A Next Generation Impactor (NGI) operated at a flow rate of 30 L/min was used for determination of particle size distribution. Sample canisters were seated into an actuator with two waste actuations and two additional waste priming actuations. Five actuations were collected in the NGI with a USP throat attached. The valve, actuator, throat, NGI cups, stages, and filter were rinsed with volumetrically dispensed solvent. The sample solutions were assayed using a drug-specific chromatographic method. The fine particle fraction was defined using the sum of stages 3 through filter. Delivered dose uniformity through use testing was performed using a Dose Uniformity Sampling Apparatus as described by USP <601>. Inhalers were seated and primed as described before. Two actuations were collected and assayed at beginning, middle and end of use.

No trends in aerosol performance or delivered dose uniformity were observed for the duration of the study (3 months) or as a function of storage temperature. Hence, all aerosol performance test results were pooled. Table 18b lists the average performance of the different configuration. The fine particle dose is the sum of collected mass on stages 3 to filter of the impactor, normalized by the metered dose. The average aerosol performance for all three configurations was equivalent.

**Table 18b: Average aerosol performance for combination co-suspensions in Example 21**

| # | MMAD in µm | | FPD in % | |
|---|---|---|---|---|
| | FF | GP | FF | GP |
| 1A | 2.8 | 3.4 | 52 | 44 |
| 1B | 2.9 | 3.6 | 51 | 45 |
| 1C | 2.9 | 3.6 | 51 | 45 |

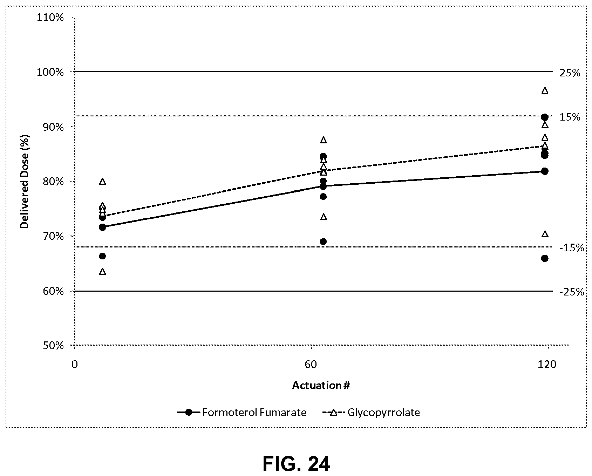

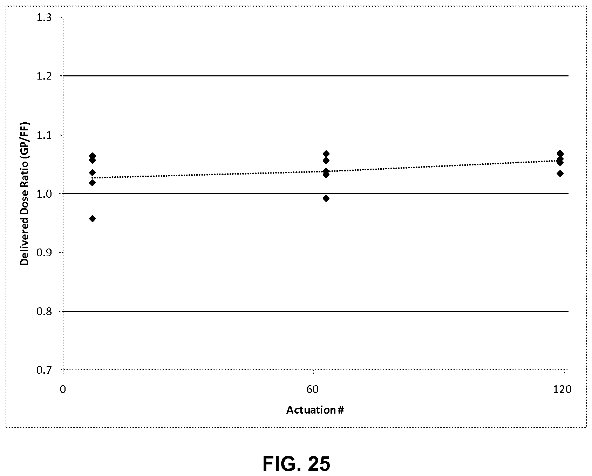

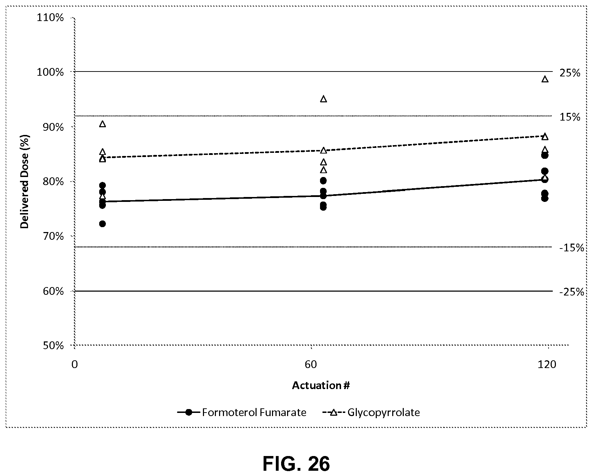

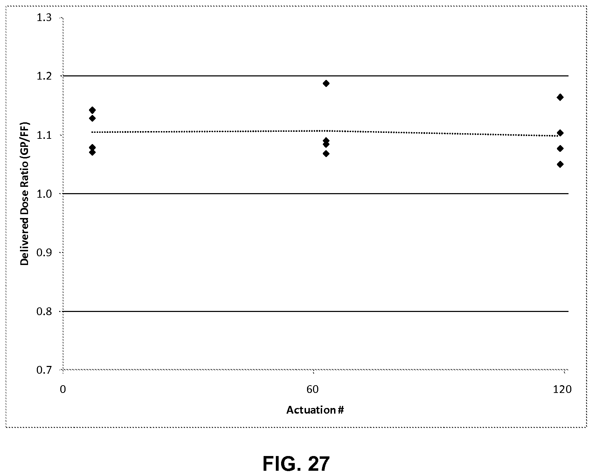

Dose content uniformity was tested through canister life for both actives of the combination product. Figures 24 and 26 show the ex-actuator dose for configuration 1A and 1B, respectively, normalized by the actual metered doses of the canister. Assuming an actuator deposition of 20% the target ex-actuator doses for both actives were 80%. The individual FF and GP doses are represented by dots and triangles, respectively. The closed line denotes the mean of the formoterol doses, and the broken line denotes the mean of the glycopyrrolate doses. Figures 25 and 27 show the ratio of the normalized ex actuator doses for configuration 1A and 1B, respectively. The result indicates that the dose ratio remained constant through canister life. Furthermore the variability of the dose ratio is much lower than that of the individual doses, indicating that a co-suspension with a consistent carrier to active ratio was formed and maintained through container life.

The results show that, when formulated according to the disclosure provided herein, combination product co-suspensions are formed with suspending particles containing one of the active pharmaceutical ingredients, in this case FF. Suspending particle to active agent particle ratios can be adjusted to achieve targeted dose content uniformity while maintaining similar aerosol performance.

### Example 22

MDIs containing FF and GP were prepared to provided target delivered doses of 2.4 and 18 µg per actuation FF and GP, respectively. GP active agent was micronized and had a d₁₀, d₅₀, d₉₀ and span of 0.6, 1.7, 3.6 and 1.9 µm respectively as measured by laser diffraction as described Example 21. FF was incorporated into spray dried suspending particles and prepared as described in Example 21, with a composition of 2% FF, 91.5% DSPC and 6.5% CaCL₂. The GP, FF and GP + FF MDIs were prepared by weighing the target masses of active agent particles and suspending particles into fluorinated ethylene polymer (FEP) coated aluminum canisters (Presspart, Blackburn, UK) with a 19 mL volume. The canisters were crimp sealed with 50 µl valves (# BK 357, Bespak, King's Lynn, UK) and filled with 10.2 g of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes. The canisters were fitted with polypropylene actuators with a 0.3 mm orifice (# BK 636, Bespak, King's Lynn, UK).

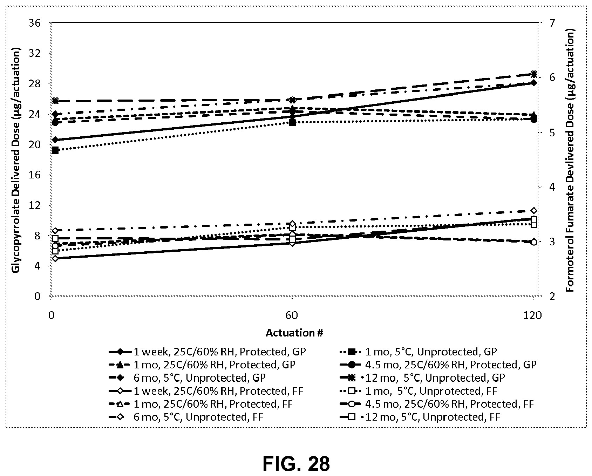

Long term stability and delivery characteristics of these MDI compositions were assessed. In particular the aerosol particle size distribution and delivered dose characteristics of such compositions were evaluated as in accordance with USP <601> as described in Example 21, under various conditions and, in some instances, for periods of time extending up to 12 months. For example, as is shown in Figure 28, the delivered dose uniformity provided by the compositions prepared according to Example 21 was substantially preserved, even after 12 months storage of such compositions at 5°C or after 4.5 months at 25 °C and 60 % relative humidity (RH) for samples stored inside aluminum foil pouches to minimize water ingress into the MDI canister (i.e., "protected storage").

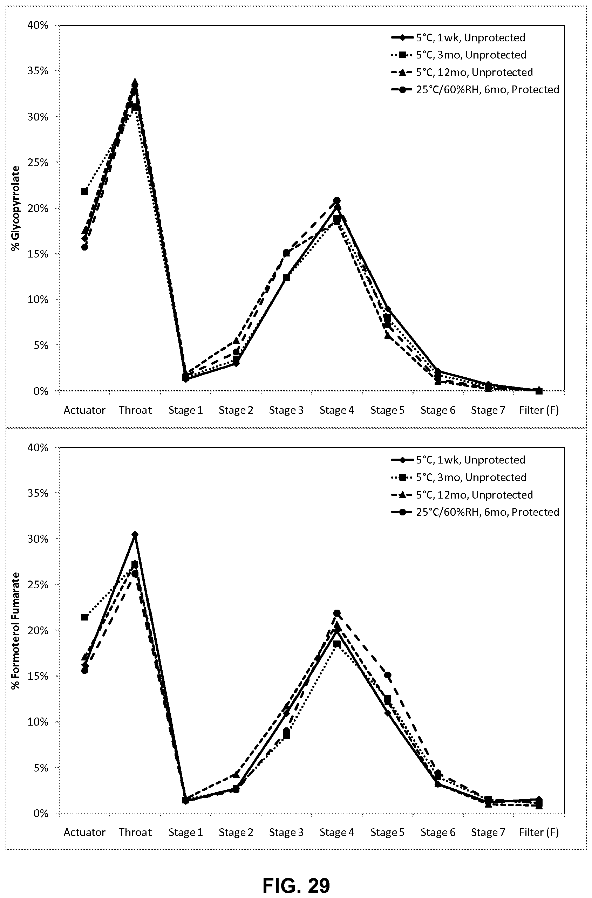

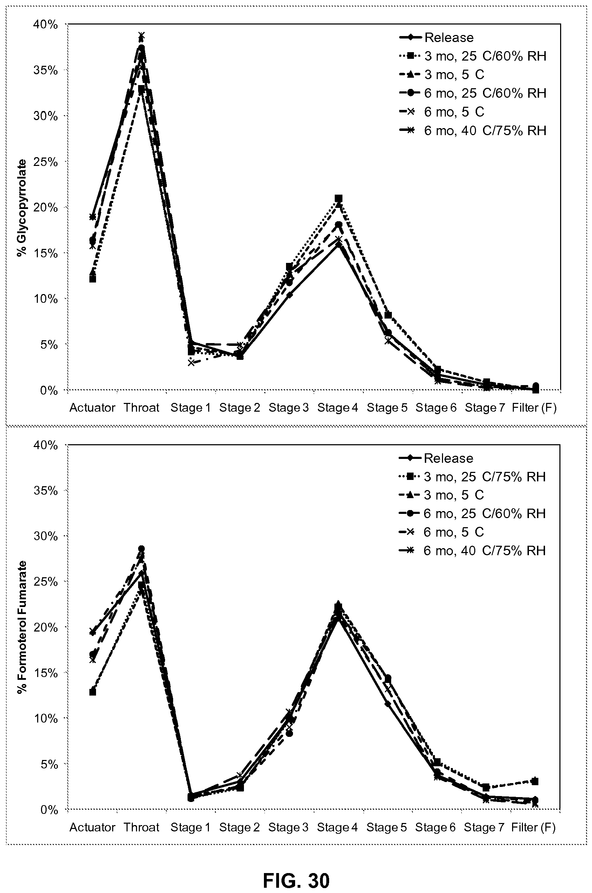

The aerosol performance of such compositions was also evaluated throughout unprotected storage conditions extending up to 12 months and protected storage conditions extending up to 6 months. As is shown in Figure 29, the GP and FF particle size distributions provided by this co-suspension composition were substantially preserved after 12 months of protected storage at 5°C and six months of unprotected storage conditions at 25°C and 60% RH. As is shown in Figure 30, even under stressed conditions (40°C, 75% RH), the compositions showed no noticeable degradation in the particle size distribution of GP and FF delivered from the metered dose inhalers after six months.

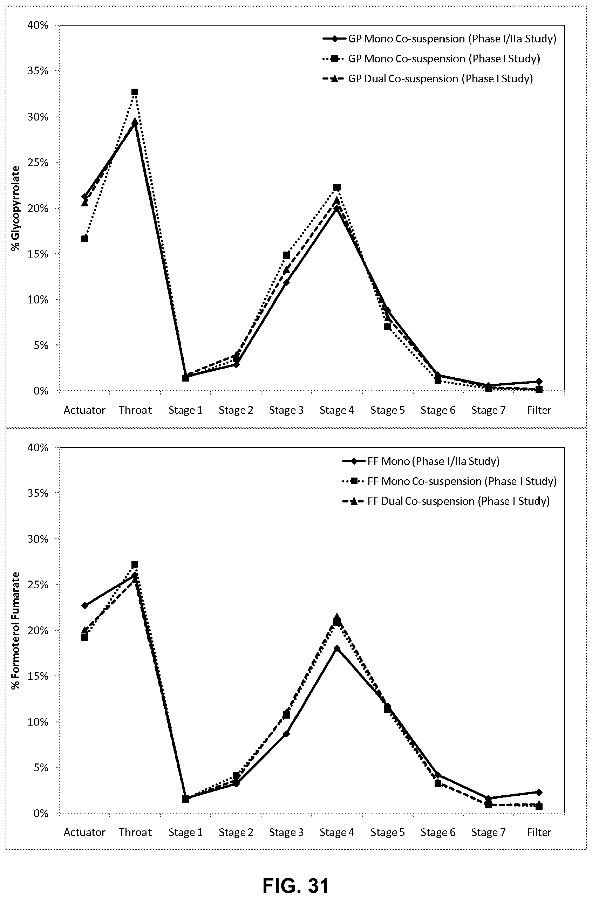

As can be seen in Figure 31 the aerosol performance of the combination co-suspension composition including both GP and FF active agent was no different than the aerosol performance achieved by a suspension composition including FF alone or a co-suspension composition containing GP alone, demonstrating that the aerosol properties of the individual active agents are substantially the same when achieved from the single component or dual combination co-suspensions.

### Example 23

An exemplary dual co-suspension composition according to the present description was produced and metered dose inhalers incorporating the composition were prepared. The composition included a combination of glycopyrrolate (GP) and formoterol fumarate (FF), with each being provided as a micronized, crystalline material. A combination crystalline co-suspension MDI was manufactured by semi-automated suspension filling. The dual co-suspension consisted of a combination of two microcrystalline active pharmaceutical ingredients (also referred to as "APIs" or "API" in the singular), GP and FF, co-suspended with suspending particles in HFA 134a propellant. The dual co-suspension was formulated to provide a delivered dose of 18 µg GP per actuation and 4.8 µg FF per actuation. In preparing the dual co-suspension compositions, in certain compositions, the FF API material used was denoted as "coarse", while in other compositions, the FF API material used was denoted as "fine." Whether the co-suspension compositions incorporated coarse or fine FF, the compositions were formulated to provide a delivered FF dose of 4.8 µg per actuation. The particle size characteristics for the coarse FF, fine FF and GP API materials used in formulation the co-suspension compositions described in this Example are detailed in Table 19. In addition to the dual co-suspension compositions, a monotherapy co-suspension composition incorporating only FF active agent material was formulated. The FF monotherapy co-suspension utilized coarse FF API. A monotherapy MDI was manufactured using such FF monotherapy co-suspension, and the FF monotherapy MDI was formulated and manufactured provide a delivered dose of 4.8 µg FF per actuation.

Suspending particles were manufactured via spray dried emulsion at a feed stock concentration of 80 mg/mL with a composition of 93.44% DSPC (1,2-Distearoyl-sn-Glycero-3-Phosphocholine) and 6.56% anhydrous calcium chloride (equivalent to a 2:1 DSPC:CaCl₂ mole/mole ratio). During the emulsion prep, DSPC and CaCl₂ was dispersed with a high shear mixer at 8000-10000 rpm in a vessel containing heated water (80 ± 3 °C) with PFOB slowly added during the process. The emulsion was then processed with 6 passes in a high pressure homogenizer (10000-25000 psi). The emulsion was then spray dried via a spray dryer fitted with a 0.42" atomizer nozzle with a set atomizer gas flow of 18 SCFM. The drying gas flow rate was set to 72 SCFM with an inlet temperature of 135 °C, outlet temperature 70 °C, and an emulsion flow rate of 58 mL/min.

For the MDI manufacturing, a drug addition vessel (DAV) was prepared for suspension filling in the following manner: first adding half of suspending particle quantity, next filling microcrystalline materials, and lastly adding the remaining half of suspending particles to the top. Materials were added to the vessel in a humidity controlled environment of <10% RH. The DAV was then connected to a 4 L suspension vessel and flushed with HFA 134a propellant and then mixed with gently to form a slurry. The slurry is then transferred back to the suspension mixing vessel and diluted with additional HFA-134a to form the final suspension at target concentration stirring gently with an impeller. The temperature inside the vessel was maintained at 21-23 °C throughout the entire batch production. After recirculation for 30 min the suspension was filled into 14 mL fluorinated ethylene polymer (FEP) coated aluminum canisters (Presspart, Blackburn, UK) through 50 µl valves (Bespak, King's Lynn, UK). Sample canisters were the selected at random for total canister analysis to ensure correct formulation quantities. The optical diameter and particle size distribution of two lots of micronized formoterol particles was determined by laser diffraction as described in Example 1. Table 19 lists the d₁₀, d₅₀ and d₉₀ values for the different lots of micronized material used. d₁₀, d₅₀ and d₉₀ denote the particle size at which the cumulative volume distribution reported by the particle sizing instrument reaches 10%, 50% and 90%, respectively.

The particle size distributions provided by both dual co-suspension formulations prepared in accordance with this Example were compared to the particle size distribution provided by co-suspension compositions prepared according to Example 21. The results of this comparison are provided in Table 20, where "%FPF FF" and "%FPF GP" represent the fine particle mass of the specified active agent on Stages 3 through filter of an NGI, divided by actuator mass, and multiplied by 100.

**Table 19: Particle Size Distributions for micronized Formoterol Fumarate and Glycopyrrolate used to prepare Dual Co-Suspensions**

| **Designation** | **d₁₀ (µm)** | **d₅₀ (µm)** | **d₉₀ (µm)** | **Span** |
|---|---|---|---|---|
| Coarse FF API | 0.6 | 1.9 | 4.4 | 2.0 |
| Fine FF API | 0.5 | 1.3 | 2.3 | 1.5 |
| GP API | 0.5 | 1.3 | 3.0 | 1.9 |

**Table 20: Particle Size Distributions for Different, Exemplary GP/FF Co-suspensions**

| | MMAD FF | %FPF FF | MMAD GP | %FPF GP | MMAD DSPC | %FPF DSPC |
|---|---|---|---|---|---|---|
| Dual Co-Suspension 1 (FF coarse) | 3.4 | 59% | 2.9 | 65% | 2.9 | 64% |
| Dual Co-Suspension 2 (FF fine) | 2.7 | 62% | 3.0 | 62% | 3.1 | 62% |
| Spray-dried FF | 2.7 | 66% | 2.9 | 65% | not tested | not tested |

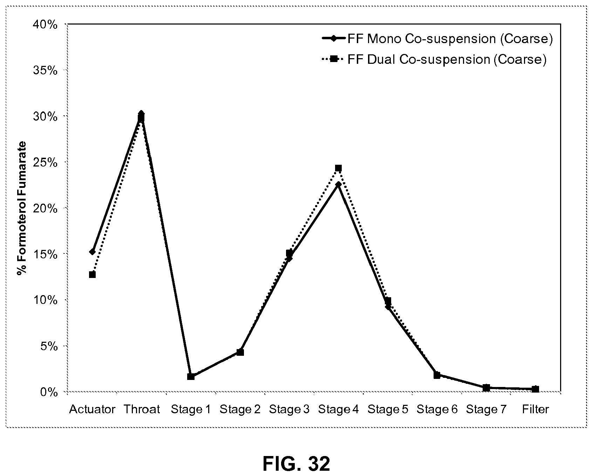

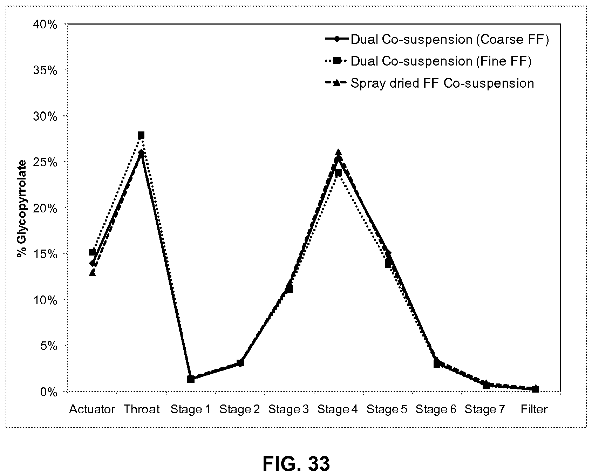

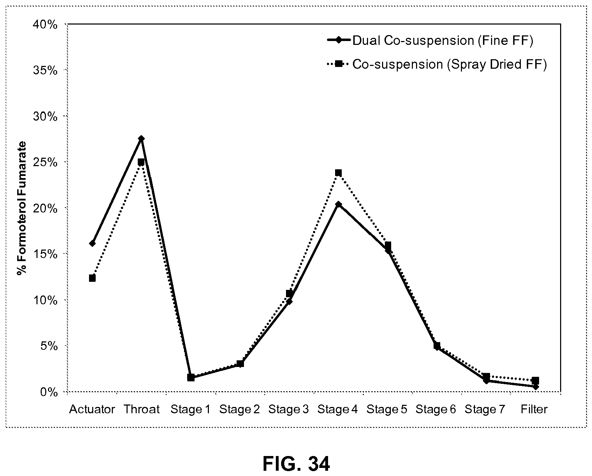

The aerosol performance of the dual co-suspension compositions prepared according to this Example was evaluated and compared to the co-suspension composition prepared according to Example 21, with aerosol performance being determined in accordance with USP <601> as described Example 1. The results of such comparisons are provided in Figure 32 through Figure 34. As is easily appreciated by reference to these figures, regardless of whether the crystalline formoterol material used in providing the dual co-suspension was fine or coarse, the FF and GP particle size distributions for the dual co-suspension compositions were substantially the same as those achieved by the co-suspension composition prepared according to Example 21 where FF is incorporated into the suspending particles via spray drying.

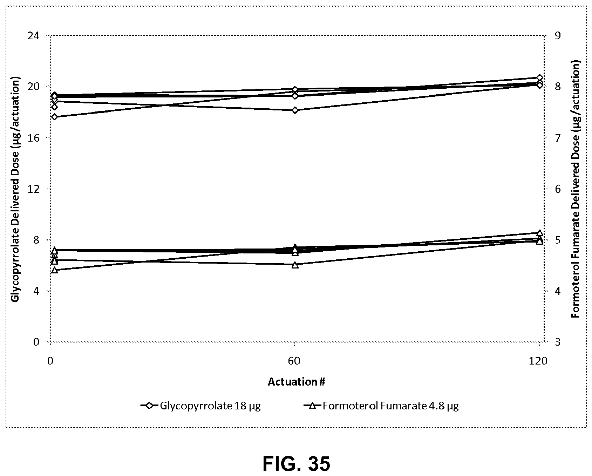

In addition, the delivered dose uniformity for GP and FF provided by the dual co-suspension compositions as described in this Example was assessed determined in accordance with USP <601> as described Example 1. The results of this assessment are illustrated in Figure 35. The dual co-suspension formulations provided desirable DDU characteristics for both GP and FF as all actuations delivered the expected dose within ± 25% of the mean.

### Example 24

Dual co-suspension compositions were prepared with suspending particles including either mometasone furoate (MF) or budesonide (BD), and MDIs incorporating the composition were prepared. The triple co-suspension composition included a combination of crystalline glycopyrrolate (GP) and formoterol fumarate (FF) active agent particles co-suspended with suspending particles including either MF or BD. Each of the APIs were provided as a micronized, crystalline material.

Suspending particles containing 50% (w/w) of either BD or MF were manufactured as follows: high shear homogenization of a dispersion containing 2.8 g of DSPC (1,2-Distearoyl-sn-Glycero-3-Phosphocholine), and 0.26 g of calcium chloride in 400 mL of hot water (75 °C) using a high shear mixer was performed while 56.6 g of PFOB were added slowly. Micronized MF or BD (in 1:1 weight proportion to DSPC) was added to the resulting coarse emulsion, which was further homogenized using a high pressure homogenizer (Model C3, Avestin, Ottawa, CA) at pressures of up to 170 MPa for 3 to 5 passes. The emulsion was spray dried using the following spray drying conditions: inlet temperature 90-95 °C; outlet temperature 95-72 °C; emulsion feed rate 2-8 mL/min; total dry nitrogen flow 525-850 L/min. The particle size distribution of the resulting powders was determined by laser diffraction, 50% by volume of the suspending particles were smaller than 1.8 µm, the span of the distribution was 1.6 µm.
MDI canisters containing either 50% (w/w) MF or BD containing suspending particles were prepared, targeting a 50 or 100 µgper actuation of MF or BD, respectively. Metered dose inhalers were prepared by weighing the target masses of active agent containing suspending particles and in some cases additional suspending particles, to reach a total suspension concentration of 5.5 mg/mL, into fluorinated ethylene polymer (FEP) coated aluminum canisters (Presspart, Blackburn, UK) with 14 mL volume. The canisters were crimp sealed with 50 µl valves (Bespak, King's Lynn, UK) and filled with 10 mL of HFA 134a (1,1,1,2-tetrafluoroethane) (Ineos Fluor, Lyndhurst, UK) by overpressure through the valve stem. After injecting the propellant, the canisters were sonicated for 15 seconds and agitated on a wrist action shaker for 30 minutes. The canisters were fitted with polypropylene actuators with a 0.3 mm orifice (Bespak, King's Lynn, UK).

The aerosol particle size distributions of the above MDIs were determined in accordance with USP <601> as described in Example 1, and results are shown in Table 21. A comparable series of canisters containing MF or BD containing suspending particles in combination with GP and FF active agent particles were produced. Sufficient micronized GP and FF API material was added to such canisters in amounts sufficient to provide targeted delivered doses of 36 µg per actuation and 6 µg per actuation for GP and FF, respectively. In some cases, additional suspending particles prepared as described in Example 1 were added to reach a total suspension concentration of 5.5 mg/mL.

The aerosol particle size distributions of the above triple co-suspension MDIs were determined in accordance with USP <601> as described in Example 1, with the results are shown in Table 22. Comparison of results in Table 21 and Table 22 demonstrate that the mass mean aerodynamic diameter of the corticosteroid in the single component suspensions is equivalent to the one obtained in the corresponding triple combination compositions. As was true of the co-suspension compositions containing a combination of two different active agents, the triple co-suspension compositions prepared according to the present description avoided a combination effect. In addition the fine particle fractions of the microcrystalline active agents are mostly independent of the type of the corticosteroid in monotherapy or triple combination compositions, even though the doses per actuation of MF or BD are substantially different.

**Table 21: Suspension MDIs in HFA 134a propellant containing corticosteroid suspending particles. Aerosol properties, mass aerodynamic diameter and fine particle fraction determined by drug specific cascade impaction.**

| | **Suspension. Concentration (mg/mL)** | **MMAD** | **FPF** |
|---|---|---|---|
| | | **(µm)** | **(%)** |
| **Mometasone Furoate** | **5.5** | **2.88** | **61.0** |
| **Budesonide** | **5.6** | **3.20** | **61.7** |

**Table 22: Triple combination suspension MDIs in HFA 134a propellant including corticosteroid containing suspending particles (Mometasone Furoate or Budesonide), a LAMA (Glycopyrrolate) and a LABA (Formoterol Fumarate). Mass mean aerodynamic diameter and fine particle fraction determined by drug specific cascade impaction.**

| | **Suspension Concentration (mg/mL)** | **Drug** | **MMAD (µm)** | **FPF (%)** |
|---|---|---|---|---|
| Triple A | 2.3 | Formoterol | 3.96 | 44.4 |
| | | Glycopyrrolate | 3.71 | 49.0 |
| | | Mometasone | 2.90 | 61.6 |
| Triple B* (*with added suspending suspending particles) | 5.6 | Formoterol | 3.52 | 44.4 |
| | | Glycopyrrolate | 3.34 | 49.0 |
| | | Mometasone | 2.54 | 61.6 |
| Triple C | 5.5 | Formoterol | 3.89 | 47.1 |
| | | Glycopyrrolate | 3.74 | 50.0 |
| | | Budesonide | 3.12 | 63.1 |

### Example 25

Metered dose inhalers containing a triple co-suspension composition were prepared according to the present description. The composition included a combination of glycopyrrolate (GP), formoterol fumarate (FF), and mometasone furoate (MF) active agent particles, with each being provided as a micronized, crystalline API material.

A triple co-suspension MDI was manufactured by semi-automated suspension filling. The triple co-suspension consisted of a combination of three microcrystalline active pharmaceutical ingredients forming three different species of active agent particles: MF (corticosteroid); GP (LAMA); and FF (LABA). These three different species of active agent particles were co-suspended with suspending particles in HFA 134a propellant. The triple co-suspension was formulated to the following delivered dose targets: 50 µg per actuation MF; 36 µg per actuation GP; and 4.8 µg per actuation FF. In addition to the triple co-suspension, a monotherapy co-suspension including only MF was produced. The monotherapy MF co-suspension included MF active agent particles co-suspended in the propellant with suspending particles as described in this Example, and was formulated to provide a target delivered dose of 50 µg per actuation MF.

Suspending particles were manufactured via spray dried emulsion at a feed stock concentration of 80 mg/mL with a composition of 93.44% DSPC (1,2-Distearoyl-sn-Glycero-3-Phosphocholine) and 6.56% anhydrous calcium chloride (equivalent to a 2:1 DSPC:CaCl₂ mole/mole ratio). During the emulsion prep, DSPC and CaCl₂ were dispersed with a high shear mixer at 8000-10000 rpm in a vessel containing heated water (80 ± 3 °C) with PFOB slowly added during the process. The emulsion was then processed with 5 passes in a high pressure homogenizer (10000-25000 psi). The emulsion was then spray dried via a spray dryer fitted with a 0.42" atomizer nozzle with a set atomizer gas flow of 18 SCFM. The drying gas flow rate was set to 72 SCFM with an inlet temperature of 135 °C, outlet temperature 70 °C, and an emulsion flow rate of 58 mL/min.

For MDI manufacturing, a drug addition vessel (DAV) was used for suspension filling in the following manner: first adding half of suspending particle quantity, next filling microcrystalline materials, and lastly adding the remaining half of suspending particles to the top. Materials were added to the vessel in a humidity controlled environment of <10% RH. The DAV was then connected to a 4 L suspension vessel and flushed with HFA 134a propellant and then mixed with a magnetic stir bar. The temperature inside the vessel was maintained at 21-23 °C throughout the entire batch production. After recirculation of the batch for 30 min canisters were filled with the suspension mixture through 50 µL EPDM valves. Sample canisters were the selected at random for Total Canister Analysis to ensure correct formulation quantities. The freshly manufactured triple co-suspension MDI batch was then placed on one week quarantine before initial product performance analysis. The mometasone furoate only MDI was manufactured by suspension filling in the same manner. Fluorinated ethylene polymer (FEP) coated aluminum canisters (Presspart, Blackburn, UK) with 14 mL volume. The canisters were crimp sealed with 50 µl valves (Bespak, King's Lynn, UK) and filled with 10 mL of HFA 134a (1,1,1,2-tetrafluoroethane) (Ineos Fluor, Lyndhurst, UK) by overpressure through the valve stem. The canisters were fitted with polypropylene actuators with a 0.3 mm orifice (Bespak, King's Lynn, UK).

The primary particle size distribution of all microcrystalline APls was determined by laser diffraction as described in Example 1, results are shown in Table 23. Aerodynamic particle size distribution and mass mean aerodynamic diameter of all components upon actuation of the suspension MDIs was determined by drug specific cascade impaction in accordance with USP <601> as described in Example 1 and are shown in Table 24.

**Table 23: Triple microcrystalline Co-Suspension in HFA 134a propellant MDI. Primary particle size distribution determined by laser diffraction (Sympatec).**

| **Materials** | **x10 (µm)** | **x50 (µm)** | **x90 (µm)** | **Span** |
|---|---|---|---|---|
| **Micronized Mometasone Furoate (MF)** | 0.4 | 1.1 | 2.8 | 2.2 |
| **Micron ized Glycopyrrolate (GP)** | 0.5 | 1.3 | 3.0 | 1.8 |
| **Micronized Formoterol Fumarate Dihydrate (FF)** | 0.6 | 1.9 | 4.1 | 1.8 |

**Table 24: Triple co-suspension MDIs in HFA 134a propellant containing microcrystalline Corticosteroid (Mometasone Furoate), LABA (Formoterol Fumarate) and a LAMA (Glycopyrrolate). Aerosol properties, mass mean aerodynamic diameter and fine particle fraction were determined by drug specific cascade impaction (NGI).**

| | **Suspension Concentration (mq/mL)** | **Drug** | **MMAD (µm)** | **FPF (%)** |
|---|---|---|---|---|
| **Triple (Corticosteroid, LABA, LAMA)** | **6** | **Mometasone** | **3.18** | **62.6** |
| | | **Formoterol** | **3.50** | **59.5** |
| | | **Glycopyrrolate** | **2.97** | **64.1** |
| **Mono (Corticosteroid)** | **6** | **Mometasone** | **3.36** | **58.9** |

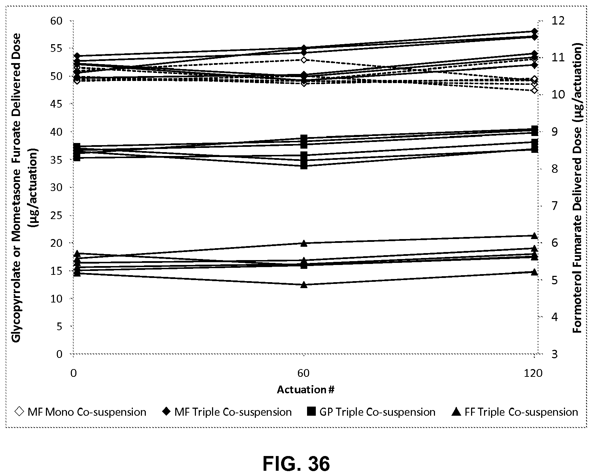

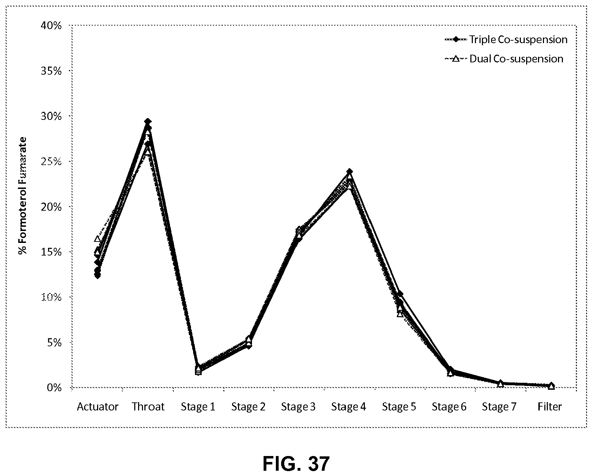

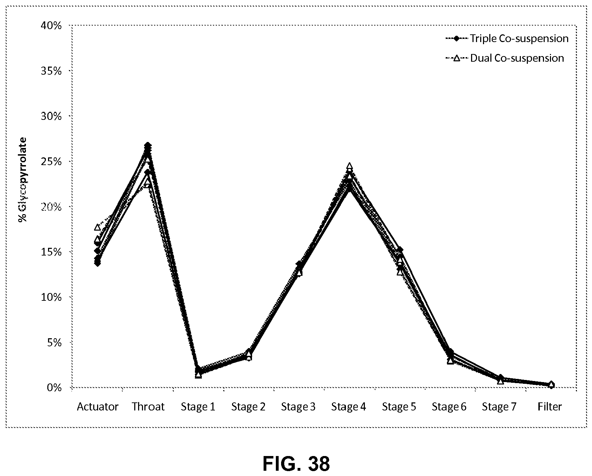

Delivered dose uniformity and the aerosol performance achieved by the triple co-suspensions prepared according to this Example was evaluated in accordance with USP <601> as described in Example 1. Figure 36 illustrates the DDU of GP, FF and MF achieved from two canisters containing MF only and two canisters containing MF, GP and FF prepared according to this Example. The DDU of MF delivered from the MF monotherapy configuration is equivalent to the one achieved with the triple co-suspension composition. The aerosol particle size distributions achieved for FF and GP from the triple co-suspension composition of this Example was compared to the one achieved from a co-suspension containing two active agents, FF and GP prepared according Example 15. The aerodynamic particle size distribution of FF and GP are equivalent whether delivered from the compositions containing two active agents or three active agents as shown in Figures 37 and 38, respectively, thus the triple co-suspension compositions prepared according to the present description avoided a combination effect.

### Example 26

Exemplary triple co-suspension compositions according to the present description were produced and metered dose inhalers incorporated in the composition were prepared. The triple co-suspensions included glycopyrrolate (GP) or tiotropium bromide (TB) in combination with formoterol fumarate (FF), and mometasone furoate (MF) active agents, with each API being used as micronized, crystalline material.

Two separate suspension MDI batches containing three active pharmaceutical ingredients (APls), a corticosteroid, a LAMA and a LABA were prepared. The APls were provided as microcrystalline materials that served as the active agent particles co-suspended with suspending particles prepared as described herein. The triple co-suspension compositions prepared as described in this Example were prepared by adding the active agent particles and suspending particles to an HFA 134a propellant.

The triple co-suspension containing glycopyrrolate (Triple GFM) was formulated to deliver 40 µg per actuation MF; 13 µg per actuation GP; and 4.8 µg per actuation FF. The active agent particles were co-suspended with suspending particles manufactured using an emulsion composed of 93.46% DSPC (1,2-Distearoyl-sn-Glycero-3-Phosphocholine) and 6.54% anhydrous calcium chloride spray dried with an 80 mg/mL feed concentration. The DSPC:CaCl₂ molar ratio of the suspending particles was 2:1. The suspending particles were combined with the active agent particles in propellant for a formulation target suspending particle concentration of 6 mg/mL. The primary particle sizes of the microcrystalline active agent particles, determined by Sympatec laser diffraction measurements as described in Example 1, are displayed below in Table 25.

The triple co-suspension containing tiotropium bromide (Triple TFM) was prepared using anhydrous tiotropium bromide (TB). The TFM triple co-suspension was formulated to deliver 50 µg per actuation MF; 9 µg per actuation TB; and 4.8 µg per actuation FF. The suspending particles were prepared as described in relation to the Triple GFM co-suspension, and the active agent particles were co-suspended with the suspending particles at a targeted suspending particle concentration of 6 mg/mL. The primary particle sizes of the microcrystalline active agent particles, determined by Sympatec laser diffraction measurements as described in Example 1, are displayed below in Table 26.

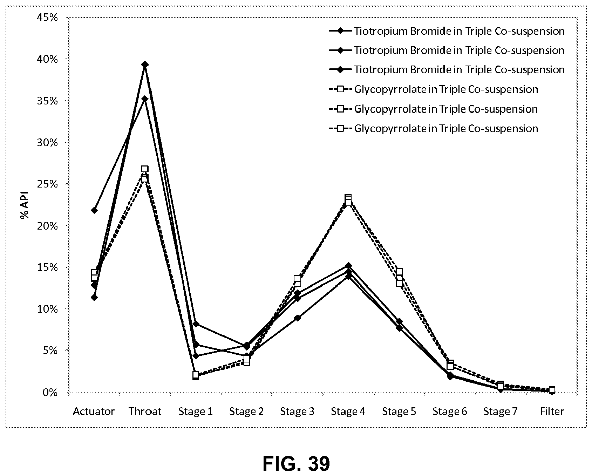

The aerosol particle size distribution, fine particle fraction, and mass median aerodynamic diameter were determined for the triple co-suspension compositions described in this Example in accordance with USP <601> as described in Example 1. Table 27 sets out the MMAD and FPF performance for Triple GFM and Triple TFM, while the desirable aerosol properties achieved by the Triple GFM and Triple TFM co-suspensions are shown in Figure 39 (showing the aerodynamic particle size distribution of GP and TB obtained from Triple GFM and Triple TFM, respectively). The fine particle fractions of the individual microcrystalline active agents, achieved in the triple formulations are remarkably similar in spite of differences in the size of the active agent particles, demonstrating the benefits of the compositions described in the present invention.

**Table 25: Triple GFM primary particle size distribution of micronized crystalline drugs determined by laser diffraction (Sympatec).**

| **Materials** | **d₁₀ (µm)** | **d₅₀ (µm)** | **d₉₀ (µm)** | **Span** |
|---|---|---|---|---|
| **Micronized Mometasone Furoate** | 0.4 | 1.0 | 2.3 | 1.9 |
| **Micronized Glycopyrrolate** | 0.5 | 1.4 | 3.4 | 2.1 |
| **Fumarate Dihydrate** | 0.5 | 1.4 | 2.7 | |

**Table 26: Triple TFM primary particle size distribution determined by laser diffraction (Sympatec).**

| **Materials** | **d₁₀ (µm)** | **d₅₀ (µm)** | **d₉₀ (µm)** | **Span** |
|---|---|---|---|---|
| **Micronized Mometasone Furoate** | 0.4 | 1.1 | 2.8 | 2.2 |
| **Micronized Tiotropium Bromide Anhydrous** | 0.5 | 1.3 | 3.9 | 2.7 |
| **Micronized Formoterol Fumarate Dihydrate** | 0.6 | 1.9 | 4.1 | 1.9 |

**Table 27: Triple GFM and Triple TFM aerosol properties, mass mean aerodynamic diameter and fine particle fraction determined by drug specific cascade impaction**

| | **Suspension Concentration (mg/mL)** | **Drug** | **MMAD (µm)** | **FFP (%)** |
|---|---|---|---|---|
| Triple GFM | 6 | Formoterol | 2.80 | 65.3 |
| | | Glycopyrrolate | 2.90 | 49.5 |
| | | Mometasone | 3.10 | 49.2 |
| Triple TFM | 6 | Formoterol | 3.82 | 42.4 |
| | | Tiotropium | 3.79 | 42.0 |
| | | Mometasone | 4.00 | 43.6 |

Also disclosed are:
Statement 1. A co-suspension deliverable from a metered dose inhaler, the stable co-suspension comprising: a suspension medium comprising a pharmaceutically acceptable propellant; a plurality of active agent particles; and a plurality of respirable suspending particles, wherein the plurality of active agent particles associate with the plurality of suspending particles despite buoyancy differences between the active agent particles and the suspending particles within the suspension medium.
Statement 2. A co-suspension according to statement 1, wherein at least 90% of the active agent particles by volume exhibits an optical diameter of 7 µm or less.
Statement 3. A co-suspension according to statement 1, wherein at least 50% of the active agent particles by volume exhibits an optical diameter of 5 µm or less
Statement 4. A co-suspension according to any preceding statement, wherein the active agent particles comprise particles of active agent in crystalline form.
Statement 5. A co-suspension according to any of statements 1 through 3, wherein the active agent particles comprise particles of active agent in amorphous form.
Statement 6. A co-suspension according to any preceding statement, wherein the active agent exhibits measurable solubility that results in dissolution selected from as much as 1 % of the total active agent mass in the suspension medium, as much as 0.5% of the total active agent mass in the suspension medium, as much as 0.05% of the total active agent mass in the suspension medium, and as much as 0.025% of the total active agent mass in the suspension medium.
Statement 7. A co-suspension according to any of statements 1 through 5, wherein the total mass of the active agent that dissolves in the suspension medium is less than 5% of the total active agent mass in the suspension medium.
Statement 8. A co-suspension according to any preceding statement, wherein the plurality of active agent particles includes two or more different active agents.
Statement 9. A co-suspension according to any preceding statement, wherein the active agent particles comprise an active agent selected from short-acting beta agonists, such as bitolterol, carbuterol, fenoterol, hexoprenaline, isoprenaline (isoproterenol), levosalbutamol, orciprenaline (metaproterenol), pirbuterol, procaterol, rimiterol, salbutamol (albuterol), terbutaline, tulobuterol, reproterol and epinephrine, long-acting beta agonists, such as bambuterol, clenbuterol, formoterol, and salmeterol, ultra long-acting beta agonists, such as carmoterol, milveterol, indacaterol, and saligenin- or indole- containing and adamantyl-derived β₂ agonists, corticosteroids, such as beclomethasone, budesonide, ciclesonide, flunisolide, fluticasone, methyl-prednisolone, mometasone, prednisone and trimacinolone, antiinflammatories, such as fluticasone propionate, beclomethasone dipropionate, flunisolide, budesonide, tripedane, cortisone, prednisone, prednisilone, dexamethasone, betamethasone, or triamcinolone acetonide, antitussives, such as noscapine, bronchodilators, such as ephedrine, adrenaline, fenoterol, formoterol, isoprenaline, metaproterenol, salbutamol, albuterol, salmeterol, terbutaline, anticholinergics, such as glycopyrrolate, dexipirronium, scopolamine, tropicamide, pirenzepine, dimenhydhnate, tiotropium, darotropium, aclidinium, trospium, ipatropium, atropine, benzatropin, or oxitropium, including any pharmaceutically acceptable salts, esters, isomers or solvates thereof.
Statement 10. A co-suspension according to any preceding statement, wherein two or more active agents are included in the active agent particles and the two or more active agents are selected from a combination of formoterol and budesonide, a combination of glycopyrrolate and formoterol, a combination of ciclesonide and formoterol, a combination of salmeterol and fluticasone, a combination of glycopyrrolate, formoterol, and budesonide, and a combination of glycopyrrolate, formoterol, and mometasone, including any pharmaceutically acceptable salts, esters, isomers or solvates thereof.
Statement 11. A co-suspension according to any preceding statement, wherein the active agent is selected from potent and highly potent active agents.
Statement 12. A co-suspension according to any preceding statement, wherein the target delivered dose of active agent is selected from between about 100 µg and about 100 mg per dose, between about 100 µg and about 10 mg per dose, and between about 100 µg and 1 mg per dose.
Statement 13. A co-suspension according to any of statements 1 through 11, wherein the target delivered dose of active agent is selected from up to about 80 µg per dose, up to about 40 µg per dose, up to about 20 µg per dose, or between about 10 µg and about 100 µg per dose.
Statement 14. A co-suspension according to any of statements 1 through 11, wherein the target delivered dose of active agent is selected from between about 0.1 and about 2 µg per dose, about 0.1 and about 1 µg per dose, and about 0.1 and about 0.5 µg per dose.
Statement 15. A co-suspension according to any preceding statement, wherein the suspending particles exhibit an MMAD selected from between about 10 µm and about 500 nm, between about 5 µm and about 750 nm, and 1 µm and about 3 µm.
Statement 16. A co-suspension according to any preceding statement, wherein the suspending particles exhibit a volume median optical diameter selected from between about 0.2 µm and about 50 µm, between about 0.5 µm and about 15 µm, between about 1.5 µm and about 10 µm, and between about 2 µm and about 5 µm.
Statement 17. A co-suspension according to any preceding statement, wherein the suspending particles are included in the suspension medium at a concentration selected from to about 30 mg/mL and up to about 25 mg/mL.
Statement 18. A co-suspension according to statement 17, wherein the suspending particles are included in the suspension medium at a concentration selected from between about 1 mg/mL to about 15 mg/mL, about 3 mg/mL to about 10 mg/mL, about 1.5 mg/mL to about 10 mg/mL.
Statement 19. A co-suspension according to statement 17, wherein the active agent particles comprise glycopyrrolate, and the suspending particles are included in the suspension medium at a concentration selected from about 6 mg/mL, between about 3 mg/mL and about 10 mg/mL, and between about 1 mg/mL and about 15 mg/mL.
Statement 20. A co-suspension according to statement 17, wherein the active agent particles comprise formoterol, and the suspending particles are included in the suspension medium at a concentration selected from about 3 mg/mL, between about 1.5 mg/mL and about 5 mg/mL, and between about 0.5 mg/mL and about 7.5 mg/mL.
Statement 21. A co-suspension according to statement 17, wherein the active agent particles comprise formoterol, and the suspending particles are included in the suspension medium at a concentration selected from about 6 mg/mL, between about 3 mg/mL and about 10 mg/mL, and between about 1 mg/mL and about 15 mg/mL.
Statement 22. A co-suspension according to statement 17, wherein the active agent particles comprise salmeterol, and the suspending particles are included in the suspension medium at a concentration selected from about 5 mg/mL, between about 3 mg/mL and about 10 mg/mL, and between about 1 mg/mL and about 15 mg/mL.
Statement 23. A co-suspension according to statement 17, wherein the active agent particles comprise budesonide, and the suspending particles are included in the suspension medium at a concentration selected from about 8 mg/mL, between about 5 mg/mL and about 20 mg/mL, and between about 0.5 mg/mL and about 30 mg/mL.
Statement 24. A co-suspension according to statement 17, wherein the active agent particles comprise fluticasone, and the suspending particles are included in the suspension medium at a concentration selected from about 6 mg/mL, between about 3 mg/mL and about 10 mg/mL, and between about 1 mg/mL and about 15 mg/mL.
Statement 25. A co-suspension according to any preceding statement, wherein the total mass of the suspending particles exceeds the total mass of the active agent particles.
Statement 26. A co-suspension according to statement 25, wherein the ratio of the total mass of the suspending particles to the total mass of the active agent particles is selected from above about 1.5, up to about 5, up to about 10, up to about 15, up to about 20, up to about 30, up to about 50, up to about 75, up to about 100, up to about 150, and up to about 200.
Statement 27. A co-suspension according to statement 25, wherein at least one of the active agents included in the active agent particles is a highly potent active agent and the ratio of the total mass of the suspending particles to the total mass of the active agent particles is selected from between about 5 and about 175, between about 10 and about 150, between about 15 and about 125, and between about 25 and about 75.
Statement 28. A co-suspension according to statement 25, wherein the active agent particles include one or more of glycopyrrolate, fluticasone, mometasone, and budesonide, and the ratio of the total mass of the suspending particles to the total mass of the active agent particles is selected from between about 1 and about 20, between about 5 and about 15, and about 10.
Statement 29. A co-suspension according to statement 25, wherein the active agent particles include one or more of fluticasone, mometasone, and budesonide, and the ratio of the total mass of the suspending particles to the total mass of the active agent particles is selected from between about 1 and about 15, between about 1.5 and about 10, and between about 2.5 and about 5.
Statement 30. A co-suspension according to statement 25, wherein the active agent particles include salmeterol, and the ratio of the total mass of the suspending particles to the total mass of the active agent particles is selected from between about 10 and about 30, between about 15 and about 25, and about 20.
Statement 31. A co-suspension according to statement 25, wherein the active agent particles include formoterol, and the ratio of the total mass of the suspending particles to the total mass of the active agent particles is selected from between about 10 and about 200, between about 50 and about 125, and about 75.
Statement 32. A co-suspension according to any preceding statement, wherein the suspending particles remain associated with active agent particles even when subjected to buoyancy forces amplified by centrifugation at an acceleration selected from accelerations of at least 1 g, at least 10 g, at least 50 g, and at least 100 g.
Statement 33. A co-suspension according to any preceding statement, wherein the suspending particles comprise an excipient selected from the group consisting of lipids, phospholipids, nonionic detergents, polymers, such as nonionic block copolymers, surfactants, such as non-ionic surfactants and biocompatible fluorinated surfactants, carbohydrates, amino acids, organic salts, peptides, proteins, alditols, and combinations thereof.
Statement 34. A co-suspension according to any preceding statement, wherein the suspending particles comprise active agent particles comprising an excipient selected from the group consisting of lipids, phospholipids, nonionic detergents, polymers, such as nonionic block copolymers, surfactants, such as non-ionic surfactants and biocompatible fluorinated surfactants, carbohydrates, amino acids, organic salts, peptides, proteins, alditols, and combinations thereof.
Statement 35. A co-suspension according to any preceding statement, wherein one or more of said plurality of suspending particles comprises an active agent.
Statement 36. A co-suspension according to any preceding statement, wherein the suspension medium comprises propellant substantially free of additional constituents.
Statement 37. The co-suspension according to statement 36, wherein the propellant comprises a propellant selected from an HFA propellant, a PFC propellant and combinations thereof.
Statement 38. A co-suspension according to any of statements 1 through 35, wherein the suspension medium comprises propellant in combination with one or more constituents selected from antisolvents, solubilizing agents, cosolvents, adjuvants, PVP and PEG.
Statement 39. A co-suspension according to any preceding statement, wherein the active agent particles are prepared by a micronization process selected from milling, grinding, crystallization, recrystallization, and supercritical or near-supercritical precipitation processes, and the suspending particles are prepared using a spray drying process.
Statement 40. A metered dose inhaler comprising a canister with an outlet valve including an actuator for dispensing a metered volume said canister containing a co-suspension as defined in any of statements 1 through 39, wherein the metered dose inhaler exhibits a delivered dose uniformity ("DDU") for the co-suspension formulation selected from a DDU of ± 30%, or better, a DDU of ± 25%, or better, and a DDU of ± 20%, or better, throughout emptying of the canister.
Statement 41. A metered dose inhaler according to statement 40, wherein the metered dose inhaler dispenses the co-suspension at an initial fine particle fraction and the initial fine particle fraction dispensed from the metered dose inhaler is substantially maintained, such that, throughout emptying of the canister, the fine particle fraction delivered from the metered dose inhaler is maintained within 80% of the initial fine particle fraction.
Statement 42. A metered dose inhaler according to statement 41, wherein the fine particle fraction delivered from the metered dose inhaler is maintained within 90% of the initial fine particle fraction.
Statement 43. A metered dose inhaler according to statement 41, wherein the fine particle fraction delivered from the metered dose inhaler is maintained within 95% of the initial fine particle fraction.
Statement 44. A metered dose inhaler according to any of statements 40 through 43, wherein the co-suspension formulation contained within the canister of the metered dose inhaler is storage stable for at least six months.
Statement 45. A metered dose inhaler according to statement 40, wherein the metered dose inhaler exhibits a delivered dose uniformity ("DDU") for the co-suspension formulation selected from a DDU of ± 30%, or better, a DDU of ± 25%, or better, and a DDU of ± 20%, or better, throughout emptying of the canister, after said canister is subjected to temperatures alternating between -5 °C and 40 °C every 6 hours for a period of six weeks.
Statement 46. A metered dose inhaler according to any of statements 41 through 43, wherein the fine particle fraction is substantially maintained throughout emptying of the canister, after said canister is subjected to temperatures alternating between -5 °C and 40 °C every 6 hours for a period of six weeks.
Statement 47. A method of preparing a metered dose inhaler containing a stable co-suspension formulation, the method comprising: loading a canister with suspending particles and active agent particles containing at least one active agent; attaching an actuator valve to an end of said canister and sealing said canister, said actuator valve adapted for dispensing a metered amount of the co-suspension formulation per actuation; and charging the canister with a pharmaceutically acceptable suspension medium comprising a propellant, wherein the active agent particles, suspending particles and suspension medium are selected such that said loading of the active agent particles and suspending particles and said charging of the canister with a pharmaceutically acceptable suspension medium provides a co-suspension formulation as defined in any of statements 1 through 39.
Statement 48. A method of preparing a metered dose inhaler containing a stable co-suspension formulation, the method comprising: loading a canister with active agent particles containing at least one active agent and suspending particles; attaching an actuator valve to an end of said container and sealing said canister, said actuator valve adapted for dispensing a metered amount of the co-suspension formulation per actuation; and charging the canister with a pharmaceutically acceptable suspension medium comprising a propellant, wherein the active agent particles, suspending particles and suspension medium are selected such that said loading of the active agent particles and suspending particles and said charging of the canister with a pharmaceutically acceptable suspension medium provides a metered dose inhaler as defined in any of statements 40 through 46.
Statement 49. A method of respiratory delivery of an active agent to a patient, the method comprising: providing a metered dose inhaler comprising a canister containing a co-suspension as defined in any of statements 1 through 39; and delivering the co-suspension to the patient using the metered dose inhaler.
Statement 50. The method of statement 49, wherein delivering the co-suspension formulation to the patient comprises delivering the co-suspension formulation at a DDU selected from a DDU of ± 30%, or better, a DDU of ± 25%, or better, and a DDU of ± 20%, or better, throughout emptying of the canister.
Statement 51. A method of respiratory delivery of an active agent to a patient, the method comprising: providing a metered dose inhaler as defined in any of statements 40 through 46; and delivering the co-suspension to the patient using the metered dose inhaler.
Statement 52. A method for treating a patient suffering from an inflammatory or obstructive pulmonary disease or condition, the method comprising administering to the patient via an MDI a therapeutically effective amount of co-suspension as defined in any of statements 1 through 39.
Statement 53. The method of statement 52, wherein the disease or condition is selected from asthma, COPD, exacerbation of airways hyper reactivity consequent to other drug therapy, allergic rhinitis, sinusitis, pulmonary vasoconstriction, inflammation, allergies, impeded respiration, respiratory distress syndrome, pulmonary hypertension, pulmonary vasoconstriction, and pulmonary inflammation and obstruction associated with cystic fibrosis.
Statement 54. The method of statement 53, wherein administering the therapeutically effective amount of the co-suspension comprises: providing a metered dose inhaler comprising a canister containing the co-suspension; and delivering the co-suspension to the patient using the metered dose inhaler such that a DDU selected from a DDU of ± 30%, or better, a DDU of ± 25%, or better, and a DDU of ± 20%, or better, throughout emptying of the canister.

## Claims

1. A co-suspension deliverable from a metered dose inhaler, the stable co-suspension comprising:
a suspension medium comprising a pharmaceutically acceptable propellant;
a plurality of active agent particles wherein the active agent particles comprise glycopyrrolate and at least 90% of the active agent particles by volume exhibits an optical diameter of 7 µm or less; and
a plurality of respirable suspending particles comprising perforated phospholipid microstructures,
wherein the total mass of the respirable suspending particles exceeds the total mass of the active agent particles, the plurality of active agent particles associate with the plurality of respirable suspending particles to form a co-suspension despite buoyancy differences between the active agent particles and the respirable suspending particles within the suspension medium.

2. A co-suspension according to any one of claims 1, wherein at least 50% of the active agent particles by volume exhibit an optical diameter of 5 µm or less.

3. A co-suspension according to any preceding claim, wherein the active agent exhibits measurable solubility that results in dissolution selected from as much as 1 % of the total active agent mass in the suspension medium, as much as 0.5% of the total active agent mass in the suspension medium, as much as 0.05% of the total active agent mass in the suspension medium, and as much as 0.025% of the total active agent mass in the suspension medium.

4. A co-suspension according to any of claims 1 to 3, wherein the total mass of the active agent that dissolves in the suspension medium is less than 5% of the total active agent mass in the suspension medium.

5. A co-suspension according to any preceding claim, wherein the plurality of active agent particles includes two or more different active agents.

6. A co-suspension according to claim 5, wherein the active agent particles comprise a second active agent selected from short-acting beta agonists, such as bitolterol, carbuterol, fenoterol, hexoprenaline, isoprenaline (isoproterenol), levosalbutamol, orciprenaline (metaproterenol), pirbuterol, procaterol, rimiterol, salbutamol (albuterol), terbutaline, tulobuterol, reproterol and epinephrine, long-acting beta agonists, such as bambuterol, clenbuterol, formoterol, and salmeterol, ultra long-acting beta agonists, such as carmoterol, milveterol, indacaterol, and saligenin- or indole- containing and adamantyl-derived β₂ agonists, corticosteroids, such as beclomethasone, budesonide, ciclesonide, flunisolide, fluticasone, methyl-prednisolone, mometasone, prednisone and triamcinolone, antiinflammatories, such as fluticasone propionate, beclomethasone dipropionate, flunisolide, budesonide, tripedane, cortisone, prednisone, prednisilone, dexamethasone, betamethasone, or triamcinolone acetonide, antitussives, such as noscapine, bronchodilators, such as ephedrine, adrenaline, fenoterol, formoterol, isoprenaline, metaproterenol, salbutamol, albuterol, salmeterol, terbutaline, anticholinergics, such as dexipirronium, scopolamine, tropicamide, pirenzepine, dimenhydhnate, tiotropium, darotropium, aclidinium, trospium, ipatropium, atropine, benzatropin, or oxitropium, including any pharmaceutically acceptable salts, esters, or solvates thereof.

7. A co-suspension according to any preceding claim, wherein two or more active agents are included in the active agent particles and the two or more active agents are selected from a combination of glycopyrrolate and formoterol, a combination of glycopyrrolate, formoterol, and budesonide, and a combination of glycopyrrolate, formoterol, and mometasone, including any pharmaceutically acceptable salts, esters, or solvates thereof.

8. A co-suspension according to any preceding claim, wherein at least one active agent is present in crystalline or substantially crystalline form.

9. A co-suspension according to any preceding claim, wherein all the active agents are present in crystalline or substantially crystalline form.

10. A co-suspension according to any preceding claim, wherein the target delivered dose of active agent is selected from between 100 µg and 100 mg per dose, between 100 µg and 10 mg per dose, and between 100 µg and 1 mg per dose.

11. A co-suspension according to any of claims 1 to 10, wherein the target delivered dose of active agent is selected from up to 80 µg per dose, up to 40 µg per dose, up to 20 µg per dose, or between 10 µg and 100 µg per dose.

12. A co-suspension according to any of claims 1 to 11, wherein the target delivered dose of active agent is selected from between 0.1 and 2 µg per dose, 0.1 and 1 µg per dose, and 0.1 and 0.5 µg per dose.

13. A co-suspension according to any preceding claim, wherein the suspending particles exhibit an MMAD selected from between 10 µm and 500 nm, between 5 µm and 750 nm, and 1 µm and 3 µm.

14. A co-suspension according to any preceding claim, wherein the suspending particles exhibit a volume median optical diameter selected from between 0.2 µm and 50 µm, between 0.5 µm and 15 µm, between 1.5 µm and 10 µm, and between 2 µm and 5 µm.

15. A co-suspension according to any preceding claim, wherein the suspending particles are included in the suspension medium at a concentration selected from up to 30 mg/mL and up to 25 mg/mL.

16. A co-suspension according to claim 15, wherein the suspending particles are included in the suspension medium at a concentration selected from between 1 mg/mL to 15 mg/mL, 3 mg/mL to 10 mg/mL, 1.5 mg/mL to 10 mg/mL.

17. A co-suspension according to claims 6 or 7, wherein the active agent particles comprise formoterol, and the suspending particles are included in the suspension medium at a concentration selected from about 3 mg/mL, between 1.5 mg/mL and 5 mg/mL, and between 0.5 mg/mL and 7.5 mg/mL.

18. A co-suspension according to claims 6 or 7, wherein the active agent particles comprise formoterol, and the suspending particles are included in the suspension medium at a concentration selected from about 6 mg/mL, between 3 mg/mL and 10 mg/mL, and between 1 mg/mL and 15 mg/mL.

19. A co-suspension according to claims 6 or 7, wherein the active agent particles comprise budesonide, and the suspending particles are included in the suspension medium at a concentration selected from about 8 mg/mL, between 5 mg/mL and 20 mg/mL, and between 0.5 mg/mL and 30 mg/mL.

20. A co-suspension according to claim 6, wherein the active agent particles comprise fluticasone, and the suspending particles are included in the suspension medium at a concentration selected from about 6 mg/mL, between 3 mg/mL and 10 mg/mL, and between 1 mg/mL and 15 mg/mL.

21. A co-suspension according to any preceding claim, wherein the ratio of the total mass of the suspending particles to the total mass of the active agent particles is selected from above 1.5, up to 5, up to 10, up to 15, up to 20, up to 30, up to 50, up to 75, up to 100, up to 150, and up to 200.

22. A co-suspension according to claim 21, wherein at least one of the active agents included in the active agent particles is a highly potent active agent and the ratio of the total mass of the suspending particles to the total mass of the active agent particles is selected from between 5 and 175, between 10 and 150, between 15 and 125, and between 25 and 75.

23. A co-suspension according to claim 22, wherein the active agent particles include one or more of fluticasone, mometasone, and budesonide, and the ratio of the total mass of the suspending particles to the total mass of the active agent particles is selected from between 1 and 20, between 5 and 15, and about 10.

24. A co-suspension according to claim 21, wherein the active agent particles include one or more of fluticasone, mometasone, and budesonide, and the ratio of the total mass of the suspending particles to the total mass of the active agent particles is selected from between 1 and 15, between 1.5 and 10, and between 2.5 and 5.

25. A co-suspension according to claim 21, wherein the active agent particles include formoterol, and the ratio of the total mass of the suspending particles to the total mass of the active agent particles is selected from between 10 and 200, between 50 and 125, and about 75.

26. A co-suspension according to any preceding claim, wherein the suspension medium comprises propellant substantially free of additional constituents.

27. The co-suspension according to claim 26, wherein the propellant comprises a propellant selected from an HFA propellant, a PFC propellant and combinations thereof.

28. A co-suspension according to any of claims 1 to 25, wherein the suspension medium comprises propellant in combination with one or more constituents selected from antisolvents, solubilizing agents, cosolvents, adjuvants, PVP and PEG.

29. A co-suspension according to any of claims 1 to 28 for use in a method of respiratory delivery of an active agent to a patient, the method comprising: providing a metered dose inhaler comprising a canister containing a co-suspension as defined in any of claims 1 to 28; and delivering the co-suspension to the patient using the metered dose inhaler.

30. A co-suspension according to any of claims 1 to 28 for use in a method for treating a patient suffering from an inflammatory or obstructive pulmonary disease or condition.

31. The co-suspension for use of claim 30, wherein the disease or condition is selected from asthma, COPD, exacerbation of airways hyper reactivity consequent to other drug therapy, allergic rhinitis, sinusitis, pulmonary vasoconstriction, inflammation, allergies, impeded respiration, respiratory distress syndrome, pulmonary hypertension, and pulmonary inflammation and obstruction associated with cystic fibrosis.

## Patentansprüche

1. Co-Suspension, verabreichbar aus einem Dosierinhalator, wobei die stabile Co-Suspension Folgendes umfasst:
ein Suspensionsmedium, welches ein pharmazeutisch annehmbares Treibmittel umfasst,
mehrere Wirkstoffpartikel, wobei die Wirkstoffpartikel Glycopyrrolat umfassen und mindestens 90 Vol.-% der Wirkstoffpartikel einen optischen Durchmesser von 7 µm oder weniger aufweisen, und
mehrere lungengängige suspendierende Partikel, die perforierte Phospholipidmikrostrukturen umfassen,
wobei die Gesamtmasse der lungengängigen suspendierenden Partikel über der Gesamtmasse der Wirkstoffpartikel liegt und die mehreren Wirkstoffpartikel mit den mehreren lungengängigen suspendierenden Partikeln trotz Auftriebunterschieden zwischen den Wirkstoffpartikeln und den lungengängigen suspendierenden Partikeln im Suspensionsmedium unter Bildung einer Co-Suspension assoziieren.

2. Co-Suspension nach Anspruch 1, wobei mindestens 50 Vol.-% der Wirkstoffpartikel einen optischen Durchmesser von 5 µm oder weniger aufweisen.

3. Co-Suspension nach einem der vorhergehenden Ansprüche, wobei der Wirkstoff eine messbare Löslichkeit aufweist, die zu einem Auflösen ausgewählt aus ganzen 1% der Gesamtwirkstoffmasse im Suspensionsmedium, ganzen 0,5% der Gesamtwirkstoffmasse im Suspensionsmedium, ganzen 0,05% der Gesamtwirkstoffmasse im Suspensionsmedium und ganzen 0,025% der Gesamtwirkstoffmasse im Suspensionsmedium führt.

4. Co-Suspension nach einem der Ansprüche 1 bis 3, wobei die Gesamtmasse des Wirkstoffs, die im Suspensionsmedium in Lösung geht, weniger als 5% der Gesamtwirkstoffmasse im Suspensionsmedium beträgt.

5. Co-Suspension nach einem der vorhergehenden Ansprüche, wobei die mehreren Wirkstoffpartikel zwei oder mehr verschiedene Wirkstoffe einschließen.

6. Co-Suspension nach Anspruch 5, wobei die Wirkstoffpartikel einen zweiten Wirkstoff ausgewählt aus Betaagonisten mit kurzer Wirkungsdauer wie Bitolterol, Carbuterol, Fenoterol, Hexoprenalin, Isoprenalin (Isoproterenol), Levosalbutamol, Orciprenalin (Metaproterenol), Pirbuterol, Procaterol, Rimiterol, Salbutamol (Albuterol), Terbutalin, Tulobuterol, Reproterol und Epinephrin, Betaagonisten mit langer Wirkungsdauer wie Bambuterol, Clenbuterol, Formoterol und Salmeterol, Betaagonisten mit ultralanger Wirkungsdauer wie Carmoterol, Milveterol, Indacaterol und saligenin- oder indolhaltigen und von Adamantyl abgeleiteten β₂-Agonisten, Corticosteroiden wie Beclomethason, Budesonid, Ciclesonid, Flunisolid, Fluticason, Methyl-prednisolon, Mometason, Prednison und Triamcinolon, entzündungshemmenden Mitteln wie Fluticasonpropionat, Beclomethasondipropionat, Flunisolid, Budesonid, Tripedan, Cortison, Prednison, Prednisolon, Dexamethason, Betamethason oder Triamcinolinacetonid, Antitussiva wie Noscapin, Bronchodilatatoren wie Ephedrin, Adrenalin, Fenoterol, Formoterol, Isoprenalin, Metaproterenol, Salbutamol, Albuterol, Salmeterol, Terbutalin, Anticholinergika wie Dexipirronium, Scopolamin, Tropicamid, Pirenzepin, Dimenhydrinat, Tiotropium, Darotropium, Aclidinium, Trospium, Ipatropium, Atropin, Benzatropin oder Oxitropium einschließlich pharmazeutisch annehmbare Salze, Ester oder Solvate davon umfassen.

7. Co-Suspension nach einem der vorhergehenden Ansprüche, wobei zwei oder mehr Wirkstoffe in den Wirkstoffpartikeln eingeschlossen sind und die zwei oder mehr Wirkstoffe aus einer Kombination von Glycopyrrolat und Formoterol, einer Kombination von Glycopyrrolat, Formoterol und Budesonid und einer Kombination von Glycopyrrolat, Formoterol und Mometason einschließlich pharmazeutisch annehmbarer Salze, Ester oder Solvate davon ausgewählt sind.

8. Co-Suspension nach einem der vorhergehenden Ansprüche, wobei mindestens ein Wirkstoff in kristalliner oder im Wesentlichen kristalliner Form vorliegt.

9. Co-Suspension nach einem der vorhergehenden Ansprüche, wobei alle Wirkstoffe in kristalliner oder im Wesentlichen kristalliner Form vorliegen.

10. Co-Suspension nach einem der vorhergehenden Ansprüche, wobei die vorgesehene verabreichte Dosis an Wirkstoff aus zwischen 100 µg und 100 mg pro Dosis, zwischen 100 µg und 10 mg pro Dosis und zwischen 100 µg und 1 mg pro Dosis ausgewählt ist.

11. Co-Suspension nach einem der Ansprüche 1 bis 10, wobei die vorgesehene verabreichte Dosis an Wirkstoff aus bis zu 80 µg pro Dosis, bis zu 40 µg pro Dosis, bis zu 20 µg pro Dosis oder zwischen 10 µg und 100 µg pro Dosis ausgewählt ist.

12. Co-Suspension nach einem der Ansprüche 1 bis 11, wobei die vorgesehene verabreichte Dosis an Wirkstoff aus zwischen 0,1 und 2µg pro Dosis, 0,1 und 1 µg pro Dosis und 0,1 und 0,5 µg pro Dosis ausgewählt ist.

13. Co-Suspension nach einem der vorhergehenden Ansprüche, wobei die suspendierenden Partikel einen MMAD ausgewählt aus zwischen 10 µm und 500 nm, zwischen 5 µm und 750 nm und 1 µm und 3 µm aufweisen.

14. Co-Suspension nach einem der vorhergehenden Ansprüche, wobei die suspendierenden Partikel einen volumenmittleren optischen Durchmesser ausgewählt aus zwischen 0,2 µm und 50 µm, zwischen 0,5 µm und 15 µm, zwischen 1,5 µm und 10 µm und zwischen 2 µm und 5 µm aufweisen.

15. Co-Suspension nach einem der vorhergehenden Ansprüche, wobei die suspendierenden Partikel in einer Konzentration ausgewählt aus bis zu 30 mg/ml und bis zu 25 mg/ml im Suspensionsmedium eingeschlossen sind.

16. Co-Suspension nach Anspruch 15, wobei die suspendierenden Partikel in einer Konzentration ausgewählt aus zwischen 1 mg/ml bis 15 mg/ml, 3 mg/ml bis 10 mg/ml, 1,5 mg/ml bis 10 mg/ml im Suspensionsmedium eingeschlossen sind.

17. Co-Suspension nach Anspruch 6 oder 7, wobei die Wirkstoffpartikel Formoterol umfassen und die suspendierenden Partikel in einer Konzentration ausgewählt aus etwa 3 mg/ml, zwischen 1,5 mg/ml und 5 mg/ml und zwischen 0,5 mg/ml und 7,5 mg/ml im Suspensionsmedium eingeschlossen sind.

18. Co-Suspension nach Anspruch 6 oder 7, wobei die Wirkstoffpartikel Formoterol umfassen und die suspendierenden Partikel in einer Konzentration ausgewählt aus etwa 6 mg/ml, zwischen 3 mg/ml und 10 mg/ml und zwischen 1 mg/ml und 15 mg/ml im Suspensionsmedium eingeschlossen sind.

19. Co-Suspension nach Anspruch 6 oder 7, wobei die Wirkstoffpartikel Budenosid umfassen und die suspendierenden Partikel in einer Konzentration ausgewählt aus etwa 8 mg/ml, zwischen 5 mg/ml und 20 mg/ml und zwischen 0,5 mg/ml und 30 mg/ml im Suspensionsmedium eingeschlossen sind.

20. Co-Suspension nach Anspruch 6, wobei die Wirkstoffpartikel Fluticason umfassen und die suspendierenden Partikel in einer Konzentration ausgewählt aus etwa 6 mg/ml, zwischen 3 mg/ml und 10 mg/ml und zwischen 1 mg/ml und 15 mg/ml im Suspensionsmedium eingeschlossen sind.

21. Co-Suspension nach einem der vorhergehenden Ansprüche, wobei das Verhältnis der Gesamtmasse an suspendierenden Partikeln zur Gesamtmasse an Wirkstoffpartikeln aus über 1, 5, bis zu 5, bis zu 10, bis zu 15, bis zu 20, bis zu 30, bis zu 50, bis zu 75, bis zu 100, bis zu 150 und bis zu 200 ausgewählt ist.

22. Co-Suspension nach Anspruch 21, wobei es sich bei mindestens einem der in den Wirkstoffpartikeln eingeschlossenen Wirkstoffe um einen hochwirksamen Wirkstoff handelt und das Verhältnis der Gesamtmasse der suspendierenden Partikel zur Gesamtmasse der Wirkstoffpartikel aus zwischen 5 und 175, zwischen 10 und 150, zwischen 15 und 125 und zwischen 25 und 75 ausgewählt ist.

23. Co-Suspension nach Anspruch 22, wobei die Wirkstoffpartikel Fluticason, Mometason und/oder Budesonid umfassen und das Verhältnis der Gesamtmasse an suspendierenden Partikeln zur Gesamtmasse an Wirkstoffpartikeln aus zwischen 1 und 20, zwischen 5 und 15 und etwa 10 ausgewählt ist.

24. Co-Suspension nach Anspruch 21, wobei die Wirkstoffpartikel Fluticason, Mometason und/oder Budesonid umfassen und das Verhältnis der Gesamtmasse an suspendierenden Partikeln zur Gesamtmasse an Wirkstoffpartikeln aus zwischen 1 und 15, zwischen 1,5 und 10 und zwischen 2,5 und 5 ausgewählt ist.

25. Co-Suspension nach Anspruch 21, wobei die Wirkstoffpartikel Formoterol umfassen und das Verhältnis der Gesamtmasse an suspendierenden Partikeln zur Gesamtmasse an Wirkstoffpartikeln aus zwischen 10 und 200, zwischen 50 und 125 und etwa 75 ausgewählt ist.

26. Co-Suspension nach einem der vorhergehenden Ansprüche, wobei das Suspensionsmedium Treibmittel umfasst, das im Wesentlichen frei von zusätzlichen Bestandteilen ist.

27. Co-Suspension nach Anspruch 26, wobei das Treibmittel ein aus einem HFA-Treibmittel, einem PFC-Treibmittel und Kombinationen davon ausgewähltes Treibmittel umfasst.

28. Co-Suspension nach einem der Ansprüche 1 bis 25, wobei das Suspensionsmedium Treibmittel in Kombination mit einem oder mehreren Bestandteilen ausgewählt aus Antilösungsmitteln, Solubilisierungsmitteln, Co-Solventien, Adjuvantien, PVP und PEG umfasst.

29. Co-Suspension nach einem der Ansprüche 1 bis 28 zur Verwendung bei einem Verfahren zur Verabreichung eines Wirkstoffs an die Atemwege eines Patienten, wobei das Verfahren Folgendes umfasst: die Bereitstellung eines Dosierinhalators, der einen Kanister mit einer wie in einem der Ansprüche 1 bis 28 definierten Co-Suspension umfasst, und die Verabreichung der Co-Suspension an den Patienten unter Verwendung des Dosierinhalators.

30. Co-Suspension nach einem der Ansprüche 1 bis 28 zur Verwendung bei einem Verfahren zur Behandlung eines Patienten, der an einer entzündlichen oder obstruktiven Lungenkrankheit bzw. einem entzündlichen oder obstruktiven Lungenleiden leidet.

31. Co-Suspension zur Verwendung nach Anspruch 30, wobei die Krankheit bzw. das Leiden aus Asthma, COPD, eine Verschlimmerung der Hyperreaktivität der Atemwege als Folge einer anderen Arzneimitteltherapie, allergischer Rhinitis, Sinusitis, pulmonaler Gefäßverengung, Entzündung, Allergien, einer behinderten Atmung, Atemnotsyndrom, pulmonaler Hypertonie und mit zystischer Fibrose assoziierter pulmonaler Entzündung und Obstruktion ausgewählt ist.

## Revendications

1. Co-suspension administrable à partir d'un inhalateur à dose mesurée, la co-suspension stable comprenant :
un milieu de suspension comprenant un propulseur pharmaceutiquement acceptable ;
une pluralité de particules d'agent actif, les particules d'agent actif comprenant du glycopyrrolate et au moins 90% en volume des particules d'agent actif présentant un diamètre optique inférieur ou égal à 7 µm ; et
une pluralité de particules respirables en suspension comprenant des microstructures perforées de phospholipide,
la masse totale des particules respirables en suspension excèdant la masse totale des particules d'agent actif, la pluralité de particules d'agent actif s'associant avec la pluralité de particules respirables en suspension pour former une co-suspension malgré les différences de flottabilité entre les particules d'agent actif et les particules respirables en suspension dans le milieu de suspension.

2. Co-suspension selon la revendication 1, au moins 50% en volume des particules d'agent actif présentant un diamètre optique inférieur ou égal à 5 µm.

3. Co-suspension selon l'une quelconque des revendications précédentes, l'agent actif présentant une solubilité mesurable qui résulte en une dissolution choisie parmi au plus 1% de la masse totale d'agent actif dans le milieu de suspension, au plus 0,5% de la masse totale d'agent actif dans le milieu de suspension, au plus 0,05% de la masse totale d'agent actif dans le milieu de suspension et au plus 0,025% de la masse totale d'agent actif dans le milieu de suspension.

4. Co-suspension selon l'une quelconque des revendications 1 à 3, la masse totale de l'agent actif qui se dissout dans le milieu de suspension étant inférieure à 5% de la masse totale d'agent actif dans le milieu de suspension.

5. Co-suspension selon l'une quelconque des revendications précédentes, la pluralité de particules d'agent actif incluant deux agents actifs différents ou plus.

6. Co-suspension selon la revendication 5, les particules d'agent actif comprenant un deuxième agent actif choisi parmi les agonistes bêta à courte durée d'action, tels que le bitoltérol, le carbutérol, le fénotérol, l'hexoprénaline, l'isoprénaline (isoprotérénol), le lévosalbutamol, l'orciprénaline (métaprotérénol), le pirbutérol, le procatérol, le rimitérol, le salbutamol (albutérol), la terbutaline, le tulobutérol, le reprotérol et l'épinéphrine, les agonistes bêta à longue durée d'action, tels que le bambutérol, le clenbutérol, le formotérol, et le salmétérol, les agonistes bêta à ultra-longue durée d'action, tels que le carmotérol, le milvétérol, l'indacatérol, et les agonistes β₂ contenant saligénine ou indole et dérivés d'adamantyle, les corticostéroïdes, tels que la béclométasone, le budénoside, le ciclésonide, le flunisolide, la fluticasone, la méthyl-prednisolone, la mométasone, la prednisone et la triamcinolone, les anti-inflammatoires, tels que le propionate de fluticasone, le dipropionate de béclométasone, le flunisolide, le budésonide, le tripédane, la cortisone, le prednisone, la prednisilone, la dexaméthasone, la béthaméthasone, ou l'acétonide de triamcinolone, les antitussifs, tels que la noscapine, les bronchodilatateurs, tels que l'éphédrine, l'adrénaline, le fénotérol, le formotérol, l'isoprénaline, le métaprotérénol, le salbutamol, l'albutérol, le salmétérol, la terbutaline, les anticholinergiques, tels que le dexipirronium, la scopolamine, le tropicamide, la pirenzépine, le dimenhydrinate, le tiotropium, le darotropium, l'aclidinium, le trospium, l'ipatropium, l'atropine, la benzatropine, ou l'oxitropium, incluant de quelconques sels, esters, ou solvates pharmaceutiquement acceptables correspondants.

7. Co-suspension selon l'une quelconque des revendications précédentes, deux agents actifs ou plus étant inclus dans les particules d'agent actif et les deux agents actifs ou plus étant choisis parmi une combinaison de glycopyrrolate et de formotérol, une combinaison de glycopyrrolate, de formotérol et de budésonide, et une une combinaison de glycopyrrolate, de formotérol et de mométasone, incluant de quelconques sels, esters, ou solvates pharmaceutiquement acceptables correspondants.

8. Co-suspension selon l'une quelconque des revendications précédentes, au moins un agent actif étant présent sous forme cristalline ou sensiblement cristalline.

9. Co-suspension selon l'une quelconque des revendications précédentes, tous les agents actifs étant présents sous forme cristalline ou sensiblement cristalline.

10. Co-suspension selon l'une quelconque des revendications précédentes, la dose cible administrée d'agent actif étant choisie parmi entre 100 µg et 100 mg par dose, entre 100 µg et 10 mg par dose, et entre 100 µg et 1 mg par dose.

11. Co-suspension selon l'une quelconque des revendications 1 à 10, la dose cible administrée d'agent actif étant choisie parmi jusqu'à 80 µg par dose, jusqu'à 40 µg par dose, jusqu'à 20 µg par dose, ou entre 10 µg et 100 µg par dose.

12. Co-suspension selon l'une quelconque des revendications 1 à 11, la dose cible administrée d'agent actif étant choisie parmi entre 0,1 µg et 2 µg par dose, entre 0,1 µg et 1 µg par dose, et entre 0,1 µg et 0,5 µg par dose.

13. Co-suspension selon l'une quelconque des revendications précédentes, les particules en suspension présentant un DAMM (diamètre aérodynamique moyen en masse) choisi parmi entre 10 µm et 500 nm, entre 5 µm et 750 nm, et entre 1 µm et 3 µm.

14. Co-suspension selon l'une quelconque des revendications précédentes, les particules en suspension présentant un diamètre optique moyen en volume choisi parmi entre 0,2 µm et 50 µm, entre 0,5 µm et 15 µm, entre 1,5 µm et 10 µm, et entre 2 µm et 5 µm.

15. Co-suspension selon l'une quelconque des revendications précédentes, les particules en suspension étant incluses dans le milieu de suspension en une concentration choisie parmi jusqu'à 30 mg/mL et jusqu'à 25 mg/mL.

16. Co-suspension selon la revendication 15, les particules en suspension étant incluses dans le milieu de suspension en une concentration choisie parmi entre 1 mg/mL et 15 mg/mL, entre 3 mg/mL et 10 mg/mL, et entre 1,5 mg/mL et 10 mg/mL.

17. Co-suspension selon la revendication 6 ou 7, les particules d'agent actif comprenant du formotérol, et les particules en suspension étant incluses dans le milieu de suspension en une concentration choisie parmi environ 3 mg/mL, entre 1,5 mg/mL et 5 mg/mL, et entre 0,5 mg/mL et 7,5 mg/mL.

18. Co-suspension selon la revendication 6 ou 7, les particules d'agent actif comprenant du formotérol, et les particules en suspension étant incluses dans le milieu de suspension en une concentration choisie parmi environ 6 mg/mL, entre 3 mg/mL et 10 mg/mL, et entre 1 mg/mL et 15 mg/mL.

19. Co-suspension selon la revendication 6 ou 7, les particules d'agent actif comprenant du budésonide, et les particules en suspension étant incluses dans le milieu de suspension en une concentration choisie parmi environ 8 mg/mL, entre 5 mg/mL et 20 mg/mL, et entre 0,5 mg/mL et 30 mg/mL.

20. Co-suspension selon la revendication 6, les particules d'agent actif comprenant de la fluticasone, et les particules en suspension étant incluses dans le milieu de suspension en une concentration choisie parmi environ 6 mg/mL, entre 3 mg/mL et 10 mg/mL, et entre 1 mg/mL et 15 mg/mL.

21. Co-suspension selon l'une quelconque des revendications précédentes, le rapport de la masse totale de particules en suspension à la masse totale des particules d'agent actif étant choisi parmi supérieur à 1,5, jusqu'à 5, jusqu'à 10, jusqu'à 15, jusqu'à 20, jusqu'à 30, jusqu'à 50, jusqu'à 75, jusqu'à 100, jusqu'à 150, et jusqu'à 200.

22. Co-suspension selon la revendication 21, au moins un des agents actifs inclus dans les particules d'agent actif étant un agent actif hautement puissant et le rapport de la masse totale des particules en suspension à la masse totale des particules d'agent actif étant choisi parmi entre 5 et 175, entre 10 et 150, entre 15 et 125, et entre 25 et 75.

23. Co-suspension selon la revendication 22, les particules d'agent actif incluant un ou plusieurs parmi la fluticasone, la mométasone et le budésonide, et le rapport de la masse totale des particules en suspension à la masse totale des particules d'agent actif étant choisi parmi entre 1 et 20, entre 5 et 15, et environ 10.

24. Co-suspension selon la revendication 21, les particules d'agent actif incluant un ou plusieurs parmi la fluticasone, la mométasone et le budésonide, et le rapport de la masse totale des particules en suspension à la masse totale des particules d'agent actif étant choisi parmi entre 1 et 15, entre 1,5 et 10, et entre 2,5 et 5.

25. Co-suspension selon la revendication 21, les particules d'agent actif incluant du formotérol, et le rapport de la masse totale des particules en suspension à la masse totale des particules d'agent actif étant choisi parmi entre 10 et 200, entre 50 et 125, et environ 75.

26. Co-suspension selon l'une quelconque des revendications précédentes, le milieu de suspension comprenant un propulseur sensiblement exempt de constituants supplémentaires.

27. Co-suspension selon la revendication 26, le propulseur comprenant un propulseur choisi parmi un propulseur HFA (hydrofluoroalcane), un propulseur PFC (perfluorocarbure) et une combinaison correspondante.

28. Co-suspension selon l'une quelconque des revendications 1 à 25, le milieu de suspension comprenant un propulseur en combinaison avec un ou plusieurs constituants choisis parmi des anti-solvants, des agents de solubilisation, des co-solvants, des adjuvants, de la PVP (polyvinylpyrrolidone) et du PEG (polyéthylèneglycol).

29. Co-suspension selon l'une quelconque des revendications 1 à 28, pour une utilisation dans un procédé d'administration respiratoire d'un agent actif à un patient, le procédé comprenant :
la mise à disposition d'un inhalateur à dose mesurée comprenant un canister contenant une co-suspension telle que définie selon l'une quelconque des revendications 1 à 28 ; et
l'administration de la co-suspension à un patient à l'aide de l'inhalateur à dose mesurée.

30. Co-suspension selon l'une quelconque des revendications 1 à 28, pour une utilisation dans un procédé pour le traitement d'un patient souffrant d'une maladie ou d'une affection inflammatoire ou pulmonaire obstructive.

31. Co-suspension pour une utilisation selon la revendication 30, la maladie ou l'affection étant choisie parmi l'asthme, la BPCO (bronchopneumopathie chronique obstructive), l'exacerbation de l'hyperréactivité des voies respiratoires en conséquence d'une autre thérapie médicamenteuse, la rhinite allergique, la sinusite, la vasoconstriction pulmonaire, l'inflammation, les allergies, la respiration entravée, le syndrome de détresse respiratoire, l'hypertension pulmonaire, et l'inflammation et l'obstruction pulmonaire associée à la fibrose cystique.
